# EUROPEAN PATENT SPECIFICATION

(11) **EP 1 620 131 B1**
(45) Date of publication and mention of the grant of the patent: **04.11.2009**
(21) Application number: 04750380.0
(22) Date of filing: 20.04.2004
(51) Int. Cl.: A61K 48/00, A61K 38/25, A61P 13/12

(54) **GHRH FOR USE IN TREATING CHRONIC RENAL FAILURE**
GHRH ZUR VERWENDUNG BEI DER BEHANDLUNG VON CHRONISCHER NIERENINSUFFIZIENZ
GHRH POUR LE TRAITEMENT DES INSUFFISANCES RENALES CHRONIQUES

(30) Priority: 21.04.2003 US 464266 P
(43) Date of publication of application: 01.02.2006
(73) Proprietor: Advisys, Inc., The Woodlands, TX 77381 (US)
(72) Inventor: DRAGHIA-AKLI, Ruxandra, Houston, TX 77035 (US); SCOTT, Clara, Spring, TX 77379 (US); BROWN, Patricia, A., Conroe, TX 77302 (US)
(74) Representative: Fisher, Adrian John
(86) International application number: PCT/US2004/012159
(87) International publication number: WO 2004/093920

(56) References cited:
- WO-A-01/66149
- WO-A-01/66149
- WO-A-99/05300
- WO-A-99/05300
- WO-A-03/049700
- WO-A-03/049700
- PASQUALINI T ET AL: "Growth acceleration in children with chronic renal failure treated with growth-hormone-releasing hormone (GHRH)" MEDICINA, BUENOS AIRES, AR, vol. 56, no. 3, 1996, pages 241-246, XP002965537 ISSN: 0025-7680
- PASQUALINI T (REPRINT) ET AL: "TREATMENT OF CHRONIC- RENAL - FAILURE WITH GROWTH HORMONE - RELEASING HORMONE" PEDIATRIC RESEARCH, (NOV 1994) VOL. 36, NO. 5, PP. 682. ISSN: 0031-3998., 1994, XP008037490
- DATABASE BIOSIS [Online] BIOSCIENCES INFORMATION SERVICE, PHILADELPHIA, PA, US; September 2003 (2003-09), DRAGHIA-AKLI R ET AL: "Effects of plasmid-mediated growth hormone releasing hormone supplementation in young, healthy Beagle dogs." XP002420623 Database accession no. PREV200400245143 & JOURNAL OF ANIMAL SCIENCE, vol. 81, no. 9, September 2003 (2003-09), pages 2301-2310, ISSN: 0021-8812
- DRAGHIA-AKLI RUXANDRA ET AL: "Effects of plasmid-mediated growth hormone-releasing hormone in severely debilitated dogs with cancer." MOLECULAR THERAPY : THE JOURNAL OF THE AMERICAN SOCIETY OF GENE THERAPY. DEC 2002, vol. 6, no. 6, December 2002 (2002-12), pages 830-836, XP002322179 ISSN: 1525-0016
- DRAGHIA-AKLI RUXANDRA ET AL: "Myogenic expression of an injectable protease-resistant growth hormone-releasing hormone augments long-term growth in pigs" NATURE BIOTECHNOLOGY, NATURE PUBLISHING, US, vol. 17, no. 12, December 1999 (1999-12), pages 1179-1183, XP002159711 ISSN: 1087-0156
- PERFUMO F (REPRINT) ET AL: "EFFECTS OF GHRH ON HYPOPHYSEAL SECRETION IN CHILDREN WITH CHRONIC- RENAL - FAILURE (CRF)" HELVETICA PAEDIATRICA ACTA, VOL. 41, NO. 1-2, PP. 148., 1986, XP008037491
- RAMIREZ G (REPRINT) ET AL: "RESPONSE TO GROWTH HORMONE - RELEASING HORMONE IN ADULT RENAL - FAILURE PATIENTS ON HEMODIALYSIS" METABOLISM-CLINICAL AND EXPERIMENTAL, VOL. 39, NO. 7, PP. 764-768., 1990, XP008037489
- PASQUALINI T ET AL: "Growth acceleration in children with chronic renal failure treated with growth-hormone-releasing hormone (GHRH)" MEDICINA, BUENOS AIRES, AR, vol. 56, no. 3, 1996, pages 241-246, XP002965537 ISSN: 0025-7680
- PASQUALINI T (REPRINT) ET AL: "TREATMENT OF CHRONIC- RENAL - FAILURE WITH GROWTH HORMONE - RELEASING HORMONE" PEDIATRIC RESEARCH, (NOV 1994) VOL. 36, NO. 5, PP. 682. ISSN: 0031-3998., 1994, XP008037490
- DRAGHIA-AKLI RUXANDRA ET AL: "Effects of plasmid-mediated growth hormone-releasing hormone in severely debilitated dogs with cancer." MOLECULAR THERAPY : THE JOURNAL OF THE AMERICAN SOCIETY OF GENE THERAPY. DEC 2002, vol. 6, no. 6, December 2002 (2002-12), pages 830-836, XP002322179 ISSN: 1525-0016
- DRAGHIA-AKLI RUXANDRA ET AL: "Myogenic expression of an injectable protease-resistant growth hormone-releasing hormone augments long-term growth in pigs" NATURE BIOTECHNOLOGY, NATURE PUBLISHING, US, vol. 17, no. 12, December 1999 (1999-12), pages 1179-1183, XP002159711 ISSN: 1087-0156 cited in the application
- PERFUMO F (REPRINT) ET AL: "EFFECTS OF GHRH ON HYPOPHYSEAL SECRETION IN CHILDREN WITH CHRONIC- RENAL - FAILURE (CRF)" HELVETICA PAEDIATRICA ACTA, VOL. 41, NO. 1-2, PP. 148., 1986, XP008037491
- RAMIREZ G (REPRINT) ET AL: "RESPONSE TO GROWTH HORMONE - RELEASING HORMONE IN ADULT RENAL - FAILURE PATIENTS ON HEMODIALYSIS" METABOLISM-CLINICAL AND EXPERIMENTAL, VOL. 39, NO. 7, PP. 764-768., 1990, XP008037489

### SEQUENCE LISTING

<110> ADViSYS, Inc.
<120> Plasmid Mediated GHRH- Supplementation for Renal Failures
<130> 108328.00175 - AVSI-0037
<160> 24
<170> PatentIn version 3.1
<210> 1
   <211> 40
   <212> PRT
   <213> artificial sequence
<220>
   <223> This is a GHRH analog
<400> 1
   His Val Asp Ala Ile Phe Thr Asn Ser Tyr Arg Lys Val Leu Ala Gln
   1 5 10 15
   Leu Ser Ala Arg Lys Leu Leu Gln Asp Ile Leu Asn Arg Gln Gln Gly
   20 25 30
   Glu Arg Asn Gln Glu Gln Gly Ala
   35 40
<210> 2
   <211> 40
   <212> PRT
   <213> artificial sequence
<220>
   <223> This is a GHRH analog.
<400> 2
   Tyr Ile Asp Ala Ile Phe Thr Asn Ser Tyr Arg Lys Val Leu Ala Gln
   1 5 10 15
   Leu Ser Ala Arg Lys Leu Leu Gln Asp Ile Leu Asn Arg Gln Gln Gly
   20 25 30
   Glu Arg Asn Gln Glu Gln Gly Ala
   35 40
<210> 3
   <211> 40
   <212> PRT
   <213> artificial sequence
<220>
   <223> This is a GHRH analog.
<400> 3
   Tyr Val Asp Ala Ile Phe Thr Asn Ser Tyr Arg Lys Val Leu Ala Gln
   1 5 10 15
   Leu Ser Ala Arg Lys Leu Leu Gln Asp Ile Leu Asn Arg Gln Gln Gly
   20 25 30
   Glu Arg Asn Gln Glu Gln Gly Ala
   35 40
<210> 4
   <211> 40
   <212> PRT
   <213> artificial sequence
<220>
   <223> This is a GHRH analog.
<400> 4
   Tyr Ala Asp Ala Ile Phe Thr Asn Ser Tyr Arg Lys Val Leu Ala Gln
   1 5 10 15
   Leu Ser Ala Arg Lys Leu Leu Gln Asp Ile Leu Asn Arg Gln Gln Gly
   20 25 30
   Glu Arg Asn Gln Glu Gln Gly Ala
   35 40
<210> 5
<211> 44
   <212> PRT
   <213> artificial sequence
<220>
   <223> This is a GHRH analog.
<400> 5
   Thr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Ser Asn Gln Glu Arg Gly Ala Arg Ala Arg Leu
   35 40
<210> 6
   <211> 40
   <212> PRT
   <213> artificial sequence
<220>
   <223> This is a GHRH analog.
<220>
   <221> MISC_FEATURE
   <222> (1)..(1)
   <223> Xaa at position 1 may be tyrosine, or histidine
<220>
   <221> MISC_FEATURE
   <222> (2)..(2)
   <223> Xaa at position 2 may be alanine, valine, or isoleucine.
<220>
   <221> MISC_FEATURE
   <222> (15)..(15)
   <223> Xaa at position 15 may be alanine, valine, or isoleucine.
<220>
   <221> MISC_FEATURE
   <222> (27)..(27)
   <223> Xaa at position 27 may be methionine, or leucine.
<220>
   <221> MISC_FEATURE
   <222> (28)..(28)
   <223> Xaa at position 28 may be serine or asparagine.
<220>
   <221> MISC_FEATURE
   <222> (34)..(34)
   <223> Xaa at position 34 may be arginine or serine.
<220>
   <221> MISC_FEATURE
   <222> (38)..(38)
   <223> Xaa at position 38 may be glutamine or arginine.
<400> 6
   Xaa Xaa Asp Ala Ile Phe Thr Asn Ser Tyr Arg Lys Val Leu Xaa Gln
   1               5                   10                  15
   Leu Ser Ala Arg Lys Leu Leu Gln Asp Ile Xaa Xaa Arg Gln Gln Gly
               20                  25                  30
   Glu Xaa Asn Gln Glu Xaa Gly Ala
           35                  40
<210> 7
   <211> 323
   <212> DNA
   <213> artificial sequence
<220>
   <223> Nucleic acid sequence of promoter c5-12.
<400> 7
   cggccgtccg ccctcggcac catcctcacg acacccaaat atggcgacgg gtgaggaatg 60
   gtggggagtt atttttagag cggtgaggaa ggtgggcagg cagcaggtgt tggcgctcta 120
   aaaataactc ccgggagtta tttttagagc ggaggaatgg tggacaccca aatatggcga 180
   cggttcctca cccgtcgcca tatttgggtg tccgccctcg gccggggccg cattcctggg 240
   ggccgggcgg tgctcccgcc cgcctcgata aaaggctccg gggccggcgg cggcccacga 300
   gctacccgga ggagcgggag gcg 323
<210> 8
   <211> 190
   <212> DNA
   <213> artificial sequence
<220>
   <223> Nucleic acid sequence of hGH 3'-UTR.
<400> 8
   gggtggcatc cctgtgaccc ctccccagtg cctctcctgg ccctggaagt tgccactcca 60
   gtgcccacca gccttgtcct aataaaatta agttgcatca ttttgtctga ctaggtgtcc 120
   ttctataata ttatggggtg gaggggggtg gtatggagca aggggcaagt tgggaagaca 180
   acctgtaggg 190
<210> 9
   <211> 219
   <212> DNA
   <213> artificial sequence
<220>
   <223> cDNA of Porcine GHRH.
<400> 9
   atggtgctct gggtgttctt ctttgtgatc ctcaccctca gcaacagctc ccactgctcc 60
   ccacctcccc ctttgaccct caggatgcgg cggcacgtag atgccatctt caccaacagc 120
   taccggaagg tgctggccca gctgtccgcc cgcaagctgc tccaggacat cctgaacagg 180
   cagcagggag agaggaacca agagcaagga gcataatga 219
<210> 10
   <211> 40
   <212> PRT
   <213> artificial sequence
<220>
   <223> Amino acid sequence for porcine GHRH.
<400> 10
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Gln Glu Gln Gly Ala
           35                  40
<210> 11
   <211> 3534
   <212> DNA
   <213> artificial sequence
<220>
   <223> HV-GHRH plasmid.
<400> 11
   gttgtaaaac gacggccagt gaattgtaat acgactcact atagggcgaa ttggagctcc      60
   accgcggtgg cggccgtccg ccctcggcac catcctcacg acacccaaat atggcgacgg     120
   gtgaggaatg gtggggagtt atttttagag cggtgaggaa ggtgggcagg cagcaggtgt     180
   tggcgctcta aaaataactc ccgggagtta tttttagagc ggaggaatgg tggacaccca     240
   aatatggcga cggttcctca cccgtcgcca tatttgggtg tccgccctcg gccggggccg     300
   cattcctggg ggccgggcgg tgctcccgcc cgcctcgata aaaggctccg gggccggcgg     360
   cggcccacga gctacccgga ggagcgggag gcgccaagct ctagaactag tggatcccaa     420
   ggcccaactc cccgaaccac tcagggtcct gtggacagct cacctagctg ccatggtgct     480
   ctgggtgttc ttctttgtga tcctcaccct cagcaacagc tcccactgct ccccacctcc     540
   ccctttgacc ctcaggatgc ggcggcacgt agatgccatc ttcaccaaca gctaccggaa     600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga gggggggccc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 12
   <211> 3534
   <212> DNA
   <213> artificial sequence
<220>
   <223> TI-GHRH plasmid.
<400> 12
   gttgtaaaac gacggccagt gaattgtaat acgactcact atagggcgaa ttggagctcc      60
   accgcggtgg cggccgtccg ccctcggcac catcctcacg acacccaaat atggcgacgg     120
   gtgaggaatg gtggggagtt atttttagag cggtgaggaa ggtgggcagg cagcaggtgt     180
   tggcgctcta aaaataactc ccgggagtta tttttagagc ggaggaatgg tggacaccca     240
   aatatggcga cggttcctca cccgtcgcca tatttgggtg tccgccctcg gccggggccg     300
   cattcctggg ggccgggcgg tgctcccgcc cgcctcgata aaaggctccg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggtatat cgatgccatc ttcaccaaca gctaccggaa 600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga gggggggccc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 13
   <211> 3534
   <212> DNA
   <213> artificial sequence
<220>
   <223> TV-GHRH plasmid.
<400> 13
   gttgtaaaac gacggccagt gaattgtaat acgactcact atagggcgaa ttggagctcc 60
   accgcggtgg cggccgtccg ccctcggcac catcctcacg acacccaaat atggcgacgg 120
   gtgaggaatg gtggggagtt atttttagag cggtgaggaa ggtgggcagg cagcaggtgt 180
   tggcgctcta aaaataactc ccgggagtta tttttagagc ggaggaatgg tggacaccca 240
   aatatggcga cggttcctca cccgtcgcca tatttgggtg tccgccctcg gccggggccg 300
   cattcctggg ggccgggcgg tgctcccgcc cgcctcgata aaaggctccg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggtatgt agatgccatc ttcaccaaca gctaccggaa 600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga gggggggccc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 14
   <211> 3534
   <212> DNA
   <213> artificial sequence
<220>
   <223> 15/27/28 GHRH plasmid.
<400> 14
   gttgtaaaac gacggccagt gaattgtaat acgactcact atagggcgaa ttggagctcc     60
   accgcggtgg cggccgtccg ccctcggcac catcctcacg acacccaaat atggcgacgg    120
   gtgaggaatg gtggggagtt atttttagag cggtgaggaa ggtgggcagg cagcaggtgt    180
   tggcgctcta aaaataactc ccgggagtta tttttagagc ggaggaatgg tggacaccca    240
   aatatggcga cggttcctca cccgtcgcca tatttgggtg tccgccctcg gccggggccg    300
   cattcctggg ggccgggcgg tgctcccgcc cgcctcgata aaggctccgg gggccggcgg     360
   cggcccacga gctacccgga ggagcgggag gcgccaagct ctagaactag tggatcccaa    420
   ggcccaactc cccgaaccac tcagggtcct gtggacagct cacctagctg ccatggtgct    480
   ctgggtgttc ttctttgtga tcctcaccct cagcaacagc tcccactgct ccccacctcc    540
   ccctttgacc ctcaggatgc ggcggtatat cgatgccatc ttcaccaaca gctaccggaa    600
   ggtgctggcc cagctgtccg cccgcaagct gctccaggac atcctgaaca ggcagcaggg    660
   agagaggaac caagagcaag gagcataatg actgcaggaa ttcgatatca agcttatcgg    720
   ggtggcatcc ctgtgacccc tccccagtgc ctctcctggc cctggaagtt gccactccag    780
   tgcccaccag ccttgtccta ataaaattaa gttgcatcat tttgtctgac taggtgtcct    840
   tctataatat tatggggtgg aggggggtgg tatggagcaa ggggcaagtt gggaagacaa    900
   cctgtagggc ctgcggggtc tattgggaac caagctggag tgcagtggca caatcttggc    960
   tcactgcaat ctccgcctcc tgggttcaag cgattctcct gcctcagcct cccgagttgt   1020
   tgggattcca ggcatgcatg accaggctca gctaattttt gtttttttgg tagagacggg   1080
   gtttcaccat attggccagg ctggtctcca actcctaatc tcaggtgatc tacccacctt   1140
   ggcctcccaa attgctggga ttacaggcgt gaaccactgc tcccttccct gtccttctga   1200
   ttttaaaata actataccag caggaggacg tccagacaca gcataggcta cctggccatg   1260
   cccaaccggt gggacatttg agttgcttgc ttggcactgt cctctcatgc gttgggtcca   1320
   ctcagtagat gcctgttgaa ttcgataccg tcgacctcga gggggggccc ggtaccagct   1380
   tttgttccct ttagtgaggg ttaatttcga gcttggcgta atcatggtca tagctgtttc   1440
   ctgtgtgaaa ttgttatccg ctcacaattc cacacaacat acgagccgga agcataaagt   1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 15
   <211> 3534
   <212> DNA
   <213> artificial sequence
<220>
   <223> Plasmid sequence for wildtype GHRH.
<400> 15
   gttgtaaaac gacggccagt gaattgtaat acgactcact atagggcgaa ttggagctcc 60
   accgcggtgg cggccgtccg ccctcggcac catcctcacg acacccaaat atggcgacgg 120
   gtgaggaatg gtggggagtt atttttagag cggtgaggaa ggtgggcagg cagcaggtgt 180
   tggcgctcta aaaataactc ccgggagtta tttttagagc ggaggaatgg tggacaccca 240
   aatatggcga cggttcctca cccgtcgcca tatttgggtg tccgccctcg gccggggccg 300
   cattcctggg ggccgggcgg tgctcccgcc cgcctcgata aaaggctccg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggtatgc agatgccatc ttcaccaaca gctaccggaa 600
   ggtgctgggc cagctgtccg cccgcaagct gctccaggac atcatgagca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga gggggggccc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 16
   <211> 4260
   <212> DNA
   <213> Artificial sequence
<220>
   <223> pSP-SEAP cDNA construct.
<400> 16
   ggccgtccgc cttcggcacc atcctcacga cacccaaata tggcgacggg tgaggaatgg 60
   tggggagtta tttttagagc ggtgaggaag gtgggcaggc agcaggtgtt ggcgctctaa 120
   aaataactcc cgggagttat ttttagagcg gaggaatggt ggacacccaa atatggcgac 180
   ggttcctcac ccgtcgccat atttgggtgt ccgccctcgg ccggggccgc attcctgggg 240
   gccgggcggt gctcccgccc gcctcgataa aaggctccgg ggccggcggc ggcccacgag 300
   ctacccggag gagcgggagg cgccaagctc tagaactagt ggatcccccg ggctgcagga 360
   attcgatatc aagcttcgaa tcgcgaattc gcccaccatg ctgctgctgc tgctgctgct 420
   gggcctgagg ctacagctct ccctgggcat catcccagtt gaggaggaga acccggactt 480
   ctggaaccgc gaggcagccg aggccctggg tgccgccaag aagctgcagc ctgcacagac 540
   agccgccaag aacctcatca tcttcctggg cgatgggatg gggtgtctca cggtgacagc 600
   tgccaggatc ctaaaagggc agaagaagga caaactgggg cctgagatac ccctggccat 660
   ggaccgcttc ccatatgtgg ctctgtccaa gacatacaat gtagacaaac atgtgccaga 720
   cagtggagcc acagccacgg cctacctgtg cggggtcaag ggcaacttcc agaccattgg 780
   cttgagtgca gccgcccgct ttaaccagtg caacacgaca cgcggcaacg aggtcatctc 840
   cgtgatgaat cgggccaaga aagcagggaa gtcagtggga gtggtaacca ccacacgagt 900
   gcagcacgcc tcgccagccg gcacctacgc ccacacggtg aaccgcaact ggtactcgga 960
   cgccgacgtg cctgcctcgg cccgccagga ggggtgccag gacatcgcta cgcagctcat 1020
   ctccaacatg gacattgacg tgatcctagg tggaggccga aagtacatgt ttcgcatggg 1080
   aaccccagac cctgagtacc cagatgacta cagccaaggt gggaccaggc tggacgggaa 1140
   gaatctggtg caggaatggc tggcgaagcg ccagggtgcc cggtatgtgt ggaaccgcac 1200
   tgagctcatg caggcttccc tggacccgtc tgtgacccat ctcatgggtc tctttgagcc 1260
   tggagacatg aaatacgaga tccaccgaga ctccacactg gacccctccc tgatggagat 1320
   gacagaggct gccctgcgcc tgctgagcag gaacccccgc ggcttcttcc tcttcgtgga 1380
   gggtggtcgc atcgaccatg gtcatcatga aagcagggct taccgggcac tgactgagac 1440
   gatcatgttc gacgacgcca ttgagagggc gggccagctc accagcgagg aggacacgct 1500
   gagcctcgtc actgccgacc actcccacgt cttctccttc ggaggctacc ccctgcgagg 1560
   gagctccatc ttcgggctgg cccctggcaa ggcccgggac aggaaggcct acacggtcct 1620
   cctatacgga aacggtccag gctatgtgct caaggacggc gcccggccgg atgttaccga 1680
   gagcgagagc gggagccccg agtatcggca gcagtcagca gtgcccctgg acgaagagac 1740
   ccacgcaggc gaggacgtgg cggtgttcgc gcgcggcccg caggcgcacc tggttcacgg 1800
   cgtgcaggag cagaccttca tagcgcacgt catggccttc gccgcctgcc tggagcccta 1860
   caccgcctgc gacctggcgc cccccgccgg caccaccgac gccgcgcacc cgggttactc 1920
   tagagtcggg gcggccggcc gcttcgagca gacatgataa gatacattga tgagtttgga 1980
   caaaccacaa ctagaatgca gtgaaaaaaa tgctttattt gtgaaatttg tgatgctatt 2040
   gctttatttg taaccattat aagctgcaat aaacaagtta acaacaacaa ttgcattcat 2100
   tttatgtttc aggttcaggg ggaggtgtgg gaggtttttt aaagcaagta aaacctctac 2160
   aaatgtggta aaatcgataa ggatccgtcg accgatgccc ttgagagcct tcaacccagt 2220
   cagctccttc cggtgggcgc ggggcatgac tatcgtcgcc gcacttatga ctgtcttctt 2280
   tatcatgcaa ctcgtaggac aggtgccggc agcgctcttc cgcttcctcg ctcactgact 2340
   cgctgcgctc ggtcgttcgg ctgcggcgag cggtatcagc tcactcaaag gcggtaatac 2400
   ggttatccac agaatcaggg gataacgcag gaaagaacat gtgagcaaaa ggccagcaaa 2460
   aggccaggaa ccgtaaaaag gccgcgttgc tggcgttttt ccataggctc cgcccccctg 2520
   acgagcatca caaaaatcga cgctcaagtc agaggtggcg aaacccgaca ggactataaa 2580
   gataccaggc gtttccccct ggaagctccc tcgtgcgctc tcctgttccg accctgccgc 2640
   ttaccggata cctgtccgcc tttctccctt cgggaagcgt ggcgctttct catagctcac 2700
   gctgtaggta tctcagttcg gtgtaggtcg ttcgctccaa gctgggctgt gtgcacgaac 2760
   cccccgttca gcccgaccgc tgcgccttat ccggtaacta tcgtcttgag tccaacccgg 2820
   taagacacga cttatcgcca ctggcagcag ccactggtaa caggattagc agagcgaggt 2880
   atgtaggcgg tgctacagag ttcttgaagt ggtggcctaa ctacggctac actagaagga 2940
   cagtatttgg tatctgcgct ctgctgaagc cagttacctt cggaaaaaga gttggtagct 3000
   cttgatccgg caaacaaacc accgctggta gcggtggttt ttttgtttgc aagcagcaga 3060
   ttacgcgcag aaaaaaagga tctcaagaag atcctttgat cttttctacg gggtctgacg 3120
   ctcagtggaa cgaaaactca cgttaaggga ttttggtcat gagattatca aaaaggatct 3180
   tcacctagat ccttttaaat taaaaatgaa gttttaaatc aatctaaagt atatatgagt 3240
   aaacttggtc tgacagttac caatgcttaa tcagtgaggc acctatctca gcgatctgtc 3300
   tatttcgttc atccatagtt gcctgactcc ccgtcgtgta gataactacg atacgggagg 3360
   gcttaccatc tggccccagt gctgcaatga taccgcgaga cccacgctca ccggctccag 3420
   atttatcagc aataaaccag ccagccggaa gggccgagcg cagaagtggt cctgcaactt 3480
   tatccgcctc catccagtct attaattgtt gccgggaagc tagagtaagt agttcgccag 3540
   ttaatagttt gcgcaacgtt gttgccattg ctacaggcat cgtggtgtca cgctcgtcgt 3600
   ttggtatggc ttcattcagc tccggttccc aacgatcaag gcgagttaca tgatccccca 3660
   tgttgtgcaa aaaagcggtt agctccttcg gtcctccgat cgttgtcaga agtaagttgg 3720
   ccgcagtgtt atcactcatg gttatggcag cactgcataa ttctcttact gtcatgccat 3780
   ccgtaagatg cttttctgtg actggtgagt actcaaccaa gtcattctga gaatagtgta 3840
   tgcggcgacc gagttgctct tgcccggcgt caatacggga taataccgcg ccacatagca 3900
   gaactttaaa agtgctcatc attggaaaac gttcttcggg gcgaaaactc tcaaggatct 3960
   taccgctgtt gagatccagt tcgatgtaac ccactcgtgc acccaactga tcttcagcat 4020
   cttttacttt caccagcgtt tctgggtgag caaaaacagg aaggcaaaat gccgcaaaaa 4080
   agggaataag ggcgacacgg aaatgttgaa tactcatact cttccttttt caatattatt 4140
   gaagcattta tcagggttat tgtctcatga gcggatacat atttgaatgt atttagaaaa 4200
   ataaacaaat aggggttccg cgcacatttc cccgaaaagt gccacctgac gcgccctgta 4260
<210> 17
   <211> 2710
   <212> DNA
   <213> artificial sequence
<220>
   <223> Codon optimized GHRH plasmid for mouse.
<400> 17
   tgtaatacga ctcactatag ggcgaattgg agctccaccg cggtggcggc cgtccgccct 60
   cggcaccatc ctcacgacac ccaaatatgg cgacgggtga ggaatggtgg ggagttattt 120
   ttagagcggt gaggaaggtg ggcaggcagc aggtgttggc gctctaaaaa taactcccgg 180
   gagttatttt tagagcggag gaatggtgga cacccaaata tggcgacggt tcctcacccg 240
   tcgccatatt tgggtgtccg ccctcggccg gggccgcatt cctgggggcc gggcggtgct 300
   cccgcccgcc tcgataaaag gctccggggc cggcggcggc ccacgagcta cccggaggag 360
   cgggaggcgc caagcggatc ccaaggccca actccccgaa ccactcaggg tcctgtggac 420
   agctcaccta gctgccatgg tgctctgggt gctctttgtg atcctcatcc tcaccagcgg 480
   cagccactgc agcctgcctc ccagccctcc cttcaggatg cagaggcacg tggacgccat 540
   cttcaccacc aactacagga agctgctgag ccagctgtac gccaggaagg tgatccagga 600
   catcatgaac aagcagggcg agaggatcca ggagcagagg gccaggctga gctgataagc 660
   ttatcggggt ggcatccctg tgacccctcc ccagtgcctc tcctggccct ggaagttgcc 720
   actccagtgc ccaccagcct tgtcctaata aaattaagtt gcatcatttt gtctgactag 780
   gtgtccttct ataatattat ggggtggagg ggggtggtat ggagcaaggg gcaagttggg 840
   aagacaacct gtagggctcg agggggggcc cggtaccagc ttttgttccc tttagtgagg 900
   gttaatttcg agcttggtct tccgcttcct cgctcactga ctcgctgcgc tcggtcgttc 960
   ggctgcggcg agcggtatca gctcactcaa aggcggtaat acggttatcc acagaatcag 1020
   gggataacgc aggaaagaac atgtgagcaa aaggccagca aaaggccagg aaccgtaaaa 1080
   aggccgcgtt gctggcgttt ttccataggc tccgcccccc tgacgagcat cacaaaaatc 1140
   gacgctcaag tcagaggtgg cgaaacccga caggactata aagataccag gcgtttcccc 1200
   ctggaagctc cctcgtgcgc tctcctgttc cgaccctgcc gcttaccgga tacctgtccg 1260
   cctttctccc ttcgggaagc gtggcgcttt ctcatagctc acgctgtagg tatctcagtt 1320
   cggtgtaggt cgttcgctcc aagctgggct gtgtgcacga accccccgtt cagcccgacc 1380
   gctgcgcctt atccggtaac tatcgtcttg agtccaaccc ggtaagacac gacttatcgc 1440
   cactggcagc agccactggt aacaggatta gcagagcgag gtatgtaggc ggtgctacag 1500
   agttcttgaa gtggtggcct aactacggct acactagaag aacagtattt ggtatctgcg 1560
   ctctgctgaa gccagttacc ttcggaaaaa gagttggtag ctcttgatcc ggcaaacaaa 1620
   ccaccgctgg tagcggtggt ttttttgttt gcaagcagca gattacgcgc agaaaaaaag 1680
   gatctcaaga agatcctttg atcttttcta cggggctagc gcttagaaga actcatccag 1740
   cagacggtag aatgcaatac gttgagagtc tggagctgca ataccataca gaaccaggaa 1800
   acggtcagcc cattcaccac ccagttcctc tgcaatgtca cgggtagcca gtgcaatgtc 1860
   ctggtaacgg tctgcaacac ccagacgacc acagtcaatg aaaccagaga aacgaccatt 1920
   ctcaaccatg atgttcggca ggcatgcatc accatgagta actaccaggt cctcaccatc 1980
   cggcatacga gctttcagac gtgcaaacag ttcagccggt gccagaccct gatgttcctc 2040
   atccaggtca tcctggtcaa ccagacctgc ttccatacgg gtacgagcac gttcaatacg 2100
   atgttttgcc tggtggtcaa acggacaggt agctgggtcc agggtgtgca gacgacgcat 2160
   tgcatcagcc atgatagaaa ctttctctgc cggagccagg tgagaagaca gcaggtcctg 2220
   acccggaact tcacccagca gcagccagtc acgaccagct tcagtaacta catccagaac 2280
   tgcagcacac ggaacaccag tggttgccag ccaagacaga cgagctgctt catcctgcag 2340
   ttcattcaga gcaccagaca ggtcagtttt aacaaacaga actggacgac cctgtgcaga 2400
   cagacggaaa acagctgcat cagagcaacc aatggtctgc tgtgcccagt cataaccaaa 2460
   cagacgttca acccaggctg ccggagaacc tgcatgcaga ccatcctgtt caatcatgcg 2520
   aaacgatcct catcctgtct cttgatcaga tcttgatccc ctgcgccatc agatccttgg 2580
   cggcaagaaa gccatccagt ttactttgca gggcttccca accttaccag agggcgcccc 2640
   agctggcaat tccggttcgc ttgctgtcca taaaaccgcc cagtctagca actgttggga 2700
   agggcgatcg 2710
<210> 18
   <211> 2713
   <212> DNA
   <213> artificial sequence
<220>
   <223> Codon optimized GHRH plasmid for rat.
<400> 18
   tgtaatacga ctcactatag ggcgaattgg agctccaccg cggtggcggc cgtccgccct 60
   cggcaccatc ctcacgacac ccaaatatgg cgacgggtga ggaatggtgg ggagttattt 120
   ttagagcggt gaggaaggtg ggcaggcagc aggtgttggc gctctaaaaa taactcccgg 180
   gagttatttt tagagcggag gaatggtgga cacccaaata tggcgacggt tcctcacccg 240
   tcgccatatt tgggtgtccg ccctcggccg gggccgcatt cctgggggcc gggcggtgct 300
   cccgcccgcc tcgataaaag gctccggggc cggcggcggc ccacgagcta cccggaggag 360
   cgggaggcgc caagcggatc ccaaggccca actccccgaa ccactcaggg tcctgtggac    420
   agctcaccta gctgccatgg ccctgtgggt gttcttcgtg ctgctgaccc tgaccagcgg    480
   aagccactgc agcctgcctc ccagccctcc cttcagggtg cgccggcacg ccgacgccat    540
   cttcaccagc agctacagga ggatcctggg ccagctgtac gctaggaagc tcctgcacga    600
   gatcatgaac aggcagcagg gcgagaggaa ccaggagcag aggagcaggt tcaactgata    660
   agcttatcgg ggtggcatcc ctgtgacccc tccccagtgc ctctcctggc cctggaagtt    720
   gccactccag tgcccaccag ccttgtccta ataaaattaa gttgcatcat tttgtctgac    780
   taggtgtcct tctataatat tatggggtgg aggggggtgg tatggagcaa ggggcaagtt    840
   gggaagacaa cctgtagggc tcgagggggg gcccggtacc agcttttgtt ccctttagtg    900
   agggttaatt tcgagcttgg tcttccgctt cctcgctcac tgactcgctg cgctcggtcg    960
   ttcggctgcg gcgagcggta tcagctcact caaaggcggt aatacggtta tccacagaat   1020
   caggggataa cgcaggaaag aacatgtgag caaaaggcca gcaaaaggcc aggaaccgta   1080
   aaaaggccgc gttgctggcg tttttccata ggctccgccc ccctgacgag catcacaaaa   1140
   atcgacgctc aagtcagagg tggcgaaacc cgacaggact ataaagatac caggcgtttc   1200
   cccctggaag ctccctcgtg cgctctcctg ttccgaccct gccgcttacc ggatacctgt   1260
   ccgcctttct cccttcggga agcgtggcgc tttctcatag ctcacgctgt aggtatctca   1320
   gttcggtgta ggtcgttcgc tccaagctgg gctgtgtgca cgaacccccc gttcagcccg   1380
   accgctgcgc cttatccggt aactatcgtc ttgagtccaa cccggtaaga cacgacttat   1440
   cgccactggc agcagccact ggtaacagga ttagcagagc gaggtatgta ggcggtgcta   1500
   cagagttctt gaagtggtgg cctaactacg gctacactag aagaacagta tttggtatct   1560
   gcgctctgct gaagccagtt accttcggaa aaagagttgg tagctcttga tccggcaaac   1620
   aaaccaccgc tggtagcggt ggtttttttg tttgcaagca gcagattacg cgcagaaaaa   1680
   aaggatctca agaagatcct ttgatctttt ctacggggct agcgcttaga agaactcatc   1740
   cagcagacgg tagaatgcaa tacgttgaga gtctggagct gcaataccat acagaaccag   1800
   gaaacggtca gcccattcac cacccagttc ctctgcaatg tcacgggtag ccagtgcaat   1860
   gtcctggtaa cggtctgcaa cacccagacg accacagtca atgaaaccag agaaacgacc   1920
   attctcaacc atgatgttcg gcaggcatgc atcaccatga gtaactacca ggtcctcacc   1980
   atccggcata cgagctttca gacgtgcaaa cagttcagcc ggtgccagac cctgatgttc   2040
   ctcatccagg tcatcctggt caaccagacc tgcttccata cgggtacgag cacgttcaat   2100
   acgatgtttt gcctggtggt caaacggaca ggtagctggg tccagggtgt gcagacgacg 2160
   cattgcatca gccatgatag aaactttctc tgccggagcc aggtgagaag acagcaggtc 2220
   ctgacccgga acttcaccca gcagcagcca gtcacgacca gcttcagtaa ctacatccag 2280
   aactgcagca cacggaacac cagtggttgc cagccaagac agacgagctg cttcatcctg 2340
   cagttcattc agagcaccag acaggtcagt tttaacaaac agaactggac gaccctgtgc 2400
   agacagacgg aaaacagctg catcagagca accaatggtc tgctgtgccc agtcataacc 2460
   aaacagacgt tcaacccagg ctgccggaga acctgcatgc agaccatcct gttcaatcat 2520
   gcgaaacgat cctcatcctg tctcttgatc agatcttgat cccctgcgcc atcagatcct 2580
   tggcggcaag aaagccatcc agtttacttt gcagggcttc ccaaccttac cagagggcgc 2640
   cccagctggc aattccggtt cgcttgctgt ccataaaacc gcccagtcta gcaactgttg 2700
   ggaagggcga tcg 2713
<210> 19
   <211> 2716
   <212> DNA
   <213> artificial sequence
<220>
   <223> Codon optimized GHRH plasmid for bovine.
<400> 19
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct gtgggtgttc 420
   ttcctggtga ccctgaccct gagcagcgga agccacggca gcctgcccag ccagcccctg 480
   aggatcccta ggtacgccga cgccatcttc accaacagct acaggaagat cctgggccag 540
   ctgagcgcta ggaagctcct gcaggacatc atgaacaggc agcagggcga gaggaaccag 600
   gagcagggcg cctgataagc ttatcggggt ggcatccctg tgacccctcc ccagtgcctc 660
   tcctggccct ggaagttgcc actccagtgc ccaccagcct tgtcctaata aaattaagtt 720
   gcatcatttt gtctgactag gtgtccttct ataatattat ggggtggagg ggggtggtat 780
   ggagcaaggg gcaagttggg aagacaacct gtagggctcg agggggggcc cggtaccagc 840
   ttttgttccc tttagtgagg gttaatttcg agcttggtct tccgcttcct cgctcactga 900
   ctcgctgcgc tcggtcgttc ggctgcggcg agcggtatca gctcactcaa aggcggtaat 960
   acggttatcc acagaatcag gggataacgc aggaaagaac atgtgagcaa aaggccagca 1020
   aaaggccagg aaccgtaaaa aggccgcgtt gctggcgttt ttccataggc tccgcccccc 1080
   tgacgagcat cacaaaaatc gacgctcaag tcagaggtgg cgaaacccga caggactata 1140
   aagataccag gcgtttcccc ctggaagctc cctcgtgcgc tctcctgttc cgaccctgcc 1200
   gcttaccgga tacctgtccg cctttctccc ttcgggaagc gtggcgcttt ctcatagctc 1260
   acgctgtagg tatctcagtt cggtgtaggt cgttcgctcc aagctgggct gtgtgcacga 1320
   accccccgtt cagcccgacc gctgcgcctt atccggtaac tatcgtcttg agtccaaccc 1380
   ggtaagacac gacttatcgc cactggcagc agccactggt aacaggatta gcagagcgag 1440
   gtatgtaggc ggtgctacag agttcttgaa gtggtggcct aactacggct acactagaag 1500
   aacagtattt ggtatctgcg ctctgctgaa gccagttacc ttcggaaaaa gagttggtag 1560
   ctcttgatcc gacaaacaaa ccaccgctgg tagcggtggt ttttttgttt gcaagcagca 1620
   gattacgcgc agaaaaaaag gatctcaaga agatcctttg atcttttcta cggggtctga 1680
   cgctcagcta gcgctcagaa gaactcgtca agaaggcgat agaaggcgat gcgctgcgaa 1740
   tcgggagcgg cgataccgta aagcacgagg aagcggtcag cccattcgcc gccaagctct 1800
   tcagcaatat cacgggtagc caacgctatg tcctgatagc ggtccgccac acccagccgg 1860
   ccacagtcga tgaatccaga aaagcggcca ttttccacca tgatattcgg caagcaggca 1920
   tcgccatgag tcacgacgag atcctcgccg tcgggcatgc gcgccttgag cctggcgaac 1980
   agttcggctg gcgcgagccc ctgatgctct tcgtccagat catcctgatc gacaagaccg 2040
   gcttccatcc gagtacgtgc tcgctcgatg cgatgtttcg cttggtggtc gaatgggcag 2100
   gtagccggat caagcgtatg cagccgccgc attgcatcag ccatgatgga tactttctcg 2160
   gcaggagcaa ggtgagatga caggagatcc tgccccggca cttcgcccaa tagcagccag 2220
   tcccttcccg cttcagtgac aacgtcgagc acagctgcgc aaggaacgcc cgtcgtggcc 2280
   agccacgata gccgcgctgc ctcgtcctgc agttcattca gggcaccgga caggtcggtc 2340
   ttgacaaaaa gaaccgggcg cccctgcgct gacagccgga acacggcggc atcagagcag 2400
   ccgattgtct gttgtgccca gtcatagccg aatagcctct ccacccaagc ggccggagaa 2460
   cctgcgtgca atccatcttg ttcaatcatg cgaaacgatc ctcatcctgt ctcttgatca 2520
   gatcttgatc ccctgcgcca tcagatcctt ggcggcaaga aagccatcca gtttactttg 2580
   cagggcttcc caaccttacc agagggcgcc ccagctggca attccggttc gcttgctgtc 2640
   cataaaaccg cccagtctag caactgttgg gaagggcgat cgtgtaatac gactcactat 2700
   agggcgaatt ggagct 2716
<210> 20
   <211> 2716
   <212> DNA
   <213> artificial sequence
<220>
   <223> Codon optimized GHRH plasmid for ovine.
<400> 20
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct gtgggtgttc 420
   ttcctggtga ccctgaccct gagcagcgga agccacggca gcctgcccag ccagcccctg 480
   aggatcccta ggtacgccga cgccatcttc accaacagct acaggaagat cctgggccag 540
   ctgagcgcta ggaagctcct gcaggacatc atgaacaggc agcagggcga gaggaaccag 600
   gagcagggcg cctgataagc ttatcggggt ggcatccctg tgacccctcc ccagtgcctc 660
   tcctggccct ggaagttgcc actccagtgc ccaccagcct tgtcctaata aaattaagtt 720
   gcatcatttt gtctgactag gtgtccttct ataatattat ggggtggagg ggggtggtat 780
   ggagcaaggg gcaagttggg aagacaacct gtagggctcg agggggggcc cggtaccagc 840
   ttttgttccc tttagtgagg gttaatttcg agcttggtct tccgcttcct cgctcactga 900
   ctcgctgcgc tcggtcgttc ggctgcggcg agcggtatca gctcactcaa aggcggtaat 960
   acggttatcc acagaatcag gggataacgc aggaaagaac atgtgagcaa aaggccagca 1020
   aaaggccagg aaccgtaaaa aggccgcgtt gctggcgttt ttccataggc tccgcccccc 1080
   tgacgagcat cacaaaaatc gacgctcaag tcagaggtgg cgaaacccga caggactata 1140
   aagataccag gcgtttcccc ctggaagctc cctcgtgcgc tctcctgttc cgaccctgcc 1200
   gcttaccgga tacctgtccg cctttctccc ttcgggaagc gtggcgcttt ctcatagctc 1260
   acgctgtagg tatctcagtt cggtgtaggt cgttcgctcc aagctgggct gtgtgcacga 1320
   accccccgtt cagcccgacc gctgcgcctt atccggtaac tatcgtcttg agtccaaccc 1380
   ggtaagacac gacttatcgc cactggcagc agccactggt aacaggatta gcagagcgag 1440
   gtatgtaggc ggtgctacag agttcttgaa gtggtggcct aactacggct acactagaag 1500
   aacagtattt ggtatctgcg ctctgctgaa gccagttacc ttcggaaaaa gagttggtag 1560
   ctcttgatcc gacaaacaaa ccaccgctgg tagcggtggt ttttttgttt gcaagcagca 1620
   gattacgcgc agaaaaaaag gatctcaaga agatcctttg atcttttcta cggggtctga 1680
   cgctcagcta gcgctcagaa gaactcgtca agaaggcgat agaaggcgat gcgctgcgaa 1740
   tcgggagcgg cgataccgta aagcacgagg aagcggtcag cccattcgcc gccaagctct 1800
   tcagcaatat cacgggtagc caacgctatg tcctgatagc ggtccgccac acccagccgg 1860
   ccacagtcga tgaatccaga aaagcggcca ttttccacca tgatattcgg caagcaggca 1920
   tcgccatgag tcacgacgag atcctcgccg tcgggcatgc gcgccttgag cctggcgaac 1980
   agttcggctg gcgcgagccc ctgatgctct tcgtccagat catcctgatc gacaagaccg 2040
   gcttccatcc gagtacgtgc tcgctcgatg cgatgtttcg cttggtggtc gaatgggcag 2100
   gtagccggat caagcgtatg cagccgccgc attgcatcag ccatgatgga tactttctcg 2160
   gcaggagcaa ggtgagatga caggagatcc tgccccggca cttcgcccaa tagcagccag 2220
   tcccttcccg cttcagtgac aacgtcgagc acagctgcgc aaggaacgcc cgtcgtggcc 2280
   agccacgata gccgcgctgc ctcgtcctgc agttcattca gggcaccgga caggtcggtc 2340
   ttgacaaaaa gaaccgggcg cccctgcgct gacagccgga acacggcggc atcagagcag 2400
   ccgattgtct gttgtgccca gtcatagccg aatagcctct ccacccaagc ggccggagaa 2460
   cctgcgtgca atccatcttg ttcaatcatg cgaaacgatc ctcatcctgt ctcttgatca 2520
   gatcttgatc ccctgcgcca tcagatcctt ggcggcaaga aagccatcca gtttactttg 2580
   cagggcttcc caaccttacc agagggcgcc ccagctggca attccggttc gcttgctgtc 2640
   cataaaaccg cccagtctag caactgttgg gaagggcgat cgtgtaatac gactcactat 2700
   agggcgaatt ggagct 2716
<210> 21
   <211> 2713
   <212> DNA
   <213> artificial sequence
<220>
   <223> Codon optimized GHRH- plasmid for chicken.
<400> 21
   tgtaatacga ctcactatag ggcgaattgg agctccaccg cggtggcggc cgtccgccct     60
   cggcaccatc ctcacgacac ccaaatatgg cgacgggtga ggaatggtgg ggagttattt    120
   ttagagcggt gaggaaggtg ggcaggcagc aggtgttggc gctctaaaaa taactcccgg    180
   gagttatttt tagagcggag gaatggtgga cacccaaata tggcgacggt tcctcacccg    240
   tcgccatatt tgggtgtccg ccctcggccg gggccgcatt cctgggggcc gggcggtgct    300
   cccgcccgcc tcgataaaag gctccggggc cggcggcggc ccacgagcta cccggaggag    360
   cgggaggcgc caagcggatc ccaaggccca actccccgaa ccactcaggg tcctgtggac    420
   agctcaccta gctgccatgg ccctgtgggt gttctttgtg ctgctgaccc tgacctccgg    480
   aagccactgc agcctgccac ccagcccacc cttccgcgtc aggcgccacg ccgacggcat    540
   cttcagcaag gcctaccgca agctcctggg ccagctgagc gcacgcaact acctgcacag    600
   cctgatggcc aagcgcgtgg gcagcggact gggagacgag gccgagcccc tgagctgata    660
   agcttatcgg ggtggcatcc ctgtgacccc tccccagtgc ctctcctggc cctggaagtt    720
   gccactccag tgcccaccag ccttgtccta ataaaattaa gttgcatcat tttgtctgac    780
   taggtgtcct tctataatat tatggggtgg aggggggtgg tatggagcaa ggggcaagtt    840
   gggaagacaa cctgtagggc tcgagggggg gcccggtacc agcttttgtt ccctttagtg    900
   agggttaatt tcgagcttgg tcttccgctt cctcgctcac tgactcgctg cgctcggtcg    960
   ttcggctgcg gcgagcggta tcagctcact caaaggcggt aatacggtta tccacagaat   1020
   caggggataa cgcaggaaag aacatgtgag caaaaggcca gcaaaaggcc aggaaccgta   1080
   aaaaggccgc gttgctggcg tttttccata ggctccgccc ccctgacgag catcacaaaa   1140
   atcgacgctc aagtcagagg tggcgaaacc cgacaggact ataaagatac caggcgtttc   1200
   cccctggaag ctccctcgtg cgctctcctg ttccgaccct gccgcttacc ggatacctgt   1260
   ccgcctttct cccttcggga agcgtggcgc tttctcatag ctcacgctgt aggtatctca   1320
   gttcggtgta ggtcgttcgc tccaagctgg gctgtgtgca cgaacccccc gttcagcccg   1380
   accgctgcgc cttatccggt aactatcgtc ttgagtccaa cccggtaaga cacgacttat   1440
   cgccactggc agcagccact ggtaacagga ttagcagagc gaggtatgta ggcggtgcta   1500
   cagagttctt gaagtggtgg cctaactacg gctacactag aagaacagta tttggtatct   1560
   gcgctctgct gaagccagtt accttcggaa aaagagttgg tagctcttga tccggcaaac   1620
   aaaccaccgc tggtagcggt ggtttttttg tttgcaagca gcagattacg cgcagaaaaa   1680
   aaggatctca agaagatcct ttgatctttt ctacggggct agcgcttaga agaactcatc   1740
   cagcagacgg tagaatgcaa tacgttgaga gtctggagct gcaataccat acagaaccag   1800
   gaaacggtca gcccattcac cacccagttc ctctgcaatg tcacgggtag ccagtgcaat   1860
   gtcctggtaa cggtctgcaa cacccagacg accacagtca atgaaaccag agaaacgacc   1920
   attctcaacc atgatgttcg gcaggcatgc atcaccatga gtaactacca ggtcctcacc   1980
   atccggcata cgagctttca gacgtgcaaa cagttcagcc ggtgccagac cctgatgttc   2040
   ctcatccagg tcatcctggt caaccagacc tgcttccata cgggtacgag cacgttcaat   2100
   acgatgtttt gcctggtggt caaacggaca ggtagctggg tccagggtgt gcagacgacg   2160
   cattgcatca gccatgatag aaactttctc tgccggagcc aggtgagaag acagcaggtc   2220
   ctgacccgga acttcaccca gcagcagcca gtcacgacca gcttcagtaa ctacatccag   2280
   aactgcagca cacggaacac cagtggttgc cagccaagac agacgagctg cttcatcctg   2340
   cagttcattc agagcaccag acaggtcagt tttaacaaac agaactggac gaccctgtgc   2400
   agacagacgg aaaacagctg catcagagca accaatggtc tgctgtgccc agtcataacc   2460
   aaacagacgt tcaacccagg ctgccggaga acctgcatgc agaccatcct gttcaatcat   2520
   gcgaaacgat cctcatcctg tctcttgatc agatcttgat cccctgcgcc atcagatcct   2580
   tggcggcaag aaagccatcc agtttacttt gcagggcttc ccaaccttac cagagggcgc   2640
   cccagctggc aattccggtt cgcttgctgt ccataaaacc gcccagtcta gcaactgttg   2700
   ggaagggcga tcg                                                      2713
<210> 22
   <211> 40
   <212> PRT
   <213> artificial sequence
<220>
   <223> Modified AA sequence for GHRH
<400> 22
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Ser Asn Gln Glu Arg Gly Ala
           35                  40
<210> 23
   <211> 40
   <212> PRT
   <213> artificial sequence
<220>
   <223> This is an amino acid sequence for Dog-GHRH
<400> 23
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Arg Glu Gln Gly Ala
           35                  40
<210> 24
   <211> 40
   <212> PRT
   <213> Artificial Sequence
<220>
   <223> This is an amino acid sequence for Cat-GHRH
<400> 24
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Gln Glu Gln Gly Ala
           35                  40

## Description

### BACKGROUND

This invention pertains to compositions for the treatment of kidney failure, treatment of anemia, and other conditions commonly associated with chronic kidney failure in a subject. The compositions are growth hormone releasing hormone ("GHRH") compositions, or compositions comprising an isolated nucleic acid molecule that encodes GHRH or functional biological equivalent.

Kidney failure and its complications, such as anemia, decreased life expectancy, and other conditions, can be related to a primary kidney disease, such as glomerulonephritis or pyelonephritis or are a consequence of a long-term chronic disease as cancer, heart failure, diabetes or severe allergic reactions. This invention relates to a plasmid-mediated supplementation for:
(1) Treatment of renal failure;
(2) Treatment of complications of renal failure, such as anemia and wasting;
(3) Increased survival and extending life expectancy for subjects with renal failure; and
(4) Improved welfare for subjects with renal failure.

**Kidney failure:** The predicted increase in the number of people with kidney failure and end-stage renal disease places an enormous burden on the healthcare provider system (Hostetter and Lising, 2002). In order to reduce this burden, strategies are needed to improve the detection of kidney disease, and preventative measures must be targeted at those at greatest risk of disease (Crook et al., 2002). Important risk factors include hypertension, diabetes, obesity and cancer (A1 Suwaidi et al., 2002; Nampoory et al., 2002). Serum creatinine, proteinuria, and microalbuminuria as early detection markers of disease are important, but treatments that could delay or prevent kidney failure could be of significant benefit for patients and the medical system (LeBrun et al., 2000; Sakhuja et al., 2000).

Recombinant human growth hormone ("GH") has proven effective in promoting growth in short children with chronic renal failure before and after renal transplantation. The action of GH and its mediator, insulin-like growth factor-I ("IGF-I") on body composition, protein, glucose and bone metabolism offers real therapeutic options for these patients. One might be the improvement of the catabolic state in adults with end-stage renal failure. In a few pilot studies and two placebo-controlled studies of 6 months duration, GH treatment in adults on dialysis showed clear anabolic effects resulting in a significant increase in lean body mass (Wuhl and Schaefer, 2002).

Chronic renal failure ("CRF") has limited therapeutic options for both humans and companion animals (e.g. pets). To date, three strategies help delay rather than treat chronic kidney disease progression: early identification of patients, modification of risk factors, and implementation of the best interventions. Unfortunately, the lack of efficient drug therapy for supporting CRF patients increases significantly their morbidity and mortality (Levin, 2001). Chronic dialysis or kidney transplant are the most common forms of treatment for humans. The proportion of older patients accepted for dialysis is increasing every year both in the U.S. and abroad. Of the two treatment modalities for end-stage renal disease, i.e. dialysis and transplantation, the latter offers more freedom and is associated with better clinical outcome (Rao, 2002). Unfortunately, it has become increasingly important to offer the kidney transplant to only those who have no significant co-morbid conditions or other high risk factors, so as to improve the odds of success after renal transplantation (Levin, 2001). Waiting lists are growing, and clinical conditions in some patients may deteriorate before a kidney becomes available (Braun, 2002).

For pets, most studies are focusing on nutrition and dietary changes as the only available current therapy. Ad libitum feeding and increased ash intake were associated with increased odds of CRF. Increased dietary fiber, magnesium, protein and sodium were associated with decreased odds of CRF (Hughes et al., 2002; Polzin et al., 2000). Other data suggest that feeding a diet specifically formulated to meet the needs of cats with CRF, together with phosphate binding drugs if required, controls hyperphosphataemia and secondary renal hyperparathyroidism, and is associated with an increased survival time (Elliott et al., 2000). Dietary modifications are beneficial in minimizing extra-renal manifestations of uremia and mortality rate in dogs with mild and moderate spontaneous CRF. Results are consistent with the hypothesis that delay in development of uremic crises and associated mortality rate in dogs fed renal food is associated, at least in part, with a reduction in the rate of progression of renal failure (Jacob et al., 2002). In a small number of cases of kidney transplant in dogs, the median survival time post-intervention was 8 months (Mathews et al., 2000).

**Anemia:** Anemia refers to a condition in which there is a reduction of the number or volume of red blood corpuscles or of the total amount of hemoglobin in the bloodstream, resulting in paleness and generalized weakness of the subject. The production of red blood cells in mammals is known as erythropoiesis. Erythropoiesis is primarily controlled by erythropoietin ("EPO"), an acidic glycoprotein. EPO stimulates the production of new erythrocytes to replace those lost to the aging process. Additionally, EPO production is stimulated under conditions of hypoxia, wherein the oxygen supply to the tissues is reduced below normal physiological levels despite adequate perfusion of the tissue by blood. Hypoxia may be caused by hemorrhaging, radiation-induced erythrocyte destruction, various anemias, high altitude, or long periods of unconsciousness. In response to tissues undergoing hypoxic stress, EPO will increase red blood cell production by stimulating the conversion of primitive precursor cells in the bone marrow into proerythroblasts that subsequently mature, synthesize hemoglobin and are released into the circulation as red blood cells.

EPO is normally present in low concentrations in plasma, where it is sufficient to maintain equilibrium between normal blood cell loss (*i.e.*, through aging) and red blood cell production. Anemia is a decrease in red blood cell mass caused by decreased production or increased destruction of red blood cells. EPO supplementation is currently used for treatment of the anemias associated with different diseases, such as end-stage renal failure (Cremagnani et al., 1993b; Diez et al., 1996b) and acquired immunodeficiency syndrome ("AIDS") (Sowade et al., 1998), particularly in subjects who are being treated with zidovudine ("AZT"). EPO is also used for amelioration of the anemia associated with cancer chemotherapy (Vansteenkiste et al., 2002).

There is a significant body of data that suggests that anemia in patients with CRF is also correlated with unfavorable changes in the GH axis. Short-term studies have proved that correction of anemia with recombinant human erythropoietin ("rhEPO") therapy is accompanied by several changes in GH secretion in uremic patients on dialysis (Diez et al., 1996a). Long-term treatment with rhEPO is associated with complex and profound effects on somatotrope cell function, characterized by diverse effects on GH responses to stimuli that release GH through different mechanisms. Some of these rhEPO-induced alterations in somatotrope function are dependent on the duration of treatment. For instance, correction of the anemia is accompanied by a clear increase in the area under the curve and the area above the baseline of GH secretion in response to GHRH stimulation. These changes are statistically significant after 3 and 6 months of therapy, although no changes are observed at 12 months (Diez et al., 1999b).

Another group of anemic disorders, each of which results from an inherited abnormality in globin production, is termed the hemoglobinopathies. Hemoglobinopathies include a spectrum of disorders that can be classified broadly into two types. The first types are those that result from an inherited structural alteration in one of the globin chains, for example, sickle cell anemia. These disorders give rise to the production of abnormal hemoglobin molecules (Papassotiriou et al., 2000). The second major subdivision of hemoglobinopathies, the thalassemias, results from inherited defects in the rate of synthesis of one or more of the globin chains. This causes ineffective erythropoiesis, hemolysis, and varying degrees of anemia due to the inadequate production of red blood cells. Accordingly, EPO can be used in the treatment of anemias, for example, hemoglobinopathies that are characterized by low or defective red blood cell production and/or increased red blood cell destruction (Makis et al., 2001; Payen et al., 2001).

Additional research has indicated that anemic patients with panhypopituitarism, a condition in which hemoglobin ("Hb") concentration remained as low as 11.0 g/dl in spite of appropriate replacement with thyroid and adrenocortical hormones, who were treated with recombinant human GH experienced an increase in EPO levels (Sohmiya and Kato, 2000). Recombinant human GH was constantly infused subcutaneously for 12 months, which caused the plasma EPO levels to nearly double, with a concomitant increase of Hb concentration. When the administration of human GH was interrupted, both plasma EPO levels and Hb concentrations decreased. There was a close correlation between plasma GH and EPO levels before and during the human GH administration. Plasma GH levels were well correlated with Hb concentrations before and during human GH administration. Plasma IGF-I levels were also correlated with Hb concentrations, but not with plasma EPO levels.

U.S. Patents No. 5,846,528 ("the '528 patent") and 6,274,158 ("the '158 patent") teach that conditions of anemia can be treated by deliberately increasing EPO. In addition, the '528 patent teaches the use of recombinant adeno-associated virus ("AAV") virions for delivery of DNA molecules encoding EPO to muscle cells and tissue in the treatment of anemia. The '528 patent shows a direct *in vivo* injection of recombinant AAV virions into muscle tissue (*e.g.*, by intramuscular injection), and *in vitro* transduction of muscle cells that can be subsequently introduced into a subject for treatment. Thus, a sustained high-level expression of a delivered nucleotide sequence encoding EPO results, whereby *in vivo* secretion from transduced muscle cells allows systemic delivery. The '158 patent teaches the use of the subcutaneous, intravenous or oral administration of recombinant human EPO as a hemostatic agent for the treatment or prevention of bleeding from any organ or body part involved with benign or malignant lesions, surgical trauma, non-healing or difficult to treat lesions, or radiation injury.

In brief, anemia can be caused by a specific disease, environmental factors, or the effects of a disease treatment. As discussed, circulating levels of EPO can be increased directly (*e.g.* injections of recombinant EPO) or indirectly (*e.g.* injections of recombinant GH). Although not wanting to be bound by theory, the related art suggests that anemic conditions can be successfully treated by methods or compounds capable of increasing the circulating levels of EPO. However, a skilled artisan recognizes that biological systems are immeasurably complex, and the ability to accurately predict which methods or compounds will elicit a specific biological response is outside the realm of a skilled artesian. Only through diligent laboratory experiments can insight into compounds or methods to treat anemia be discovered.

**Wasting:** Wasting of a subject can be defined as decreased body weight of at least 5-10% of the minimum ideal weight of the individual, characterized by significant loss of both adipose tissue and muscle mass, which makes weight gain especially difficult for patients with a progressive disease (*e.g.* cancer, AIDS *etc.).* Wasting or cachexia is a classic clinical phenomenon that evokes historical images of sickbeds and patients with "consumption." It simply means "poor condition" in Greek. Accelerated loss of skeletal muscle can occur in a setting of late stage kidney failure, cancer, AIDS, or tuberculosis, as well as other chronic conditions (Barber et al., 1999; Weinroth et al., 1995). Other clinical manifestations include anorexia, muscle wasting, and/or loss of adipose tissue and fatigue, which results in poor performance status (Davis and Dickerson, 2000). Because weight loss and a poor performance status lead to a poor prognosis, wasting can become the direct cause of death. In contrast to simple starvation, the weight loss cannot be adequately treated with aggressive feeding. The weight loss therefore cannot be entirely attributed to poor intake, but is also a result of increased basal energy expenditure.

Malnutrition and wasting are important determinants of morbidity and mortality in patients with chronic renal failure on dialysis. Even patients with a relatively modest degree of chronic renal insufficiency are characterized by reduced lean body mass, bone mineral content, and basal energy expenditure (O'Sullivan et al., 2002). Furthermore, semi-starvation, reduced physical activity, and aging are external factors possibly confounding a direct relationship between the primary organ impairments and alterations in peripheral skeletal muscle and exercise capacity (Franssen et al., 2002; Hansen et al., 2000). Although not wanting to be bound by theory, cytokine release and/or activation and liberation is postulated to be responsible for the wasting syndrome. The related art teaches that many agents have been evaluated for treatment of wasting, with only modest benefit obtained from progestational agents (Barber et al., 1999; Nelson, 2001). In contrast, recombinant GH, IGF-I, and IGF-binding protein 3 ("IGFBP-3") therapies are effective in producing a benefit in cachexia of different causes (Bartlett et al., 1994; Welle, 1998). These hormones may be useful anabolic agents to counteract muscle wasting under conditions including surgical stress, renal failure, muscular dystrophy, glucocorticoid administration and HIV infection (Welle, 1998). Thus, the related art suggests that wasting may be treated by methods or compounds that increase the circulating levels of GH, IGF-I or IGFBP-3 (Chen et al., 2001). Unfortunately, the complexity of biological systems makes it impossible to accurately predict what methods or compounds will elicit a specific biological response. Thus, only through meticulous laboratory experiments can an insight to useful compounds or methods to treat wasting be elucidated by one skilled in the art.

**Growth Hormone ("GH") and Immune Function:** The central role of GH is controlling somatic growth in humans and other vertebrates, and the physiologically relevant pathways regulating GH secretion from the pituitary are well known. The GH production pathway is composed of a series of interdependent genes whose products are required for normal growth. The GH pathway genes include: (1) ligands, such as GH and IGF-I; (2) transcription factors such as prophet of pit-1, or prop-1, and pit-1: (3) agonists and antagonists, such as growth hormone releasing hormone ("GHRH") and somatostatin ("SS"), respectively; and (4) receptors, such as GHRH receptor ("GHRH-R") and the GH receptor ("GH-R"). These genes are expressed in different organs and tissues, including the hypothalamus, pituitary, liver, and bone. Effective and regulated expression of the GH pathway is essential for optimal linear growth, as well as homeostasis of carbohydrate, protein, and fat metabolism. GH synthesis and secretion from the anterior pituitary is stimulated by GHRH and inhibited by somatostatin, both hypothalamic hormones. GH increases production of IGF-I, primarily in the liver, and other target organs. IGF-I and GH, in turn, feedback on the hypothalamus and pituitary to inhibit GHRH and GH release. GH elicits both direct and indirect actions on peripheral tissues, the indirect effects being mediated mainly by IGF-I.

The immune function is modulated by IGF-I, which has two major effects on B cell development: potentiation and maturation, and as a B-cell proliferation cofactor that works together with interlukin-7 ("IL-7"). These activities were identified through the use of anti-IGF-I antibodies, antisense sequences to IGF-I, and the use of recombinant IGF-I to substitute for the activity. There is evidence that macrophages are a rich source of IGF-I. The treatment of mice with recombinant IGF-I confirmed these observations as it increased the number of pre-B and mature B cells in bone marrow (Jardieu et al., 1994). The mature B cell remained sensitive to IGF-I as immunoglobulin production was also stimulated by IGF-I *in vitro* and *in vivo* (Robbins et al., 1994).

The production of recombinant proteins in the last 2 decades provided a useful tool for the treatment of many diverse conditions. For example, recombinant proteins were used to treat GH-deficiencies in short stature children. They have also been used as anabolic agents in burn, sepsis, and AIDES patients. However, resistance to GH action has been reported in malnutrition and infection. GH replacement therapy is widely used clinically, with beneficial effects, but therapy is associated with several disadvantages: GH must be administered subcutaneously or intramuscularly once a day to three times a week for months, or usually years; insulin resistance and impaired glucose tolerance are developed; and bone epiphysis growth and closure in pediatric patients is accelerated (Blethen and MacGillivray, 1997; Blethen and Rundle, 1996).

In contrast, essentially no side effects have been reported for recombinant GHRH therapies. Extracranially secreted GHRH, as mature peptide or truncated molecules (as seen with pancreatic islet cell tumors and variously located carcinoids) are often biologically active and can even produce acromegaly (Esch et al., 1982; Thorner et al., 1984). Administration of recombinant GHRH to GH-deficient children or adult humans augments IGF-I levels, increases GH secretion proportionally to the GHRH dose, yet still invokes a response to bolus doses of recombinant GHRH (Bercu et al., 1997). Thus, GHRH administration represents a more physiological alternative of increasing subnormal GH and IGF-I levels (Corpas et al., 1993).

GH is released in a distinctive pulsatile pattern that has profound importance for its biological activity (Argente et al., 1996). Secretion of GH is stimulated by GHRH and inhibited by somatostatin and both hypothalamic hormones (Thorner et al., 1995). GH pulses are a result of GHRH secretion that is associated with a diminution or withdrawal of somatostatin secretion. In addition, the pulse generator mechanism is timed by GH-negative feedback. The endogenous rhythm of GH secretion becomes entrained to the imposed rhythm of exogenous GH administration. Effective and regulated expression of the GH and IGF-I pathway is essential for optimal linear growth, homeostasis of carbohydrate, protein, and fat metabolism, and for providing a positive nitrogen balance (Murray and Shalet, 2000). Numerous studies in humans, sheep or pigs showed that continuous infusion with recombinant GHRH protein restores the normal GH pattern without desensitizing GHRH receptors or depleting GH supplies as this system is capable of feed-back regulation, which is abolished in the GH. therapies (Dubreuil et al., 1990; Vance, 1990; Vance et al., 1985). Although recombinant GHRH protein therapy entrains and stimulates normal cyclical GH secretion with virtually no side effects, the short half life of GHRH *in vivo* requires frequent (one to three times a day) intravenous, subcutaneous or intranasal (requiring 300-fold higher dose) administration. Thus, as a chronic treatment, GHRH administration is not practical.

Wild-type GHRH has a relatively short half-life in the circulatory system, both in humans (Frohman et al., 1984) and in farm animals. After 60 minutes of incubation in plasma, 95% of the GHRH(1-44)NH2 is degraded, while incubation of the shorter (1-40)OH form of the hormone, under similar conditions, shows only a 77% degradation of the peptide after 60 minutes of incubation (Frohman et al., 1989). Incorporation of cDNA coding for a particular protease-resistant GHRH analog in a therapeutic nucleic acid vector results in a molecule with a longer half-life in serum, increased potency, and provides greater GH release in plasmid-injected animals (Draghia-Akli et al., 1999), herein incorporated by reference). Mutagenesis via amino acid replacement of protease sensitive amino acids prolongs the serum half-life of the GHRH molecule. Furthermore, the enhancement of biological activity of GHRH is achieved by using super-active analogs that may increase its binding affinity to specific receptors (Draghia-Akli et al., 1999).

Extracranially secreted GHRH, as processed protein species GHRH(1-40) hydroxy or GHRH(1-44) amide or even as shorter truncated molecules, are biological active (Thorner et al., 1984). It has been reported that a low level of GHRH (100 pg/ml) in the blood supply stimulates GH secretion (Corpas et al., 1993). Direct plasmid DNA gene transfer is currently the basis of many emerging nucleic acid therapy strategies and thus does not require viral genes or lipid particles (Aihara and Miyazaki, 1998; Muramatsu et al., 2001). Skeletal muscle is target tissue, because muscle fiber has a long life span and can be transduced by circular DNA plasmids that express over months or years in an immunocompetent host (Davis et al., 1993; Tripathy et al., 1996). Previous reports demonstrated that human GHRH cDNA could be delivered to muscle by an injectable myogenic expression vector in mice where it transiently stimulated GH secretion to a modes extent over a period of two weeks (Draghia-Akli et al., 1997).

Administering novel GHRH analog proteins (U.S. Pat Nos. 5,847,066; 5846,936; 5,792,747; 5,776,901; 5,696,089; 5,486,505; 5,137,872; 5,084,442, 5,036,045; 5,023,322; 4,839,344; 4,410,512, RE33,699) or synthetic or naturally occurring peptide fragments of GHRH (U.S. Pat. Nos. 4,833,166; 4,228,158; 4,228,156; 4,226,857; 4,224,316; 4,223,021; 4,223,020; 4,223, 019) for the purpose of increasing release of growth hormone have been reported. A GHRH analog containing the following mutations have been reported (U.S. Patent No. 5,846,936): Tyr at position 1 to His; Ala at position 2 to Val, Leu, or others; Asn at position 8 to Gln, Ser, or Thr; Gly at position 15 to Ala or Leu; Met at position 27 to Nle or Leu; and Ser at position 28 to Asn. The GHRH analog is the subject of U.S. Patent Application Serial No. 09/624,268 ("the '268 patent application"), which teaches application of a GHRH analog containing mutations that improve the ability to elicit the release of growth hormone. In addition, the '268 patent application relates to the treatment of growth deficiencies; the improvement of growth performance; the stimulation of production of GH in an animal at a greater level than that associated with normal growth; and the enhancement of growth utilizing the administration of a distict GHRH analog and is herein incorporated by reference.

U.S. Patent No. 5,061,690 is directed toward increasing both birth weight and milk production by supplying to pregnant female mammals an effective amount of human GHRH or one of it analogs for 10-20 days. Application of the analogs lasts only throughout the lactation period. However, multiple administrations are presented, and there is no disclosure regarding administration of the growth hormone releasing hormone (or factor) as a DNA molecule, such as with plasmid mediated therapeutic techniques.

U.S. Patents No. 5,134,120 ("the '120 patent") and 5,292,721 ("the '721 patent") teach that by deliberately increasing GH in swine during the last 2 weeks of pregnancy and first 3 weeks of lactation resulted in the newborn piglets having marked enhancement of the ability to maintain plasma concentrations of glucose and free fatty acids when fasted after birth. In addition, the '120 and `721 patents teach that treatment of the sow during lactation results in increased milk fat in the colostrum and an increased milk yield. These effects are important in enhancing survivability of newborn pigs and weight gain prior to weaning. However the `120 and '721 patents provide no teachings regarding administration of the GHRH as a DNA form.

Previous studies have also shown that GH (Ziegler et al., 1991) and IGF-I improve renal function in patients with renal failure (Vijayan et al., 1999). GHRH stimulates IGF-I, and there is data to suggest that IGF-I rather than erythropoietin is the primary mediator of erythropoiesis during catabolic states and in uremic patients (Urena et al., 1992), and can induce a proportional increase in body mass and oxygen transport capacity (Kurtz et al., 1990). The increase in hemoglobin and hematocrit confirms the *in vivo* erythropoietic growth-promoting effects of GH that is also observed during GH treatment in GH-deficient children or adults (Christ et al., 1997; Valerio et al., 1997). At the same time, however, GH administration to normal Beagle dogs over 14 weeks of age produced a *dose-related normochromic, normocytic and non-regenerative anemia* (Prahalada et al., 1998).

Erythroid cell number is primarily regulated by erythropoietin ("EPO") but is impacted by many growth factors. For example, hypophysectomized rats show low blood cell counts for erythroid, myeloid, and lymphoid cells, and there is extensive literature showing effects of both GH and IGF-I on all hematopoietic lineages (Claustres et al., 1987; Kurtz et al., 1982; Kurtz et al., 1990). In polycytemia vera, patients present increased sensitivity of erythroid progenitor cells to IGF-I, elevated level of IGF-binding protein 1 and consequently overproduction of red blood cells (Correa et al., 1994; Mirza et al., 1997). After the treatment with human GH, plasma EPO levels double, with a concomitant increase of Hb concentration to normal levels. When the administration of GH is interrupted, both plasma EPO levels and Hb concentrations decrease.

IGF-I, the downstream effector of GHRH, is shown to maintain glomerular filtration via direct action on the glomerular vasculature and to accelerate tubular regeneration by enhancing DNA synthesis in proximal tubule cells (Hammerman, 1999; Vijayan et al., 1999). Several studies show that IGF-I delivered as a recombinant protein can prevent the development of acute renal failure in susceptible individuals, can accelerate recovery from established acute renal failure and can enhance kidney function in the setting of end-stage renal disease (Fervenza et al., 1998; Miller and Rabkin, 1997).

**Gene Delivery and *in vivo* Expression:** Recently, the delivery of specific genes to somatic tissue in a manner that can correct inborn or acquired deficiencies and imbalances was proven to be possible (Herzog et al., 2001; Song et al., 2001; Vilquin et al., 2001). Gene-based drug delivery offers a number of advantages over the administration of recombinant proteins. These advantages include the conservation of native protein structure, improved biological activity, avoidance of systemic toxicities, and avoidance of infectious and toxic impurities. In addition, nucleic acid vector therapy allows for prolonged exposure to the protein in the therapeutic range, because the newly secreted protein is present continuously in the blood circulation. In a few cases, the relatively low expression levels achieved after simple plasmid injection are sufficient to reach physiologically acceptable levels of bioactivity of secreted peptides (Danko and Wolff, 1994; Tsurumi et al., 1996).

The primary limitation of using recombinant protein is the limited availability of protein after each administration. Nucleic acid vector therapy using injectable DNA plasmid vectors overcomes this, because a single injection into the patient's skeletal muscle permits physiologic expression for extensive periods of time (WO 99/05300 and WO 01/06988). Injection of the vectors promotes the production of enzymes and hormones in animals in a manner that more closely mimics the natural process.

In a plasmid-based expression system, a non-viral gene vector may be composed of a synthetic gene delivery system in addition to the nucleic acid encoding a therapeutic gene product. In this way, the risks associated with the use of most viral vectors can be avoided. The non-viral expression vector products generally have low toxicity due to the use of "species-specific" components for gene delivery, which minimizes the risks of immunogenicity generally associated with viral vectors. Additionally, no integration of plasmid sequences into host chromosomes has been reported *in vivo* to date, so that this type of nucleic acid vector therapy should neither activate oncogenes nor inactivate tumor suppressor genes. As episomal systems residing outside the chromosomes, plasmids have defined pharmacokinetics and elimination profiles, leading to a finite duration of gene expression in target tissues.

Among the non-viral techniques for gene transfer *in vivo*, the direct injection of plasmid DNA into muscle is simple, inexpensive, and safe. However, the use of directly injectable plasmids has been limited in the past. The inefficient DNA uptake into muscle fibers after simple direct injection has led to relatively low expression levels (Prentice et al., 1994; Wells et al., 1997). In addition, the duration of transgene expression has been short (Wolff et al., 1990). Most successful previous clinical applications have been confined to vaccines (Danko and Wolff, 1994; Tsurumi et al., 1996).

Efforts have been made to enhance the delivery of plasmid DNA to cells by physical means including electroporation, sonoporation, and pressure. Recently, significant progress has been obtained using electroporation to enhance plasmid delivery *in vivo.* Administration by electroporation involves the application of a pulsed electric field to create transient pores in the cellular membrane without causing permanent damage to the cell. It thereby allows for the introduction of exogenous molecules (Smith and Nordstrom, 2000). By adjusting the electrical pulse generated by an electroporetic system, nucleic acid molecules can travel through passageways or pores in the cell that are created during the procedure. U.S. Patent 5,704,908 describes an electroporation apparatus for delivering molecules to cells at a selected location within a cavity in the body of a patient. These pulse voltage injection devices are also described in U.S. Patent Nos. 5,439,440 and 5,702,304, and PCT WO 96/12520, 96/12006, 95/19805, and 97/07826.

Electroporation has been used very successfully to transfect tumor cells after injection of plasmid (Lucas et al., 2002; Matsubara et al., 2001) or to deliver the anti-tumor drug bleomycin to cutaneous and subcutaneous tumors in humans (Gehl et al., 1998; Heller et al., 1996). Electroporation also has been extensively used in mice (Lesbordes et al., 2002; Lucas et al., 2001; Vilquin et al., 2001), rats (Terada et al., 2001; Yasui et al., 2001), and dogs (Fewell et al., 2001) to deliver therapeutic genes that encode for a variety of hormones, cytokines or enzymes.

In some instances, the effects of the electroporation-mediated treatments are dramatic. Calcitonin gene-related peptide ("CGRP") gene transfer selectively suppresses the pro-inflammatory Th1 subsets and promotes anti-inflammatory Th2 subsets. The treatment significantly decreases morbidity of diabetes, ameliorates hyperglycemia and insulin deficiency, and inhibits lymphocyte infiltration into the islets, indicating the protection of beta cells against autoimmune destruction (Sun et al., 2003). In sub-totally (5/6) nephrectomized rats, hepatocyte growth factor (HGF) gene transfer by electroporation into skeletal muscle was effective against morphologic injury. Treated rats showed better growth in body weight than untreated rats. Histologic changes such as glomerulosclerosis and interstitial fibrosis are significantly ameliorated by HGF gene transfer compared with untreated rats (Tanaka et al., 2002). Previous studies using GHRH showed that plasmid therapy with electroporation is scalable and represents a promising approach to induce production and regulated secretion of proteins in large animals and humans (Draghia-Akli et al., 1999; Draghia-Akli et al., 2002b).

The ability of electroporation to enhance plasmid uptake into the skeletal muscle has been well documented, as described above. In addition, plasmid formulated with poly-L-glutamate ("PLG") or polyvinylpyrolidone ("PVP") has been observed to increase plasmid transfection and consequently expression of the desired transgene. The anionic polymer sodium PLG enhances plasmid uptake at low plasmid concentrations, while reducing any possible tissue damage caused by the procedure. PLG is a stable compound and resistant to relatively high temperatures (Dolnik et al., 1993). PLG has been previously used to increase stability in vaccine preparations (Matsuo et al., 1994) without increasing their immunogenicity. It also has been used as an anti-toxin after antigen inhalation or exposure to ozone (Fryer and Jacoby, 1993). In addition, plasmid formulation with PLG or PVP has been observed to increase gene transfection and consequently expression to up to 10 fold in the skeletal muscle of mice, rats and dogs (Fewell et al., 2001; Mumper et al., 1998). PLG has been used to increase stability of anticancer drugs (Li et al., 2000) and as "glue" to close wounds or to prevent bleeding from tissues during wound and tissue repair (Otani et al., 1996; Otani et al., 1998).

Although there are references in the art directed to electroporation of eukaryotic cells with linear DNA (McNally et al., 1988; Neumann et al., 1982) (Toneguzzo et al., 1988) (Aratani et al., 1992; Nairn et al., 1993; Xie and Tsong, 1993; Yorifuji and Mikawa, 1990), these examples illustrate transfection into cell suspensions, cell cultures, and the like, and the transfected cells are not present in a somatic tissue.

U.S. Patent No. 4,956,288 is directed to methods for preparing recombinant host cells containing high copy number of a foreign DNA by electroporating a population of cells in the presence of the foreign DNA, culturing the cells, and killing the cells having a low copy number of the foreign DNA.

In summary, treatments for conditions associated with kidney failure, such as anemia, wasting, immune dysfunction, or other conditions commonly associated with kidney failure, are uneconomical and restricted in scope. Although it may be possible to treat these different disease conditions in a limited capacity utilizing recombinant protein technology, such treatments may have some significant drawbacks. Nucleic acid expression constructs that encode recombinant proteins are viable solutions to the problems of frequent injections and high cost of traditional recombinant therapy. The introduction of point mutations into the encoded recombinant proteins was a significant step forward in producing proteins that are more stable *in vivo* than the wild type counterparts. Each amino acid alteration in a given recombinant protein should be evaluated individually to accurately predict how changes in structure (*e.g.* amino-acid sequences) will lead to changes in function (*e.g.* increased or decreased stability) of a recombinant protein. Thus, the beneficial effects of nucleic acid expression constructs that encode expressed proteins have been ascertained through direct experimentation. There is a need in the art for expanded treatments for subjects with a disease by utilizing isolated nucleic acid expression constructs that are delivered into a subject and express stable therapeutic proteins *in vivo.*

### SUMMARY

The present invention pertains to compositions that are useful for plasmid mediated gene supplementation. The invention is directed to compositions for the treatment of renal failure and its complications, such as anemia, wasting, immune dysfunction, decreased life expectancy, decreased quality of life, and other conditions that can be related to a primary kidney disease, such as glomerulonephritis or pyelonephritis.

One embodiment of this invention comprises delivering an isolated nucleic acid expression construct that encodes a growth hormone releasing hormone ("GHRH") or functional biological equivalent thereof into a tissue, such as a muscle, of the subject. When this nucleic acid sequence is delivered into the specific cells of the subject, tissue specific expression of GHRH is achieved. The preferred method to deliver the nucleic acid sequence with the constitutive promoter and the encoding sequence of GHRH or the analog thereof is directly into the cells of the subject by the process of *in vivo* electroporation. Electroporation may involve externally supplied electrodes, or in the case of needles, internally supplied electrodes to aid in the inclusion of desired nucleotide sequences into the cells of a subject while the cells are within a tissue of the subject. In a particular embodiment, the cells of the subject are somatic cells, stem cells, or germ cells.

In specific embodiments, the isolated nucleic acid expression constructs are at least 90% identical to sequences listed in SeqID No.: 11, SeqID No.: 12, SeqID No.: 13, SeqID No.: 14, SeqID No.: 17, SeqID No.: 18, SeqID No.: 19, SeqID No.: 20, or SeqID No.: 21. The isolated nucleic acid expression construct of this invention is substantially free of a viral backbone. The isolated nucleic acid expression construct may further comprise a transfection-facilitating polypeptide, which is preferably a charged polypeptide and most preferably poly-L-glutamate.

After delivering the isolated nucleic acid expression construct into the tissues of the subject, preferably the muscle cells, expression of the encoded GHRH or functional biological equivalent thereof is initiated. The encoded GHRH is a biologically active polypeptide. The encoded functional biological equivalent of GHRH is a polypeptide that has been engineered to contain a distinct amino acid sequence while simultaneously having similar or improved biological activity when compared to the GHRH polypeptide. One embodiment of a specific encoded GHRH or functional biological equivalent thereof is at least 90% identical to the sequence of formula SeqID No.: 6. The GHRH, or functional biological equivalent that is encoded by the isolated nucleic acid expression construct and useful for this invention comprises an amino acid structure with a general sequence as follows:

-**X₁**-**X₂**=-DAIFTNSYRKVL-**X₃**-QLSARKLLQDI-**X₄-X₅**-RQQGE-**X₆**-NQE-**X₇**-GA-OH (SEQID No.: 6)

wherein: **X₁** is a D-or L-isomer of the aminoacid tyrosine ("Y") or histidine ("H"); **X₂** is a D-or L-isomer of the aminoacid alanine ("A"), valine ("V"), or isoleucine ("I"); **X₃** is a D- or L-isomer of the aminoacid alanine ("A") or glycine ("G"); **X₄** is a D-or L-isomer of the aminoacid methionine ("M") or leucine ("L"); **X₅** is a D-or L-isomer of the aminoacid serine ("S") or asparagine ("N"); **X₆** is a D-or L-isomer of the aminoacid arginine ("R") or serine ("S"); **X₇** is a D-or L-isomer of the aminoacid glutamine ("Q") or arginine ("R"); and combinations thereof. Specific examples of amino acid sequences for GHRH or functional biological equivalents that are useful for this invention are presented. In a specific embodiment, the encoded GHRH or functional biological equivalent thereof facilitates growth hormone ("GH") secretion in a subject that has received the isolated nucleic acid expression construct.

In additional embodiments, a recombinant GHRH polypeptide or functional biological equivalent thereof is delivered to the subject. The recombinant GHRH polypeptide is a biologically active polypeptide, while the functional biological equivalent of GHRH is a polypeptide that has been engineered to contain a distinct amino acid sequence while simultaneously having similar or improved biological activity when compared to the GHRH polypeptide. One embodiment of a specific GHRH or functional biological equivalent thereof is of the formula of SeqID No.: 6.

Specific embodiments of the present invention pertain to plasmid compositions of the invention for treating anemia, increasing total red blood cell mass in a subject, reversing wasting, reversing abnormal weight loss, treating immune dysfunction, and extending life expectancy of a subject with chronic kidney failure. In further specific embodiments, the subject is an animal.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

Figure 1 shows the amino acid sequence of GHRH and biological equivalents thereof.

Figure 2 shows the IGF-I levels in healthy dogs that were injected with different concentrations of the pSP-HV-GHRH plasmid.

Figure 3 shows serum IGF-I levels in normal healthy dogs treated with plasmid-mediated GHRH therapy. The results are presented as means ± SEM. P values versus baseline IGF-I levels are included for each group and time point.

Figure 4 shows the weight gain in healthy dogs that were injected with different concentrations of the pSP-HV-GHRH plasmid.

Figure 5 shows weights (in kilograms) in normal healthy dogs treated with plasmid-mediated GHRH therapy. The results are presented as means ± SEM. *P* values versus baseline weights are included for each group and time point.

Figure 6 shows hemoglobin and hematocrit ("PCV") in normal healthy dogs treated with plasmid-mediated GHRH therapy. The results are presented as means ± SEM. A. Least square mean analysis of hemoglobin for the dogs treated with 0.2 mg * *P* < 0.002, dogs treated with 0.6 mg, # *P* < 0.0001, and dogs treated with 1 mg, § *P* < 0.05. B. Least square mean analysis of PCV for the dogs treated with 0.2 mg * *P* < 0.01, dogs treated with 0.6 mg, # *P* < 0.0001, and dogs treated with 1 mg, § *P* < 0.025.

Figure 7 shows hematocrit ("PCV"), red blood cell count and mean red cell hemoglobin ("MCH") in cats with chronic renal failure treated with plasmid-mediated GHRH therapy. The results are presented as means ± SEM. A. Analysis of hematocrit ("PCV") for the GHRH-treated cats * *P* < 0.0007. B. Analysis of red blood cells for the GHRH-treated cats * *P* < 0.0006. C. Analysis of mean red cell hemoglobin ("MCH") for the GHRH-treated cats * *P* < 0.013.

Figure 8 shows albumin and total protein values in cats with chronic renal failure treated with plasmid-mediated GHRH therapy. The results are presented as means ± SEM. A. Analysis of albumin for the GHRH-treated cats * *P* < 0.00001. B. Analysis of total protein for the GHRH-treated cats * *P* < 0.00001.

Figure 9 shows hematocrit ("PCV") and hemoglobin in dogs with chronic renal failure treated with plasmid-mediated GHRH therapy. The results are presented as means ± SEM. A. Analysis of hematocrit ("PCV") for the GHRH-treated dogs * *P* < 0.06. B. Analysis of hemoglobin for the GHRH-treated dogs * *P* < 0.05.

Figure 10 shows blood urea nitrogen and creatinine values in dogs with chronic renal failure treated with plasmid-mediated GHRH therapy. The results are presented as means ± SEM. A. Analysis of blood urea nitrogen ("BUN") for the GHRH-treated dogs showed a 16% reduction in BUN. B. Analysis of creatinine for the GHRH-treated dogs showed a 17.6% decrease in creatinine levels.

Figure 11 shows IGF-I levels in cats and dogs with chronic renal failure treated with plasmid-mediated GHRH therapy. A. The results are presented as means ± SEM. A. 75% of GHRH-treated cats have increased IGF-I levels at 20-75 days after GHRH treatment (* *P* <0.05). B. 75% of GHRH-treated dogs have increased IGF-I levels at 20-75 days after GHRH treatment (* *P* <0.05).

Figure 12 shows circulating iron levels in dogs with chronic renal failure treated with plasmid-mediated GHRH therapy. The results are presented as means ± SEM. Circulating iron concentration is significantly improved in dogs treated with plasmid-mediated GHRH (* *P* <0.05).

### DETAILED DESCRIPTION

### Definitions

The term "a" or "an" as used herein in the specification may mean one or more. As used herein in the claim(s), when used in conjunction with the word "comprising", the words "a" or "an" may mean one or more than one. As used herein "another" may mean at least a second or more.

The term "abnormal weight loss," as used herein is defined as decreased body weight of at least 5-10% of the minimum ideal weight of the individual that is characterized by significant loss of both adipose tissue and muscle mass.

The term "AIDS therapy" as used herein refers to treatment of acquired immune deficiency syndrome ("AIDS") by any medical or physical means, including, but not limited to: antiretrovirals, nucleoside analogues, non-nucleoside reverse transcriptase inhibitors (NNRTIs), protease inhibitors, and/or other drugs used to boost the immune system.

The term "analog" as used herein includes any mutant of GHRH, or synthetic or naturally occurring peptide fragments of GHRH, such as HV-GHRH (SEQID No.: 1), TI-GHRH (SEQID No.: 2), TV-GHIZH (SEQID No.: 3), 15/27/28-GHRH (SEQID No.: 4), (1-44)NH₂ (SEQID No.: 5) or (1-40)OH (SEQID No.: 6) forms, or any shorter form to no less than (1-29) amino acids.

The term "anemia" as used herein refers to a condition in which there is a reduction of the number and/or volume of red blood corpuscles or of the total amount of hemoglobin in the bloodstream, resulting in paleness, generalized weakness, *etc*., of the subject.

The term "antiviral therapy" as used herein refers to a group of drugs that are of three main types, including: nucleoside analog drugs, protease (proteinase) inhibitor drugs, and non-nucleoside reverse-transcriptase inhibitor drugs (NNRTIs).

The term "baseline level" as used herein refers to a measurement, calculation, or level used as a basis for comparison of normal biological parameters to non-normal biological parameters in a specific species of the subject. For example the normal range or baseline values of serum creatinine in a dog are in the range of about 0.4-1.8mg/dl, wherein the normal range or baseline values of serum creatinine in a cat are in the range of about 0.8-2.3mg/dl. Some biological baseline level values are different from one species to another, but one of ordinary skill in the art is able to determine the normal baseline and values above or below baseline levels for symptoms of: renal failure, anemia, wasting and immune dysfunction.

The term "bodily fat proportion" as used herein is defined as the body fat mass divided by the total body weight.

The term "cachexia" as used herein is defined as the accelerated loss of skeletal muscle.

The term "cassette" as used herein is defined as one or more transgene expression vectors.

The term "cell-transfecting pulse" as used herein is defined as a transmission of a force which results in transfection of a vector, such as a linear DNA fragment, into a cell. In some embodiments, the force is from electricity, as in electroporation, or the force is from vascular pressure.

The term "coding region" as used herein refers to any portion of the DNA sequence that is transcribed into messenger RNA (mRNA) and then translated into a sequence of amino acids characteristic of a specific polypeptide.

The term "coding region" as used herein refers to any portion of the DNA sequence that is transcribed into messenger RNA (mRNA) and then translated into a sequence of amino acids characteristic of a specific polypeptide.

The term "delivery" or "delivering" as used herein is defined as a means of introducing a material into a tissue, a subject, a cell or any recipient, by means of chemical or biological process, injection, mixing, electroporation, sonoporation, or combination thereof, either under or without pressure.

The term "DNA fragment" or "nucleic acid expression construct" as used herein refers to a substantially double stranded DNA molecule. Although the fragment may be generated by any standard molecular biology means known in the art, in some embodiments the DNA fragment or expression construct is generated by restriction digestion of a parent DNA molecule. The terms "expression vector," "expression cassette," or "expression plasmid" can also be used interchangeably. Although the parent molecule may be any standard molecular biology DNA reagent, in some embodiments the parent DNA molecule is a plasmid. The term "chronically ill" as used herein is defined as patients with conditions such as chronic obstructive pulmonary disease, chronic heart failure, stroke, dementia, rehabilitation after hip fracture, chronic renal failure, rheumatoid arthritis, and multiple disorders in the elderly, with doctor visits and/or hospitalization once a month for at least two years.

The term "donor-subject" as used herein refers to any species of the animal kingdom wherein cells have been removed and maintained in a viable state for any period of time outside the subject.

The term "donor-cells" as used herein refers to any cells that have been removed and maintained in a viable state for any period of time outside the donor-subject.

The term "electroporation" as used herein refers to a method that utilized electric pulses to deliver a nucleic acid sequence into cells.

The terms "electrical pulse" and "electroporation" as used herein refer to the administration of an electrical current to a tissue or cell for the purpose of taking up a nucleic acid molecule into a cell. A skilled artisan recognizes that these terms are associated with the terms "pulsed electric field" "pulsed current device" and "pulse voltage device." A skilled artisan recognizes that the amount and duration of the electrical pulse is dependent on the tissue, size, and overall health of the recipient subject, and furthermore knows how to determine such parameters empirically.

The term "encoded GHRH" as used herein is a biologically active polypeptide of growth hormone releasing hormone.

The term "functional biological equivalent" of GHRH as used herein is a polypeptide that has a distinct amino acid sequence from a wild type GHRH polypeptide while simultaneously having similar or improved biological activity when compared to the GHRH polypeptide. The functional biological equivalent may be naturally occurring or it may be modified by an individual. A skilled artisan recognizes that the similar or improved biological activity as used herein refers to facilitating and/or releasing growth hormone or other pituitary hormones. A skilled artisan recognizes that in some embodiments the encoded functional biological equivalent of GHRH is a polypeptide that has been engineered to contain a distinct amino acid sequence while simultaneously having similar or improved biological activity when compared to the GHRH polypeptide. Methods known in the art to engineer such a sequence include site-directed mutagenesis.

The term "growth hormone" ("GH") as used herein is defined as a hormone that relates to growth and acts as a chemical messenger to exert its action on a target cell. The growth hormone may be released by the action of growth hormone releasing hormone.

The term "growth hormone releasing hormone" ("GHRH") as used herein is defined as a hormone that facilitates or stimulates release of growth hormone, and in a lesser extent other pituitary hormones, such as prolactin.

The term "heterologous nucleic acid sequence" as used herein is defined as a DNA sequence comprising differing regulatory and expression elements.

The term "immune dysfunction" as used herein refers to the abnormal, impaired, or incomplete functioning of a subject's immune system, as determined indirectly or directly by immune specific markers (*e.g.* IGF-I levels, or % lymphocytes).

The term "immunotherapy" as used herein refers to any treatment that promotes or enhances the body's immune system to build protective antibodies that will reduce the symptoms of a medical condition and/or lessen the need for medications.

The term "lean body mass" ("LBM") as used herein is defined as the mass of the body of an animal attributed to non-fat tissue such as muscle.

The term "life extension for the chronically ill" as used herein refers to an increase in the actual life expectancy for a subject that undertakes the treatment compared to a subject that did not have treatment.

The term "lymphopoiesis" as used herein is defined as the production of lymphocytes.

The term "kidney failure," or "renal failure," or "chronic renal failure" as used herein is defined as the abrupt or chronic decline in glomerular filtration rate resulting from ischemic or toxic injury to the kidney, or is secondary to a primary disease such as infection, glomerulonephritis, cardiac disease, or diabetes, and includes a decrease of glomerular capillary permeability, back-leak of glomerular filtrate, tubular obstruction, and intrarenal vasoconstriction.

The term "kidney failure therapy" as used herein refers to treatment of kidney failure by any medical or physical means, including, but not limited to immunotherapy, dialysis, kidney transplant and nutritional therapy.

The term "modified cells" as used herein is defined as the cells from a subject that have an additional nucleic acid sequence introduced into the cell.

The term "modified-donor-cells" as used herein refers to any donor-cells that have had a GHRH-encoding nucleic acid sequence delivered.

The term "nucleic acid expression construct" as used herein refers to any type of genetic construct comprising a nucleic acid coding for a RNA capable of being transcribed. The term "expression vector" can also be used interchangeably herein. In specific embodiments, the nucleic acid expression construct comprises: a promoter; a nucleotide sequence of interest; and a 3' untranslated region; wherein the promoter, the nucleotide sequence of interest, and the 3' untranslated region are operatively linked; and *in vivo* expression of the nucleotide sequence of interest is regulated by the promoter.

The term "operatively linked" as used herein refers to elements or structures in a nucleic acid sequence that are linked by operative ability and not physical location. The elements or structures are capable of, or characterized by accomplishing a desired operation. It is recognized by one of ordinary skill in the art that it is not necessary for elements or structures in a nucleic acid sequence to be in a tandem or adjacent order to be operatively linked.

The term "poly-L-glutamate ("PLG")" as used herein refers to a biodegradable polymer of L-glutamic acid that is suitable for use as a vector or adjuvant for DNA transfer into cells with or without electroporation.

The term "post-injection" as used herein refers to a time period following the introduction of a nucleic acid cassette that contains heterologous nucleic acid sequence encoding GHRH- or a biological equivalent thereof into the cells of the subject and allowing expression of the encoded gene to occur while the modified cells are within the living organism.

The term "plasmid" as used herein refers generally to a construction comprised of extra-chromosomal genetic material, usually of a circular duplex of DNA that can replicate independently of chromosomal DNA. Plasmids, or fragments thereof, may be used as vectors. Plasmids are double-stranded DNA molecule that occur or are derived from bacteria and (rarely) other microorganisms. However, mitochondrial and chloroplast DNA, yeast killer and other cases are commonly excluded.

The term "plasmid mediated gene supplementation" as used herein refers a method to allow a subject to have prolonged exposure to a therapeutic range of a therapeutic protein by utilizing a isolated nucleic acid expression construct *in vivo.*

The term "pulse voltage device," or "pulse voltage injection device" as used herein relates to an apparatus that is capable of causing or causes uptake of nucleic acid molecules into the cells of an organism by emitting a localized pulse of electricity to the cells. The cell membrane then destabilizes, forming passageways or pores. Conventional devices of this type are calibrated to allow one to select or adjust the desired voltage amplitude and the duration of the pulsed voltage. The primary importance of a pulse voltage device is the capability of the device to facilitate delivery of compositions of the invention, particularly linear DNA fragments, into the cells of the organism.

The term "plasmid backbone" as used herein refers to a sequence of DNA that typically contains a bacterial origin of replication, and a bacterial antibiotic selection gene, which are necessary for the specific growth of only the bacteria that are transformed with the proper plasmid. However, there are plasmids, called mini-circles, that lack both the antibiotic resistance gene and the origin of replication (Darquet et al., 1997; Darquet et al., 1999; Soubrier et al., 1999). The use of *in vitro* amplified expression plasmid DNA (i.e. non-viral expression systems) avoids the risks associated with viral vectors. The non-viral expression systems products generally have low toxicity due to the use of "species-specific" components for gene delivery, which minimizes the risks of immunogenicity generally associated with viral vectors. One aspect of the current invention is that the plasmid backbone does not contain viral nucleotide sequences.

The term "promoter" as used herein refers to a sequence of DNA that directs the transcription of a gene. A promoter may direct the transcription of a prokaryotic or eukaryotic gene. A promoter may be "inducible," initiating transcription in response to an inducing agent or, in contrast, a promoter may be "constitutive," whereby an inducing agent does not regulate the rate of transcription. A promoter may be regulated in a tissue-specific or tissue-preferred manner, such that it is only active in transcribing the operable linked coding region in a specific tissue type or types.

The term "replication element" as used herein comprises nucleic acid sequences that will lead to replication of a plasmid in a specified host. One skilled in the art of molecular biology will recognize that the replication element may include, but is not limited to a selectable marker gene promoter, a ribosomal binding site, a selectable marker gene sequence, and a origin of replication.

The term "residual linear plasmid backbone" as used herein comprises any fragment of the plasmid backbone that is left at the end of the process making the nucleic acid expression plasmid linear.

The term "recipient-subject" as used herein refers to any species of the animal kingdom wherein modified-donor-cells can be introduced from a donor-subject.

The term "red blood cell mass" ("RBC-mass") of a subject as used herein is determined using one of the three following tests: 1) Hematocrit: the percentage of red blood cells in plasma; 2) red blood cell ("RBC") count: the number of red blood cells in plasma; and 3) hemoglobin: the level of oxygen-carrying protein within the subjects' red blood cells.

The term "regulator protein" as used herein refers to any protein that can be used to control the expression of a gene.

The terms "subject" or "animal" as used herein refers to any species of the animal kingdom. In preferred embodiments, it refers more specifically to humans and domesticated animals used for: pets (*e.g.* cats, dogs, etc.); work (*e.g.* horses, etc.); food (cows, chicken, fish, lambs, pigs, etc); and all others known in the art.

The term "tissue" as used herein refers to a collection of similar cells and the intercellular substances surrounding them. A skilled artisan recognizes that a tissue is an aggregation of similarly specialized cells for the performance of a particular function. For the scope of the present invention, the term tissue does not refer to a cell line, a suspension of cells, or a culture of cells. In a specific embodiment, the tissue is electroporated *in vivo.* A skilled artisan recognizes that there are four basic tissues in the body: 1) epithelium; 2) connective tissues, including blood, bone, and cartilage; 3) muscle tissue; and 4) nerve tissue. In a specific embodiment, the methods and compositions are directed to transfer of linear DNA into a muscle tissue by electroporation.

The term "therapeutic element" as used herein comprises nucleic acid sequences that will lead to an *in vivo* expression of an encoded gene product. One skilled in the art of molecular biology will recognize that the therapeutic element may include, but is not limited to a promoter sequence, a transgene, a poly A sequence, or a 3' or 5' UTR.

The term "transfects" as used herein refers to introduction of a nucleic acid into a eukaryotic cell. In some embodiments, the cell is not a plant tissue or a yeast cell.

The term "vector" as used herein refers to any vehicle that delivers a nucleic acid into a cell or organism. Examples include plasmid vectors, viral vectors, liposomes, or cationic lipids.

The term "wasting" as used herein is defined as decreased body weight characterized by significant loss of both adipose tissue and muscle mass that makes weight gain especially difficult for patients with progressive diseases, such as kidney failure, cancer or AIDS. Wasting can be related to the disease itself or the effects of its treatment, or both.

The term "viral backbone" as used herein refers to a nucleic acid sequence that does not contain a promoter, a gene, and a 3' poly A signal or an untranslated region, but contains elements including, but not limited to, site-specific genomic integration Rep, inverted terminal repeats ("ITRs"), and the binding site for the tRNA primer for reverse transcription. It may also contain a nucleic acid sequence component that induces a viral immunogenicity response when inserted in vivo, allow integration, affect specificity and activity of tissue specific promoters, cause transcriptional silencing, or pose safety risks to the subject.

The term "vascular pressure pulse" refers to a pulse of pressure from a large volume of liquid to facilitate uptake of a vector into a cell. A skilled artisan recognizes that the amount and duration of the vascular pressure pulse is dependent on the tissue, size, and overall health of the recipient animal, and furthermore knows how to determine such parameters empirically.

The term "vector" as used herein refers to a construction comprised of genetic material designed to direct transformation of a targeted cell by delivering a nucleic acid sequence into that cell. A vector may contain multiple genetic elements positionally and sequentially oriented with other necessary elements such that an included nucleic acid cassette can be transcribed and when necessary translated in the transfected cells. These elements are operatively linked. The term "expression vector" refers to a DNA plasmid that contains all of the information necessary to produce a recombinant protein in a heterologous cell.

The current invention pertains to compositions for treatment of kidney failure, as well as its complications, in a subject. Specific embodiments of this invention are directed to the treatment of complications of renal failure, such as anemia, wasting, immune dysfunction, decreased life expectancy, decreased quality of life, and other conditions that can be related, to a primary kidney disease. In one embodiment of the present invention the compositions comprise an isolated nucleic acid expression construct that encodes a growth-hormone-releasing-hormone ("GHRH") or functional biological equivalent thereof which is to be delivered into a tissue, such as a muscle, of the subject. The subsequent *in vivo* expression of the GHRH or biological equivalent in the subject is sufficient to treat kidney failure, treat anemia, and other conditions commonly associated with kidney failure in order to increase survival and improve welfare in animals with renal failure.

The isolated nucleic acid expression construct can be delivered by *in vivo* electroporation. It is possible to enhance the delivery method by placing a plurality of electrodes in a selected tissue, then delivering the isolated nucleic acid expression construct to the selected tissue in an area that interposes the plurality of electrodes, and applying a cell-transfecting pulse (e.g. electrical) to the selected tissue in an area of the selected tissue where the isolated nucleic acid expression construct was delivered. However, the cell-transfecting pulse need not be an electrical pulse, a vascular pressure pulse can also be utilized. Electroporation, direct injection, gene gun, or gold particle bombardment are also used in specific embodiments to deliver the isolated nucleic acid expression construct encoding the GHRH or biological equivalent into the subject. In a preferred embodiment, the cells of the subject are somatic cells, stem cells, or germ cells. The subject in this invention comprises an animal (e.g. a human, a pig, a horse, a cow, a mouse, a rat, a monkey, a sheep, a goat, a dog, or a cat).

The expression constructs used in the invention can comprise a tissue specific promoter; a GHRH or functional biological equivalent; and a 3' untranslated region ("3' UTR") that are operatively linked. The tissue-specific promoter may be a muscle-specific promoter (e.g. SPc5-12 (SeqID No.: 7)), and the 3' UTR of the nucleic expression construct may be a human growth hormone 3' UTR (SeqID7 No.: 8), bovine growth hormone 3' UTR, skeletal alpha actin 3' UTR, or SV40 polyadenylation signal. The isolated nucleic acid expression construct of this invention comprises a construct that is substantially free of a viral backbone. In additional preferred embodiments, specific examples of nucleic acid expression constructs may be plasmids with sequences of SEQID No.: 11, SeqID No.: 12, SeqID No.: 13, and SeqID No.: 14. Although not wanting to be bound by theory, the ability of cells in a tissue to uptake the nucleic acid expression-construct can be facilitated by a transfection-facilitating polypeptide. In specific embodiments of the invention, the transfection-facilitating polypeptide comprises a charged polypeptide, preferably poly-L-glutamate.

After delivering the nucleic acid expression construct into the cells of the animal subject, expression of the encoded GHRH or functional biological equivalent thereof is initiated. In a preferred embodiment, the expression occurs in the muscle cells of the subject. The encoded GHRH comprises a biologically active polypeptide. The encoded functional biological equivalent of GHRH comprises a polypeptide having similar or improved biological activity when compared to the GHRH polypeptide. The GHRH or functional biological equivalent that is encoded by the nucleic acid expression construct and useful for this invention comprises an amino acid structure with a general sequence as follows (SeqID No.: 6):

-**X₁**-**X₂**-DAIFTNSYRKVL-**X₃**-QLSARKLLQDI-**X₄-X₅**-RQQGE-**X₆**-NQE-**X₇**-GA-OH

wherein: **X₁** is a D-or L-isomer of the aminoacid tyrosine ("Y") or histidine ("H"); **X₂** is a D-or L-isomer of the aminoacid alanine ("A"), valine ("V"), or isoleucine ("I"), **X₃.** is a D- or L-isomer of the aminoacid alanine ("A") or glycine ("G"); **X₄** is a D-or L-isomer of the aminoacid methionine ("M") or leucine ("L"); **X₅** is a D-or L-isomer of the aminoacid serine ("S") or asparagine ("N"); **X₆** is a D-or L-isomer of the aminoacid arginine ("R") or serine ("S"); **X₇** is a D-or L-isomer of the aminoacid glutamine ("Q") or arginine ("R"); and combinations thereof. Specific examples of amino acid sequences for GHRH or functional biological equivalents that are useful for this invention are presented in SeqID No.: 1; SeqID No.: 2; SeqID No.: 3; SeqID No.: 4; SeqID No.: I0; and SeqID No.: 22. In a specific embodiment, the encoded GHRH or functional biological equivalent thereof facilitates growth hormone ("GH") secretion in a subject that has received the nucleic acid expression construct.

A further embodiment of the present invention pertains to compositions for treatment of kidney failure, as well as its complications, utilizing therapy that introduces a specific recombinant GHRH polypeptide or functional biological equivalent into the subject. The recombinant GHRH is a biologically active polypeptide, and the functional biological equivalent has been engineered to contain a distinct amino acid sequence while simultaneously having similar or improved biological activity when compared to the GHRH polypeptide. One embodiment of a specific recombinant GHRH, or functional biological equivalent of GHRH, is of the formula of Seem No: 6. Administration of the encoded GHRH or functional biological equivalent thereof facilitates growth hormone ("GH") secretion in a subject that has been treated.

Additionally, the invention relates to plasmid compositions for the treatment of anemia, wasting, immune dysfunction, or other conditions commonly associated with kidney failure in order to increase the welfare and quality of life and achieve life extension for the chronically ill subject. Anemia refers to a condition in which there is a reduction of the number, volume, or both of red blood corpuscles or of the total amount of hemoglobin in the bloodstream, resulting in paleness, generalized weakness, etc. of the subject. Wasting of a subject can be defined as decreased body weight that is characterized by significant loss of both adipose tissue and muscle mass, which makes weight gain especially difficult for patients with a progressive disease (*e.g.* kidney failure, cancer, AIDS, *etc.).* Kidney failure, and its consequences, such as anemia, wasting, immune dysfunction, and decreased quality of life and life expectancy, can be related to a specific disease or the effects of a disease treatment.

More specifically, this invention pertains to compositions comprising a heterologous nucleic acid sequence encoding GHRH or a biological equivalent thereof for delivery into the cells of the subject (*e.g.* somatic, stem, or germ cells) and allowing expression of the encoded GHRH or biological equivalent gene to occur while the modified cells are within the subject. The subsequent expression of the GHRH or biological equivalent thereof is regulated by a tissue specific promoter, such as a muscle promoter. The extracranial expression and ensuing release of GHRH or a biological equivalent thereof by the modified cells can treat kidney failure, as well as treat its complications. In further specific embodiments, the subj ect is an animal.

Recombinant GH replacement therapy is widely used clinically, with beneficial effects, but generally, the doses are supraphysiological. Such elevated doses of recombinant GH are associated with deleterious side-effects, for example, up to 30% of the recombinant GH treated patients report a higher frequency of insulin resistance (Blethen, 1995; Verhelst et al., 1997) or accelerated bone epiphysis growth and closure in pediatric patients (Blethen and Rundle, 1996). In addition, molecular heterogeneity of circulating GH may have important implications in growth and homeostasis, which can lead to a less potent GH that has a reduced ability to stimulate the prolactin receptor (Satozawa et al., 2000; Tsunekawa et al., 1999; Wada et al., 1998).

Unwanted side effects result from the fact that treatment with recombinant exogenous GH protein raises basal levels of GH and abolishes the natural episodic pulses of GH. In contradistinction, no side effects have been reported for recombinant GHRH therapies. The normal levels of GHRH in the pituitary portal circulation range from about 150-to-800 pg/ml, while systemic circulating values of the hormone are up to about 100-500 pg/ml. Some patients with acromegaly caused by extracranial tumors have level that is nearly 10 times as high (*e.g.* 50 ng/ml of immunoreactive GHRH) (Thorner et al., 1984). Long-term studies using recombinant GHRH therapies (1-5 years) in children and elderly humans have shown an absence of the classical GH side-effects, such as changes in fasting glucose concentration or, in pediatric patients, the accelerated bone epiphysal growth and closure or slipping of the capital femoral epiphysis (Chevalier et al., 2000) (Duck et al., 1992; Vittone et al., 1997). Numerous studies in humans, sheep or pigs show that continuous infusion with recombinant GHRH protein restores the normal GH pattern without desensitizing GHRH receptors or depleting GH supplies (Dubreuil et al., 1990). As this system is capable of a degree of feed-back which is abolished in the GH therapies, GHRH recombinant protein therapy may be more physiological than GH therapy. However, due to the short half-life of GHRH *in vivo*, frequent (one to three times per day) intravenous, subcutaneous or intranasal (requiring 300-fold higher dose) administrations are necessary (Evans et al., 1985; Thorner et al., 1986). Thus, as a chronic therapy, recombinant GHRH protein administration is not practical. A gene transfer approach, however could overcome this limitations to GHRH use. Moreover, a wide range of doses can be therapeutic. The choice of GHRH- for a gene therapeutic application is favored by the fact that the gene, cDNA and native and several mutated molecules have been characterized for the pig and other species (Bohlen et al., 1983; Guillemin et al., 1982), and the measurement of therapeutic efficacy is straightforward and unequivocal.

Previous studies using GHRH showed that plasmid therapy with electroporation is scalable and represents a promising approach to induce production and regulated secretion of proteins in large animals and humans (Draghia-Akli et al., 1999; Draghia-Akli et al., 2002b). Electroporation also has been extensively used in rodents and other small animals (Bettan et al., 2000; Yin and Tang, 2001). It has been observed that the electrode configuration affects the electric field distribution and subsequent results (Gehl et al., 1999; Miklavcic et al., 1998). Preliminary experiments indicated that for a large animal model, needle electrodes give consistently better reproducible results than external caliper electrodes.

In addition, plasmid formulated with PLG or polyvinylpyrrolidone ('PVP") has been observed to increase gene transfection and consequently gene expression to up to 10 fold in the skeletal muscle of mice, rats and dogs (Fewell et al., 2001; Mumper et al., 1998). Although not wanting to be bound by theory, PLG increases the transfection of the plasmid during the electroporation process not only by stabilizing the plasmid DNA and facilitating the intracellular transport through the membrane pores, but also through an active mechanism. For example, positively charged surface proteins on the cells complex the negatively charged PLG linked to plasmid DNA through protein-protein interactions. When an electric field is applied, the surface proteins reverse direction and actively internalize the DNA molecules, a process that substantially increases the transfection efficiency. Furthermore, PLG prevents the muscle damage associated with *in vivo* plasmid delivery (Draghia-Akli et al., 2002a) and increases plasmid stability *in vitro* prior to injection.

The plasmid supplementation approach to treat kidney failure, treat anemia, wasting, immune dysfunction, and other conditions associated with kidney failure, with the purpose of increasing welfare, quality of life and achieve life extension for the chronically ill subject that is described herein offers advantages over the limitations of directly injecting recombinant GH or GHRH protein. Expression of novel biological equivalents of GHRH that are serum protease resistant can be directed by an expression plasmid controlled by a synthetic muscle-specific promoter. Expression of such GHRH or biological equivalent thereof elicited high GH and IGF-I levels in subjects that have had the encoding sequences delivered into the cells of the subject by intramuscular injection and *in vivo* electroporation. Although *in vivo* electroporation is the preferred method of introducing the heterologous nucleic acid encoding system into the cells of the subject, other methods exist and should be known by a person skilled in the art (*e.g.* electroporation, lipofectamine, calcium phosphate, *ex vivo* transformation, direct injection, DEAE dextran, sonication loading, receptor mediated transfection, microprojectile bombardment, etc.). For example, it may also be possible to introduce the nucleic acid sequence that encodes the GHRH or functional biological equivalent thereof directly into the cells of the subject by first removing the cells from the body of the subject or donor, maintaining the cells in culture, then introducing the nucleic acid encoding system by a variety of methods (*e.g.* electroporation, lipofectamine, calcium phosphate, ex vivo transformation, direct injection, DEAE dextran, sonication loading, receptor mediated transfection, microprojectile bombardment, etc.), and finally reintroducing the modified cells into the original subject or other host subject (the ex *vivo* method). The GHRH sequence can be cloned into an adenovirus vector or an adeno-associated vector and delivered by simple intramuscular injection, or intravenously or intra-arterially. Plasmid DNA carrying the GHRH sequence can be complexed with cationic lipids or liposomes and delivered intramuscularly, intravenously or subcutaneous.

Administration as used herein refers to the route of introduction of a vector or carrier of DNA into the body. Administration can be directly to a target tissue or by targeted delivery to the target tissue after systemic administration. In particular, the present invention can be used for treating disease by administration of the vector to the body in order to establishing controlled expression of any specific nucleic acid sequence within tissues at certain levels that are useful for plasmid mediated supplementation. The preferred means for administration of vector and use of formulations for delivery are described above.

Muscle cells have the unique ability to take up DNA from the extracellular space after simple injection of DNA particles as a solution, suspension, or colloid into the muscle. Expression of DNA by this method can be sustained for several months. DNA uptake in muscle cells is further enhanced utilizing *in vivo* electroporation.

Delivery of formulated DNA vectors involves incorporating DNA into macromolecular complexes that undergo endocytosis by the target cell. Such complexes may include lipids, proteins, carbohydrates, synthetic organic compounds, or inorganic compounds. The characteristics of the complex formed with the vector (size, charge, surface characteristics, composition) determine the bioavailability of the vector within the body. Other elements of the formulation function as ligands that interact with specific receptors on the surface or interior of the cell. Other elements of the formulation function to enhance entry into the cell, release from the endosome, and entry into the nucleus.

Delivery can also be through use of DNA transporters. DNA transporters refer to molecules which bind to DNA vectors and are capable of being taken up by epidermal cells. DNA transporters contain a molecular complex capable of non-covalently binding to DNA and efficiently transporting the DNA through the cell membrane. It is preferable that the transporter also transport the DNA through the nuclear membrane. See, *e.g.*, the following applications, all of which (including drawings) are hereby incorporated by reference: (1) Woo et al., U.S. Patent No. 6,150,168 entitled: "A DNA Transporter System and Method of Use;" (2) Woo et al., PCT/US93/02725, entitled "A DNA Transporter System and method of Use", filed Mar. 19, 1993; (3) Woo et al., U.S. Patent No. 6,177,554 "Nucleic Acid Transporter Systems and Methods of Use;" (4) Szoka et al., U.S. Patent No. 5,955,365 entitled "Self-Assembling Polynucleotide Delivery System;" and (5) Szoka et al., PCT/US93/03406, entitled "Self-Assembling Polynucleotide Delivery System", filed Apr. 5, 1993.

Another method of delivery involves a DNA transporter system. The DNA transporter system consists of particles containing several elements that are independently and non-covalently bound to DNA. Each element consists of a ligand which recognizes specific receptors or other functional groups such as a protein complexed with a cationic group that binds to DNA. Examples of cations which may be used are spermine, spermine derivatives, histone, cationic peptides and/or polylysine. One element is capable of binding both to the DNA vector and to a cell surface receptor on the target cell. Examples of such elements are organic compounds which interact with the asialoglycoprotein receptor, the folate receptor, the mannose-6-phosphate receptor, or the carnitine receptor. A second element is capable of binding both to the DNA vector and to a receptor on the nuclear membrane. The nuclear ligand is capable of recognizing and transporting a transporter system through a nuclear membrane. An example of such ligand is the nuclear targeting sequence from SV40 large T antigen or histone. A third element is capable of binding to both the DNA vector and to elements which induce episomal lysis. Examples include inactivated virus particles such as adenovirus, peptides related to influenza virus hemagglutinin, or the GALA peptide described in the Skoka patent cited above.

Administration may also involve lipids. The lipids may form liposomes which are hollow spherical vesicles composed of lipids arranged in unilamellar, bilamellar, or multilamellar fashion and an internal aqueous space for entrapping water soluble compounds, such as DNA, ranging in size from 0.05 to several microns in diameter. Lipids may be useful without forming liposomes. Specific examples include the use of cationic lipids and complexes containing DOPE which interact with DNA and with the membrane of the target cell to facilitate entry of DNA into the cell.

Gene delivery can also be performed by transplanting genetically engineered cells. For example, immature muscle cells called myoblasts may be used to carry genes into the muscle fibers. Myoblasts genetically engineered to express recombinant human growth hormone can secrete the growth hormone into the animal's blood. Secretion of the incorporated gene can be sustained over periods up to 3 months. Myoblasts eventually differentiate and fuse to existing muscle tissue. Because the cell is incorporated into an existing structure, it is not just tolerated but nurtured. Myoblasts can easily be obtained by taking muscle tissue from an individual who needs plasmid-mediated supplementation, and the genetically engineered cells can also be easily put back without causing damage to the patient's muscle. Similarly, keratinocytes may be used to delivery genes to tissues. Large numbers of keratinocytes can be generated by cultivation of a small biopsy. The cultures can be prepared as stratified sheets and when grafted to humans, generate epidermis which continues to improve in histotypic quality over many years. The keratinocytes are genetically engineered while in culture by transfecting the keratinocytes with the appropriate vector. Although keratinocytes are separated from the circulation by the basement membrane dividing the epidermis from the dermis, human keratinocytes secrete into circulation the protein produced.

Delivery may also involve the use of viral vectors. For example, an adenoviral vector may be constructed by replacing the E1 region of the virus genome with the vector elements described in this invention, including a promoter, 5'LJT'R, 3'UTR and nucleic acid cassette, and introducing this recombinant genome into 293 cells which will package this gene into an infectious virus particle. Viruses from this cell may then be used to infect tissue *ex* vivo or *in vivo* to introduce the vector into tissues leading to expression of the gene in the nucleic acid cassette.

Although not wanting to be bound by theory, it is believed that in order to provide an acceptable safety margin for the use of such heterologous nucleic acid sequences in humans, a regulated gene expression system is mandated to possess low levels of basal expression of GHRH and still retain a high ability to induce. The HV-GHRH or biological equivalent molecule displays a high degree of stability in serum, with a half-life of 6 hours, versus the natural GHRH, that has a 6-12 minutes half-life. Thus, by combining the powerful electroporation DNA delivery method with stable and regulatable GHRH or biological equivalent encoded nucleic acid sequences, a therapy can be utilized that will treat or prevent kidney failure, treat anemia, wasting, immune dysfunction, and other conditions associated with kidney failure, with the purpose of improving welfare, quality of life and achieving life extension in chronically ill patients.

### Vectors

The term "vector" is used to refer to a carrier nucleic acid molecule into which a nucleic acid sequence can be inserted for introduction into a cell wherein, in some embodiments, it can be replicated. A nucleic acid sequence can be native to the animal, or it can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), linear DNA fragments, and artificial chromosomes (*e.g.*, YACs), although in a preferred embodiment the vector contains substantially no viral sequences. One of skill in the art would be well equipped to construct a vector through standard recombinant techniques.

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operatively linked coding sequence in a particular host cell. In addition to control sequences that govern transcription and translation, vectors and expression vectors may contain nucleic acid sequences that serve other functions as well and are described *infra*.

### I. Plasmid Vectors

In certain embodiments, a linear DNA fragment from a plasmid vector is contemplated for use to transfect a eukaryotic cell, particularly a mammalian cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, *E. coli* is often transformed using derivatives of pBR322, a plasmid derived from an *E. coli* species. The plasmid pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins. A skilled artisan recognizes that any plasmid in the art may be modified for use in the methods of the present invention. In a specific embodiment, for example, a GHRH vector used for the therapeutic applications is derived from pBlueScript KS+ and has a kanamycin resistance gene.

In addition, phage vectors containing replicon and control sequences that are compatible with the host microorganism can be used as transforming vectors in connection with these hosts. For example, the phage lambda GEM^{™}-11 may be utilized in making a recombinant phage vector which can be used to transform host cells, such as, for example, *E. coli* LE392.

Further useful plasmid vectors include pIN vectors (Inouye et al., 1985); and pGEX vectors, for use in generating glutathione S-transferase ("GST") soluble fusion proteins for later purification and separation or cleavage. Other suitable fusion proteins are those with β-galactosidase, ubiquitin, and the like.

Bacterial host cells, for example, *E. coli,* comprising the expression vector, are grown in any of a number of suitable media, for example, Luria-Bertani ("LB'). The expression of the recombinant protein in certain vectors may be induced, as would be understood by those of skill in the art, by contacting a host cell with an agent specific for certain promoters, for example, by adding isopropyl beta-D-thiogalactopyranoside ("IPTG") to the media or by switching incubation to a higher temperature. After culturing the bacteria for a further period, generally between 2 and 24 hours, the cells are collected by centrifugation and washed to remove residual media.

### II. Promoters and Enhancers

A promoter is a control sequence that is a region of a nucleic acid sequence at which initiation and rate of transcription of a gene product are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control or a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant, synthetic or heterologous promoter, which refers to a promoter that is not normally associated with a nucleic acid sequence in its natural environment. A recombinant, synthetic or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e.*, containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (U.S. Patent Nos. 4,683,202 and 5,928,906). Furthermore, it is contemplated that the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression (see, for example, (Sambrook et al., 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (for example, those in the Eukaryotic Promoter Data Base, EPDB) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

Tables 1 and 2 list non-limiting examples of elements/promoters that may be employed, in the context of the present invention, to regulate the expression of a RNA. Table 2 provides non-limiting examples of inducible elements, which are regions of a nucleic acid sequence that can be activated in response to a specific stimulus.

| **TABLE 1** | |
|---|---|
| **Promoter and/or Enhancer** | |
| **Promoter/Enhancer** | **Relevant References** |
| β-Actin | (Kawamoto et al., 1988; Kawamoto et al., 1989) |
| Muscle Creatine Kinase (MCK) | (Horlick and Benfield, 1989; Jaynes et al., 1988) |
| Metallothionein (MTII) | (Inouye et al., 1994; Narum et al., 2001; Skroch et al., 1993) |
| Albumin | (Pinkert et al., 1987; Tronche et al., 1989) |
| β-Globin | (Tronche et al., 1990; Trudel and Costantini, 1987) |
| Insulin | (German et al., 1995; Ohlsson et al., 1991) |
| Rat Growth Hormone | (Larsen et al., 1986) |
| Human Serum Amyloid A (SAA) | |
| Troponin I (TN I) | (Lin et al., 1991; Yutzey and Konieczny, 1992) |
| Platelet-Derived Growth Factor (PDGF) | (Pech et al., 1989) |
| Duchenne Muscular Dystrophy | (Klamut et al., 1990; Klamut et al., 1996) |
| Cytomegalovirus (CMV) | (Boshart et al., 1985; Dorsch-Hasler et al., 1985) |
| Synthetic muscle specific promoters (c5-12, cl-28) | (Draghia-Akli et al., 1999; Draghia-Akli et al., 2002b; Li et al., 1999) |

| **TABLE 2** | |
|---|---|
| **Element/Inducer** | |
| **Element** | **Inducer** |
| MT II | Phorbol Ester (TFA)/ Heavy metals |
| MMTV (mouse mammary tumor virus) | Glucocorticoids |
| β-Interferon | Poly(rI)x / Poly(rc) |
| Adenovirus 5 E2 | E1A |
| Collagenase | Phorbol Ester (TPA) |
| Stromelysin | Phorbol Ester (TPA) |
| SV40 | Phorbol Ester (TPA) |
| Murine MX Gene | Interferon, Newcastle Disease Virus |
| GRP78 Gene | A23187 |
| a-2-Macroglobulin | IL-6 |
| Vimentin | Serum |
| MIMIC Class I Gene H-2κb | Interferon |
| HSP70 | EIA, SV40 Large T Antigen |
| Proliferin | Phorbol Ester-TPA |
| Tumor Necrosis Factor α | PMA |
| Thyroid Stimulating Hormone α Gene | Thyroid Hormone |

The identity of tissue-specific promoters or elements, as well as assays to characterize their activity, is well known to those of skill in the art. Nonlimiting examples of such regions include the human LIMK2 gene (Nomoto et al., 1999), the somatostatin receptor 2 gene (Kraus et al., 1998), murine epididymal retinoic acid-binding gene (Lareyre et al., 1999), human CD4 (Zhao-Emonet et al., 1998), mouse alpha2 (XI) collagen (Liu et al., 2000; Tsumaki et al., 1998), D1A dopamine receptor gene (Lee et al., 1997), insulin-like growth factor II (Dai et al., 2001; Wu et al., 1997), and human platelet endothelial cell adhesion molecule-1 (Almendro et al., 1996).

In a preferred embodiment, a synthetic muscle promoter is utilized, such as SPc5-12 (Li et al., 1999), which contains a proximal serum response element ("SRE") from skeletal α-actin, multiple MEF-2 sites, MEF-1 sites, and TEF-1 binding sites, and greatly exceeds the transcriptional potencies of natural myogenic promoters. The uniqueness of such a synthetic promoter is a significant improvement over, for instance, issued patents concerning a myogenic promoter and its use (U.S. Pat. No. 5,374,544) or systems for myogenic expression of a nucleic acid sequence (U.S. Pat. No. 5,298,422). In a preferred embodiment, the promoter utilized in the invention does not get shut off or reduced in activity significantly by endogenous cellular machinery or factors. Other elements, including *trans*-acting factor binding sites and enhancers may be used in accordance with this embodiment of the invention. In an alternative embodiment, a natural myogenic promoter is utilized, and a skilled artisan is aware how to obtain such promoter sequences from databases including the National Center for Biotechnology Information ("NCBI") GenBank database or the NCBI PubMed site. A skilled artisan is aware that these databases may be utilized to obtain sequences or relevant literature related to the present invention.

### III. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites ("IRES") elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Samow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

### IV. Multiple Cloning Sites

Vectors can include a MCS, which is a nucleic acid region that contains multiple restriction enzyme sites, any of which can be used in conjunction with standard recombinant technology to digest the vector (see, for example, (Carbonelli et al., 1999; Cocea, 1997; Levenson et al., 1998), incorporated herein by reference.) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### V. Restriction Enzymes

In some embodiments of the present invention, a linear DNA fragment is generated by restriction enzyme digestion of a parent DNA molecule. The term "restriction enzyme digestion" of DNA as used herein refers to catalytic cleavage of the DNA with an enzyme that acts only at certain locations in the DNA. Such enzymes are called restriction endonucleases, and the sites for which each is specific is called a restriction site. The various restriction enzymes used herein are commercially available and their reaction conditions, cofactors, and other requirements as established by the enzyme suppliers are used. Restriction enzymes commonly are designated by abbreviations composed of a capital letter followed by other letters representing the microorganism from which each restriction enzyme originally was obtained and then a number designating the particular enzyme.

In general, about 1 µg of plasmid or DNA fragment is used with about 1-2 units of enzyme in about 20 µl of buffer solution. Appropriate buffers and substrate amounts for particular restriction enzymes are specified by the manufacturer. Incubation of about 1 hour at 37°C is ordinarily used, but may vary in accordance with the supplier's instructions. After incubation, protein or polypeptide is removed by extraction with phenol and chloroform, and the digested nucleic acid is recovered from the aqueous fraction by precipitation with ethanol. Digestion with a restriction enzyme may be followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional as described in the art.

### VI. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, (Chandler et al., 1997), herein incorporated by reference.)

### VII. Termination Signals

The vectors or constructs of the present invention will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues ("polyA") to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated for use in the invention include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### VIII. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal, skeletal alpha actin 3'UTR or the human or bovine growth hormone polyadenylation signal, convenient and known to function well in various target cells. Polyadenylation may increase the stability of the transcript or may facilitate cytoplasmic transport.

### IX. Origins of Replication

In order to propagate a vector in a host cell, it may contain one or more origin of replication sites (often termed "ori"), which is a specific nucleic acid sequence at which replication is initiated. Alternatively an autonomously replicating sequence ("ARS") can be employed if the host cell is yeast.

### X. Selectable and Screenable Markers

In certain embodiments of the invention, cells containing a nucleic acid construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants. For example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase ("tk") or chloramphenicol acetyltransferase ("CAT") may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### XI. Electroporation

In certain embodiments of the present invention, a nucleic acid is introduced into an organelle, a cell, a tissue or an organism via electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent No.5,384,253 incorporated herein by reference). Alternatively, recipient cells can be made more susceptible to transformation by mechanical wounding and other methods known in the art.

Transfection of eukaryotic cells using electroporation has been quite successful. Mouse pre-B lymphocytes have been transfected with human kappa-immunoglobulin genes (Potter et al., 1984), and rat hepatocytes have been transfected with the chloramphenicol acetyltransferase gene (Tur-Kaspa et al., 1986) in this manner.

The underlying phenomenon of electroporation is believed to be the same in all cases, but the exact mechanism responsible for the observed effects has not been elucidated. Although not wanting to be bound by theory, the overt manifestation of the electroporative effect is that cell membranes become transiently permeable to large molecules, after the cells have been exposed to electric pulses. There are conduits through cell walls, which under normal circumstances maintain a resting transmembrane potential of ca. 90 mV by allowing bi-directional ionic migration.

Although not wanting to be bound by theory, electroporation makes use of the same structures, by forcing a high ionic flux through these structures and opening or enlarging the conduits. In prior art, metallic electrodes are placed in contact with tissues and predetermined voltages, proportional to the distance between the electrodes are imposed on them. The protocols used for electroporation are defined in terms of the resulting field intensities, according to the formula ***E*=*V*/*d***, where ("***E***") is the field, ("***V***") is the imposed voltage and ("***d***") is the distance between the electrodes.

The electric field intensity *E* has been a very important value in prior art when formulating electroporation protocols for the delivery of a drug or macromolecule into the cell of the subject. Accordingly, it is possible to calculate any electric field intensity for a variety of protocols by applying a pulse of predetermined voltage that is proportional to the distance between electrodes. However, a caveat is that an electric field can be generated in a tissue with insulated electrodes (i.e. flow of ions is not necessary to create an electric field). Although not wanting to be bound by theory, it is the current that is necessary for successful electroporation not electric field per se.

During electroporation, the heat produced is the product of the interelectrode impedance, the square of the current, and the pulse duration. Heat is produced during electroporation in tissues and can be derived as the product of the inter-electrode current, voltage and pulse duration. The protocols currently described for electroporation are defined in terms of the resulting field intensities ***E***, which are dependent on short voltage pulses of unknown current. Accordingly, the resistance or heat generated in a tissue cannot be determined, which leads to varied success with different pulsed voltage electroporation protocols with predetermined voltages. Although not wanting to be bound by theory, the nature of the voltage pulse to be generated is determined by the nature of the tissue, the size of the selected tissue, and the distance between electrodes. It is desirable that the voltage pulse be as homogenous as possible and of the correct amplitude. Excessive field strength results in the lysing of cells, whereas low field strength results in reduced efficacy of electroporation. Some electroporation devices utilize the distance between electrodes to calculate the electric field strength and predetermined voltage pulses for electroporation. This reliance on knowing the distance between electrodes is a limitation to the design of electrodes. Because the programmable current pulse controller will determine the impedance in a volume of tissue between two electrodes, the distance between electrodes is not a critical factor for determining the appropriate electrical current pulse. Therefore, an alternate needle electrode array design would be one that is non-symmetrical. In addition, one skilled in the art can imagine any number of suitable symmetrical and non-symmetrical needle electrode arrays. The depth of each individual electrode within an array and in the desired tissue could be varied with comparable results. In addition, multiple injection sites for the macromolecules could be added to the needle electrode array.

The ability to limit heating of cells across electrodes can increase the effectiveness of any given electroporation voltage pulsing protocol. For example, the prior art teaches the utilization of an array of six needle electrodes utilizing a predetermined voltage pulse across opposing electrode pairs. This situation sets up a centralized pattern during an electroporation event in an area where congruent and intersecting overlap points develop. Excessive heating of cells and tissue along electroporation path will kill the cells, and limit the effectiveness of the protocol. However, symmetrically arranged needle electrodes without opposing pairs can produce a decentralized pattern during an electroporation event in an area where no congruent electroporation overlap points can develop. It is preferable to use an electrode system for electroporation having a configuration of pin electrodes whereby the electroporation pulse is directed between two or more electrodes such that the direct line between any two electrodes does not pass through the center of the injected macromolecule. This is to minimize the number of cells that are under energized and thus not electroporated and the number of cells which are over energized and thus destroyed while at the same time maximizing the number of cells that lie between these extremes which are adequately energized and thus electroporated.

Controlling the current flow between electrodes allows one to determine the relative heating of cells. Thus, it is the current that determines the subsequent effectiveness of any given pulsing protocol and not the voltage across the electrodes. Predetermined voltages do not produce predetermined currents, and the usefulness of the technique is limited without a means to determine the exact dosage of current. This problem may be overcome by using a constant-current system, which effectively controls the dosage of electricity delivered to the cells in the inter-electrode space by precisely controlling the ionic flux that impinges on the conduits in the cell membranes. The advantage of a constant-current system is that it can be prevented from attaining an amplitude at which the cells are destroyed. In a predetermined voltage system, the current can attain a destructive intensity, and the operator cannot prevent that from happening. In a constant-current system, the current is preset under a threshold level where cell death does not occur. The exact setting of the current is dependent on the electrode configuration, and it must be determined experimentally. However, once the proper level has been determined, cell survival is assured from case to case. The precise dosage of electricity to tissues can be calculated as the product of the current level, the pulse length and the number of pulses delivered. These factors can be determined by the operator and do not vary with the characteristics of different tissues or variations of the electrode impedance from case to case. Thus, controlling and maintaining the current in the tissue between two electrodes under a threshold will allow one to vary the pulse conditions, reduce cell heating, create less cell death, and incorporate macromolecules into cells more efficiently when compared to predetermined voltage pulses. Furthermore, owing to the inherent repeatability of the constant-current system, effective protocols for electroporation can be developed.

### EXAMPLES

The following examples are included (i) to demonstrate preferred embodiments of the invention, and in certain instances merely (ii) to enhance understanding of the invention, and are to be seen as non-illustrative thereof (examples 3-6 and 13). It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### EXAMPLE 1

### CONSTRUCTION OF DNA VECTORS AND METHODS IN ANIMAL SUBJECT

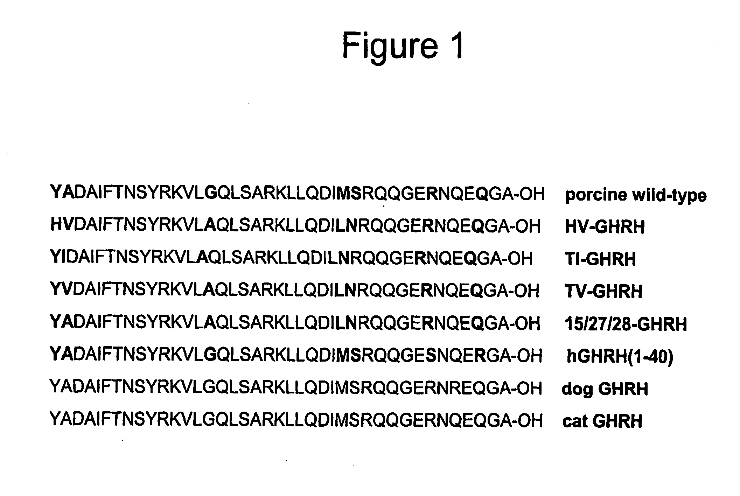

In order to treat kidney failure and its complications using plasmid mediated gene supplementation, it was first necessary to design several GHRH constructs. Briefly, the plasmid vectors contained the muscle specific synthetic promoter SPc5-12 (Li et al., 1999) attached to a wild-type or analog porcine GHRH. The analog GHRH sequences were generated by site directed mutagenesis as described (Draghia-Akli et al., 1999). Nucleic acid sequences encoding GHRH or analog were cloned into the BamHI/ HindIII sites of pSPc5-12 plasmid, to generate pSP-GHRH. Other elements contained in the plasmids include a 3' untranslated region of growth hormone. The unique nucleic acid sequences for the constructs used are shown in Figure 1.

**DNA constructs:** Plasmid vectors containing the muscle specific synthetic promoter SPc5-12 (SEQID No.: 7) were previously described (Li et al., 1999). Wild type and mutated porcine GHRH cDNAs were generated by site directed mutagenesis of GHRH cDNA (SEQID No.: 9) (Altered Sites II *in vitro* Mutagenesis System, Promega, Madison, WI), and cloned into the BamHI/ Hind III sites of pSPc5-12, to generate pSP-wt-GHRH (SEQID No.: 15), or pSP-HV-GHRH (SEQID No.: 11), respectively. For the mouse experiments, the plasmid pSPc5-12 contained a 360bp SacI/BamHI fragment of the SPc5-12 synthetic promoter (Li et al., 1999) inserted in the SacI/BamHI sites of the pSK-GHRH backbone (Draghia-Akli et al., 1997). To generate pSP-hGHRH(1-40), the human GHRH cDNA was modified by site directed mutagenesis of human (1-44)OF GHRH cDNA, and cloned into the BamHI/Hind III sites of pSP-GHRH, followed by the 3' untranslated region and poly(A) signal of hGH gene (Draghia-Akli et al., 1999). Control plasmid, pSP-β-gal, contained the *E. coli* β-galactosidase gene under control of the same muscle-specific promoter. Plasmids were grown in *E. coli* DH5α, (GIBCO, Grand Island, NY). Endotoxin-free plasmid (Qiagen Inc., Chatsworth, CA) preparations were diluted to 2 mg/mL in sterile water and stored at -80°C prior to use.

The 3' untranslated region (3'UTR) of human growth hormone was cloned downstream of GHRH cDNA. The resultant plasmids contained mutated coding region for GHRH, and the resultant aminoacid sequences were not naturally present in mammals. Although not wanting to be bound by theory, the effects on treating or preventing kidney failure and treating its complications are determined ultimately by the circulating levels of hormones. Several different plasmids that encoded different mutated amino acid sequences of GHRH or functional biological equivalent thereof are as follows:

| **Plasmid** | **Encoded Amino Acid Sequence** |
|---|---|
| **h(1-40)GHRH** | **YA**DAIFTNSYRKVL**G**QLSARKLLQDI**MS**RQQGE**S**NQE**R**G**A**-OH (SEQID No.: 22) |
| **wt-GHRH** | **YA**DAIFTNSYRKVL**G**QLSARKLLQDI**MS**RQQGERNQE**QGA**-OH (SEQID No.: 10) |
| **HV-GHRH** | **HV**DAIFTNSYRKVL**A**QLSARKLLQDI**LN**RQQGE**R**NQE**Q**GA-OH (SEQID No.: 1) |
| **TI-GHRH** | **YI**DAIFTNSYRKVL**A**QLSARKLLQDI**LNR**QQGE**R**NQE**Q**GA-OH (SEQID No. : 2) |
| **TV-GHRH** | **YV**DAIFTNSYRKVL**A**QLSARKLLQDI**LN**RQQGERNQE**Q**GA-OH (SEQID No.: 3) |
| **15/27/28-GHRH** | **YA**DAIFTNSYRKVL**A**QLSARKLLQDI**LN**RQQG**ER**NQE**Q**GA-OH (SEQID No.: 4) |

In general, the encoded GHRH or functional biological equivalent thereof is of the formula (SEQID No.: 6):

-**X₁**-**X₂**-DAIFTNSYRKVL-**X₃**-QLSARKLLQDI-**X₄**-**X₅**-RQQGE-**X₆**-NQE-**X₇**-GA-OH

wherein: **X₁** is a D-or L-isomer of the aminoacid tyrosine ("Y") or histidine ("H"); **X₂** is a D-or L-isomer of the aminoacid alanine ("A"), valine ("V"), or isoleucine ("I"); **X₃** is a D-or L-isomer of the aminoacid alanine ("A") or glycine ("G"); **X₄** is a D-or L-isomer of the aminoacid methionine ("M") or leucine ("L"); **X₅** is a D-or L-isomer of the aminoacid serine ("S") or asparagine ("N"); **X₆** is a D-or L-isomer of the aminoacid arginine ("R") or serine ("S"); **X₇** is a D-or L-isomer of the aminoacid glutamine ("Q") or arginine ("R"); and combinations thereof.

Another plasmid that was utilized included the pSP-SEAP construct (SEQID No.: 16) that contains the SacI/ HindIII Specs-12 fragment, SEAP gene and SV40 3'UTR from pSEAP-2 Basic Vector (Clontech Laboratories, Inc.; Palo Alto, CA).

The plasmids described above do not contain polylinker, IGF-I gene, a skeletal alpha-actin promoter or a skeletal alpha actin 3' UTR /NCR. Furthermore, these plasmids were introduced by muscle injection, followed by *in vivo* electroporation, as described below.

In terms of "functional biological equivalents", it is well understood by the skilled artisan that, inherent in the definition of a "biologically functional equivalent" protein and/or polynucleotide, is the concept that there is a limit to the number of changes that may be made within a defined portion of the molecule while retaining a molecule with an acceptable level of equivalent biological activity. Functional biological equivalents are thus defined herein as those proteins (and polynucleotides) in which selected aminoacids (or codons) may be substituted. A peptide comprising a functional biological equivalent of GHRH is a polypeptide that has been engineered to contain distinct amino acid sequences while simultaneously having similar or improved biologically activity when compared to GHRH. For example, one biological activity of GHRH is to facilitate GH secretion in the subject.

**Large Animal Studies: Healths Dogs:** A group of 4 dogs (2 males and 2 females) were used as controls and 3 groups of 8 dogs (4 males and 4 females) were injected with the pSP-HV-GHRH system. The dogs were injected with vehicle alone (control), or 200mcg, or 600mcg or 1000mcg of pSP-HV-GHRH followed by caliper electroporation.

**Kidney Failure Dogs:** Thirty dogs with kidney failure were used in GHRH studies. The values of creatinine had to be at least 2.5mg/dl (normal range: 0.4-1.8mg/dl) and blood urea nitrogen had to be at least 35mg/dl (normal range: 7-27mg/dl) in order for the animal to be enrolled in the study. The average age of the dogs enrolled in the study was 12.6 years. The dogs were injected with 400 mcg of pSP-HV-GHRH. The condition of inclusion in our study was a survival of at least 20 days post-injection (in order to allow for plasmid activation and expression of GHRH from the skeletal muscle), when a second blood draw could be made. The animals were weighed and bled before the treatment and at several time points post-injection. At each time point, complete CBC and metabolic profiles were assessed by the same independent laboratory (Antech Diagnostics, Irvine, CA). Wellness forms were completed by owners at each visit. The quality of life in the treated patients increased. No adverse effects linked to the therapy were noted by owners. Owners noticed a dramatic improvement in the general well-being of the treated dog compared to pre-injection status.

**Kidney Failure Cats:** Thirty cats with kidney failure were used in GHRH studies. The values of creatinine had to be at least 3.5mg/dl (normal range: 0.8-2.3mg/dl) and blood urea nitrogen had to be at least 45mg/dl (normal range: 13.4-32.5mg/dl) in order for the animal to be enrolled in the study. The average age of the cats enrolled in the study was 13 years. The cats were injected with 100 mcg of pSP-HV-GHRH. The condition of inclusion in our study was a survival of at least 20 days post-injection (in order to allow for plasmid activation and expression of GHRH from the skeletal muscle), when a second blood draw could be made. The animals were weighed and bled before the treatment and at several time points post-injection. At each time point, complete CBC and metabolic profiles were assessed by the same independent laboratory (Antech Diagnostics, Irvine, CA). Wellness forms were completed by owners at each visit. The quality of life in the treated patients increased. No adverse effects linked to the therapy were noted by owners. Owners noticed a dramatic improvement in the general well-being of the treated dog compared to pre-injection status.

**Electroporation devices:** A BTX T820 generator (BTX, division of Genetronics Inc., CA) was used to deliver square wave pulses in the initial experiments. We used voltage conditions of 150V/cm, 5 pulses, 50 milliseconds per pulse. Two- needle electrodes (BTX, a division of Genetronics Inc., CA) were used to deliver *in vivo* electric pulses. For the kidney failure cats and dogs an electrokinetic device (EKD, Advisys Inc.), delivering constant current square waves, was used. In all injections, the needles were completely inserted into the muscle.

**Intramuscular injection of plasmid DNA in healthy canine subjects:** Four groups of healthy Canines ("dogs") subjects were used for bio-distribution-toxicology studies. Twenty-eight dogs (Beagles; Harlan Sprague-Dawley, Inc.), approximately two years of age, weight range 10-18 kg for males and 7-13 kg for females, were divided into one group of four animals (Group I) and three groups of eight animals each (Groups II-IV). Normal growth, appearance, and behavior were factors used to select healthy animals for testing. Each animal received a pretest physical examination by a laboratory animal veterinarian. Animals were identified by tattoos and cage cards. Half of the animals from each group were necropsied at day 93 (2 control animals - one male (M) and one female (F), and 4 treated animals from each treatment group - two males and two females/group). At approximately day 180 the other half of the animals from each group was necropsied (2 control animals - one male and one female, and 4 treated animals from each treatment group - two males and two females/group).

Endotoxin-free plasmid preparation of pSP-HV-GHRH was diluted in water to 2 mg/mL. Animals in Groups II-IV were treated with 1X (a total of 0.2 mg plasmid), 3X (a total of 0.6 mg plasmid), and 5X (a total of 1 mg plasmid) pSP-HV-GHRH plasmid by intramuscular injection followed by electroporation on Day 0. Group I animals underwent the electroporation procedure but were not injected with the test article and served as untreated controls. The dogs were anesthetized with 2 ml of a 50:50 mixture of xylazine (Phoenix Scientific, Inc., St. Joseph, MO) and ketamine (Abbott Laboratories, Inc., North Chicago, IL) delivered intramuscularly. All animals received a tattoo of the projected injection area in order to properly identify and isolate the site. The plasmid was injected directly into the semitendinosus muscle with a 3/10 cc insulin syringe and a 29G1/2 needle (Becton-Dickinson, Franklin Lakes, NJ). Two minutes after injection, the injected muscle was electroporated (3 pulses in one orientation, 3 pulses in an orientation perpendicular to the first one, 150V/cm, 50 milliseconds/pulse) with a 2-needle electrode device, and BTX-830 electroporator (Genetronics, San Diego, CA). Animals were observed during recovery from anesthesia and then returned to their cages.

**Intramuscular injection of plasmid DNA in canine or feline subjects** **with kidney failure:** Endotoxin-free plasmid preparation of pSPc5-12-HV-GHRH was diluted in water to 2 mg/mL, and a poly-L-glutamate solution was added to a final PLG concentration of 0.01 mg/mL. Dogs and cats in this study were anesthetized with isofluorane (5% for induction, 2% for maintenance). While anesthetized, plasmid was injected directly into the semitendinosus muscle of dogs, using a 3/10 cc insulin syringe and 29G1/2" needle (Becton-Dickinson, Franklin Lacks, NJ). The injection was followed by electroporation using the EKD device. In all injections the needles were completely inserted into the muscle. Animals were allowed to recover before rejoining their owners.

**Injection, Electroporation, and Experimental Procedure for Mice** **with Implanted Tumors:** Animals received a pre-experiment physical examination by a licensed veterinarian or registered veterinary technician prior to selection for testing. At Day -7, all animals were weighed, bled, and randomly assigned to treatment and control groups (n=20/group, 10 of each sex). On Day -1, animals were weighed, bled, and injected subcutaneously in the flank with 2.5 x 10⁶ LL-2 cells in 30 µL PBS. Mice were anesthetized on Day 0 with 0.5 to 0.7 mL/kg of a combination anesthetic: ketamine (42.8 mg/mL), xylazine (8.2 mg/mL) and acepromazine (0.7 mg/mL). Twenty µg of plasmid diluted in a final volume of 25 µL was injected into the lateral gastrocnemius muscle using 3/10 cc syringe with 26 gauge needle. Two minutes after injection, the injected muscle was electroporated (3 pulses, 150V/cm, 50 milliseconds) with a BTX T830 electroporator and two-needle electrodes (BTX, San Diego, CA), as described (Khan et al., 2002). Animals were weighed and bled once a week, while tumor volume was evaluated twice a week using Promax NSK Electronic Digital Calipers (Fred Fowler Co., Newton, MA) and Gage Wedge for Sylvac Measuring Tools software (TAL Technologies, Philadelphia, PA). Length, width, and depth of the tumors were separately measured and then used to calculate tumor volume. This experimental procedure was repeated.

Although *in vivo* electroporation is the preferred method for delivering the nucleic acid constructs into the cells of the subject, suitable methods for nucleic acid delivery for transformation of an organelle, a cell, a tissue or an organism for use with the current invention are believed to include virtually any method by which a nucleic acid (*e.g*., DNA) can be introduced into an organelle, a cell, a tissue or an organism, as described herein or as would be known to one of ordinary skill in the art. Such methods include, but are not limited to, direct delivery of DNA such as by *ex vivo* transfection (Nabel et al., 1989; Wilson et al., 1989); by injection (U.S. Patent Nos. 5,994,624, 5,981,274, 5,945,100, 5,780,448, 5,736,524, 5,702,932, 5,656,610, 5,589,466 and 5,580,859, each incorporated herein by reference), including microinjection (Harland and Weintraub, 1985); U.S. Patent No. 5,789,215, incorporated herein by reference); by electroporation (U.S. Patent No. 5,384,253, incorporated herein by reference; (Potter et al., 1984; Tur-Kaspa et al., 1986)); by calcium phosphate precipitation (Chen and Okayama, 1987; Graham and van der Eb, 1973; Rippe et al., 1990); by using DEAE-dextran followed by polyethylene glycol (Gopal, 1985); by direct sonic loading (Fechheimer et al., 1987); by liposome mediated transfection (Hafez et al., 2001; Hamm et al., 2002; Madry et al., 2001; Raghavachari and Fahl, 2002; Wiethoff et al., 2001) and receptor-mediated transfection (Wu and Wu, 1988a; Wu and Wu, 1988b); by microprojectile bombardment (PCT Application Nos. WO 94/09699 and 95/06128; U.S. Patent Nos. 5,610,042; 5,322,783 5,563,055, 5,550,318, 5,538,877 and 5,538,880, each incorporated herein by reference); by agitation with silicon carbide fibers ((Johnson et al., 1992); U.S. Patent Nos. 5,302,523 and 5,464,765, each incorporated herein by reference); by *Agrobacterium*-mediated transformation (U.S. Patent Nos. 5,591,616 and 5,563,055, each incorporated herein by reference); by PEG-mediated transformation of protoplasts (Omirulleh et al., 1993); U.S. Patent Nos. 4,684,611 and 4,952,500, each incorporated herein by reference); by desiccation/inhibition-mediated DNA uptake (Potrykus et al., 1985), and any combination of such methods. Through the application of techniques such as these, organelle(s), cell(s), tissue(s) or organism(s) may be stably or transiently transformed.

**Body weight data:** In the toxicology study, dogs were weighed and numbered before the injection/electroporation procedure. Two pre-injection blood draws (days -6 and 0), and six post-treatment draws (days 28, 56, 90, 120, 157 and 180) were performed. Animals enrolled in the kidney failure study (dogs and cats) were weighed before the plasmid injection and at several time points post-injection using the same calibrated scale.

**Blood and urine values:** Blood and urine samples were collected before plasmid injection, at the previously stated post-injection time-points, and analyzed for biochemistry, metabolism and hormones. Average CBC, biochemistry and hormone values for days -6 and 0 served as baseline reference for each dog. Whole blood was collected in Monoject® Lavender Stopper blood collection tubes with 3.0 mg EDTA (Sherwood Medical, St. Louis, MO) and submitted for CBC analysis (Antech Diagnostics, Irvine, CA). Serum was aliquoted for radioimmunoassay and biochemical analysis (Antech Diagnostics, Irvine, CA).

**CBC and Biochemistries for Mouse Experiments:** At necropsy, in both studies, whole blood was collected in Microtainer Brand tubes with EDTA (Becton Dickinson, Franklin Lakes, NJ) for CBC analysis and in Microtainer Serum Separator tubes (Becton Dickinson, Franklin Lakes, NJ) for serum biochemistries. All tests were performed by IDEXX Contract Research Services (West Sacramento, CA). Parameters tested in the biochemical analysis were: ALT (alanine aminotransferase), AST (aspartate aminotransferase), creatinine kinase, albumin, total protein, bilirubin, cholesterol, glucose, calcium, phosphorous, bicarbonate, chloride, potassium, and sodium.

**Plasma IGF-I:** IGF-I levels were measured by heterologous human radioimmunometric assay (Diagnostic System Lab., Webster, TX). The sensitivity of the assay was 0.8ng/ml; intra-assay and inter-assay variation was 3.4% and 4.5% respectively. In the mouse tumor implantation study, serum was aliquoted for IGF-I measurement using mouse IGF-I kit (Diagnostic Systems Laboratories, Inc., Webster, TX). All samples were analyzed in the same assay and the intra-assay variability was 4.2%.

**Necropsy and Histopathology for Mice with Implanted Tumors:** All animals were weighed and then euthanized by CO₂ inhalation on Day 19. Organs (lungs, heart, liver, spleen, kidneys, and the injected gastrocnemius) were excised, weighed and checked for gross pathologies. Gastrocnemius and any of the excised organs with macroscopic abnormalities were fixed in 10% buffered formalin overnight, washed in PBS, and transferred to 70% ethanol for storage. Tumors were excised, weighed, fixed in 10% buffered formalin overnight, and stored in 70% ethanol. A complete histopathological examination was performed on internal organs (brain, heart, lung, liver, spleen, and kidneys), injected muscle and tumor of all animals in the study (20 treated and 20 controls) by a licensed veterinary pathologist (IDEXX Laboratories, Inc., West Sacramento, CA). The tissues were paraffin embedded and 5 µm mid-sagittal sections were cut. These sections were stained with hematoxylin/eosin and examined microscopically. For each organ of each animal one to three slides were read and data were recorded.

**Statistics:** Data are analyzed using STATISTICA analysis package (StatSoft, Inc. Tulsa, OK). Values shown in the Figures are the mean ± s.e.m. Specific P values were obtained by comparison using ANOVA. A P < 0.05 was set as the level of statistical significance.

### EXAMPLE 2

### LOW VOLTAGE ELECTROPORATION FOR DNA UPTAKE AND EXPRESSION IN AN ANIMAL SUBJECT

Direct intra-muscular plasmid DNA injection followed by electroporation is a method for the local and controlled delivery of plasmid DNA into skeletal muscle. It has the advantage that it uses low plasmid quantities (as low as 0.1 mg), rather than the high quantities typically used with passive delivery modalities. Although not wanting to be bound by theory, the mechanism of the increased plasmid uptake by electroporation probably occurs through newly created membrane pores with or without protein active transport. The degree of permeabilization of the muscle cells is dependent on the electric field intensity, length of pulses, shape and type of electrodes (Bureau et al., 2000) (Gilbert et al., 1997), and cell size (Somiari et al., 2000). Classical electrode configuration, plates, or a pair of wire electrodes placed 4 mm apart were shown to be effective in rodents, but in large mammals such as pigs or humans the increased resistance of the skin, the thickness of the subcutaneous fat tissue, and the concern for tissue damage if the intensity of the electric field were to be proportionally increased, make these types of electrodes impractical. The porcine or dog muscle fibers are quite large and consequently more suitable for electropermeabilization than rodent muscle. This example shows that single injections of various dosages of GHRH or analog nucleic acid sequences followed by electroporation with intramuscular applicators in a large mammal is sufficient to produce therapeutic plasma hormone levels, with biologically significant effects that can treat or prevent kidney failure, treat anemia, wasting, immune dysfunction, and other conditions associated with kidney failure, with the purpose of increasing welfare, quality of life and achieve life extension in chronically ill patients.

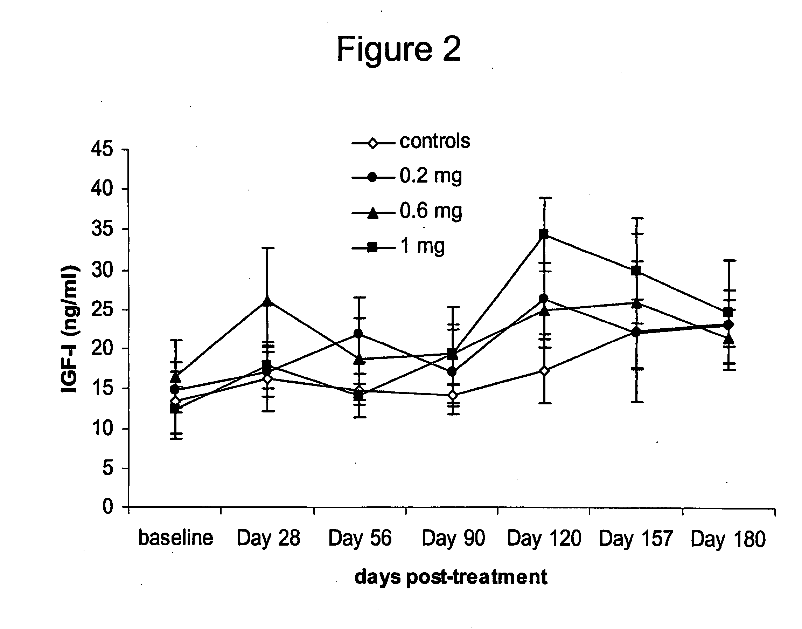

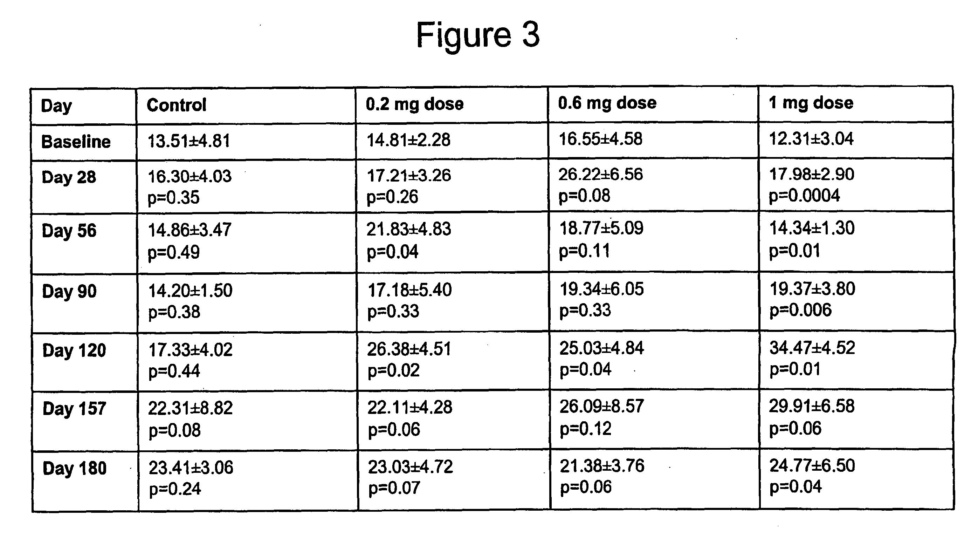

The pSP-HV-GHRH plasmid system was delivered to the semitendinosous muscle of healthy dogs via *in vivo* electroporation. A group of 4 dogs (2 males and 2 females) were used as controls and 3 groups of 8 dogs (4 males and 4 females) were injected with the pSP-HV-GHRH system. An indication of increased systemic levels of GHRH and GH is an increase in serum IGF-I concentration. Therefore, 14-180 days after injection, blood serum was collected from the dogs injected with vehicle alone (control), 200mcg, 600mcg, and 1000mcg of pSP-HV-GHRH and IGF-I levels were determined. An increase in serum IGF-I concentration over the baseline value was taken as a measure of plasmid GHRH activity (Aimaretti et al., 1998; de Boer et al., 1996). During the study, IGF-I levels were increased in plasmid-treated dogs, but the values were not significantly different among groups due to inter-animal variability. When each dog was compared to its own baseline (IGF-I post-injection - IGF-I pre-injection/IGF-I pre-injection), the group treated with 1 mg plasmid had a significant increase at all time points analyzed (Figure 2 and Figure 3). For the lower doses the increases were significant at some of the time points tested after day 56 post-injection. IGF-I levels were within normal range for all dogs.

Increased IGF-I levels corresponding to higher GHRH levels are in agreement with other studies that utilized recombinant porcine GH ("pGH") in dogs. For example, there were dose-related increased serum IGF-I levels (approximately 2-10-food) that correlated with the elevated serum GH levels in pGH-treated dogs.

Although not wanting to be bound by theory, GHRH stimulates the production and release of GH from the anterior pituitary, which in turn stimulates the production of IGF-I from the liver and other target organs. Thus, an indication of increased systemic levels of GHRH and GH is an increase in serum IGF-I concentration.

### EXAMPLE 3

### INCREASED WEIGHT GAIN IN HEALTHY ANIMAL SUBJECTS

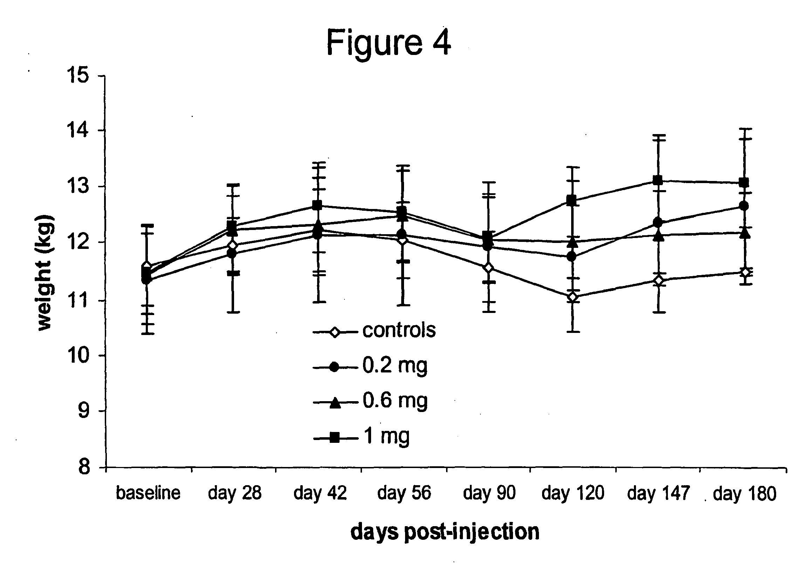

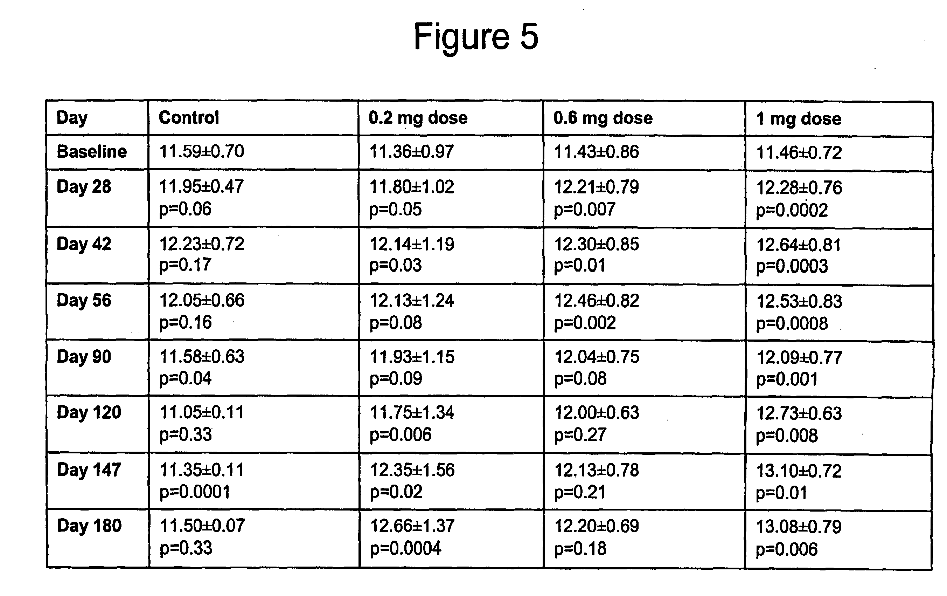

In order to show that increased levels of GHRH or biological equivalent thereof could alter metabolism in large healthy animals, body weight was determined. During the study, weights increased in plasmid-treated dogs, but the values were not significantly different among groups due to inter-animal variability. When each dog was compared to its own baseline (weight post-injection - weight pre-injection/weight pre-injection), all groups treated with plasmid had a significant weight increase at all time points analyzed (Figure 4 and 5).

These results are a good indicator that the metabolism of the dogs injected with pSP-HV-GHRH was altered in a dose dependent manner. In addition, the additional weight gain associated with increased production of GHRH also indicates that the levels of GH were increased. This observation is in agreement with other studies that utilized recombinant porcine GH ("pGH") in dogs. In one of these studies, recombinant pGH was administered for 14 weeks in dogs. Porcine GH caused increased body weight gain in mid- and high-dose groups (2.8 kg and 4.7 kg, respectively), compared to 0.4 kg and 0.8 kg in control and low-dose groups, respectively.

### EXAMPLE 4

### GHRH OR BIOLOGICAL EQUIVALENT TREATMENT DOES NOT AFFECT GLUCOSE METABOLISM IN HEALTHY ANIMAL SUBJECTS

Biochemical and blood chemistry, insulin and adrenocorticotropic ("ACTH") levels were assayed at Antech Diagnostic (Irvine, CA) at the previously stated time points. During anesthesia prior to injection and at days 93 and 180 at necropsy, a urine sample was drawn from each animal and a urine analysis was performed. All values were reviewed by a laboratory animal veterinarian. The following parameters were evaluated:

*Serum Chemistry:* amylase, globulin, gamma glutamyltransferase, total bilirubin, blood urea nitrogen, creatinine, total protein, albumin, direct bilirubin, serum alanine aminotransferase, serum aspartate aminotransferase, alkaline phosphatase, glucose, inorganic phosphorus, chloride, calcium, sodium, and potassium, triglyceride, and cholesterol.

*Hematology:* Erythrocyte counts (RBC), hematocrit, hemoglobin, total leukocyte count (WBC), and differential leukocyte counts (neutrophils, lymphocytes, monocytes, eosinophils, and basophils), platelet count, MCV, MCH, MCHC, and partial prothrombin time (PPT).

*Urine analysis:* color, consistency, volume, glucose, bilirubin, ketone (acetoacetic acid), specific gravity, blood, pH, protein, urobilinogen, nitrite, leukocytes, and microscopic examination of formed elements.

Groups of 8 dogs (4 males and 4 females) were injected with 200 mcg, 600mcg and 1000 mcg of pSP-HV-GHRH. A group of 4 dogs (2 males and 2 females) were used as controls. No statistical differences were found between experimental and control groups. Importantly, glucose and fasting insulin levels in all experimental groups and controls are within the normal range, which indicate that the therapy does not impair glucose metabolism.

### EXAMPLE 5

### GHRH OR BIOLOGICAL EQUIVALENT INCREASES RED BLOOD CELL PRODUCTION IN NORMAL HEALTH DOGS

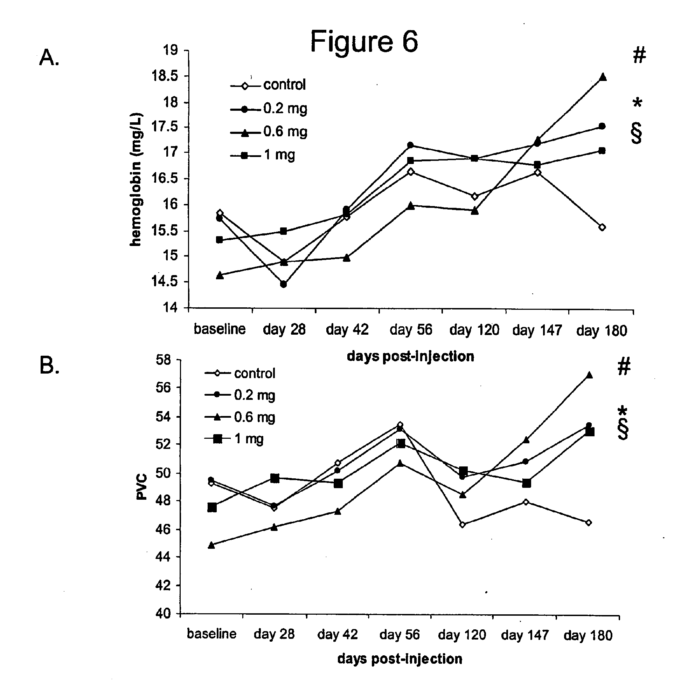

Groups of 8 dogs (4 males and 4 females) were injected with 200 mcg, 600mcg and 1000 mcg of pSP-HV-GHRH. A group of 4 dogs (2 males and 2 females) were used as controls. Several parameters were significantly different between controls and treated groups at different time points after treatment. Hemoglobin and packed cell volume ("PCV") were significantly increased in all treated dogs, least square mean < 0.05 (Figure 6), but within normal values for dogs. No dogs showed evidence of polycythemia following plasmid administration, indicating that this approach to stimulating the GHRH/GH/IGF-I axis respects normal physiologic levels of hemoglobin and red blood cells synthesis.

### EXAMPLE 6

### GHRH OR BIOLOGICAL EQUIVALENT TREATMENT EFFECTS ON BONE REMODELING

The phosphorus, calcium and calcium/phosphorous ratio were monitored for 180 days post injection. Groups of 8 dogs (4 males and 4 females) were injected with 200 mcg, 600mcg and 1000 mcg of pSP-HV-GHRH. A group of 4 dogs (2 males and 2 females) were used as controls. Calcium to phosphorous ratio, an indication of bone remodeling, was significantly increased in dogs in group IV (1 mg GHRH-plasmid treated group) versus controls at 180 days post-injection (2.75 ± 0.2 vs. 2.28 ± 0.04, *P* < 0.045).

### EXAMPLE 7

### GHRH OR BIOLOGICAL EQUIVALENT TREATMENT EFFECTS ON ANEMIA ASSOCIATED WITH KIDNEY FAILURE

In injected cats and dogs, a rapid correction of anemia was obtained, as early as twenty days after the plasmid injection.

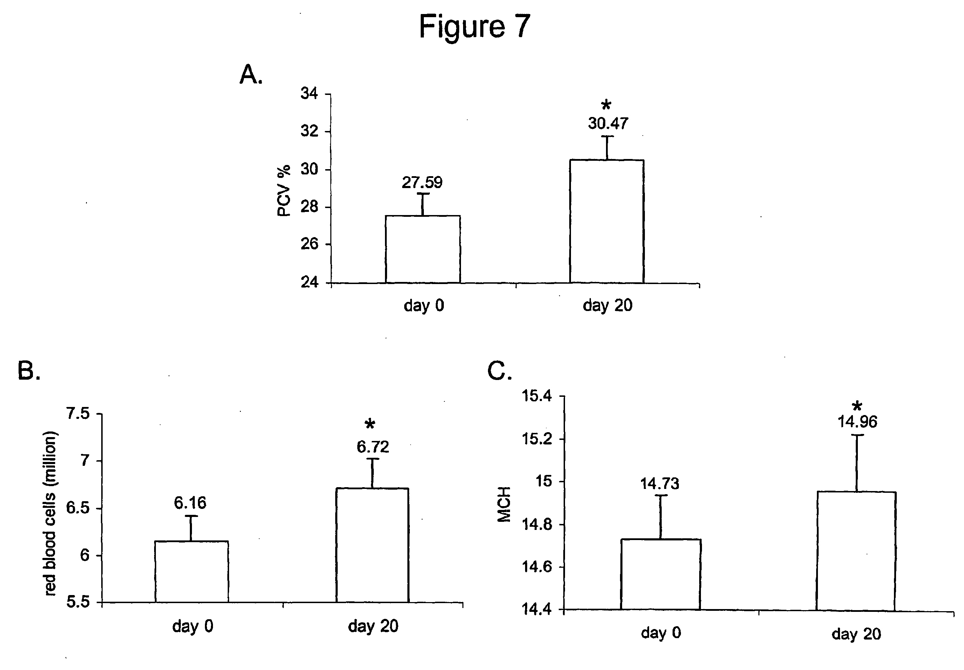

Figure 7 shows hematocrit (PCV), red blood cell count and mean red cell hemoglobin (MHC) in cats with chronic renal failure treated with plasmid-mediated GHRH therapy. The results are presented as means ± SEM. The analysis of hematocrit (PCV) for the GHRH-treated cats shows a 10% increase in their hematocrit levels * *P* < 0.0007, while red blood cells increased by 9%, * *P* < 0.0006. The mean red cell hemoglobin (MHC) for the GHRH-treated cats increased by 2%, reflecting a better utilization and incorporation into the red blood cells of the circulating iron * *P* < 0.013. Stimulation of hematopoiesis is also reflected in the dramatic 200% increase in the reticulocyte levels.

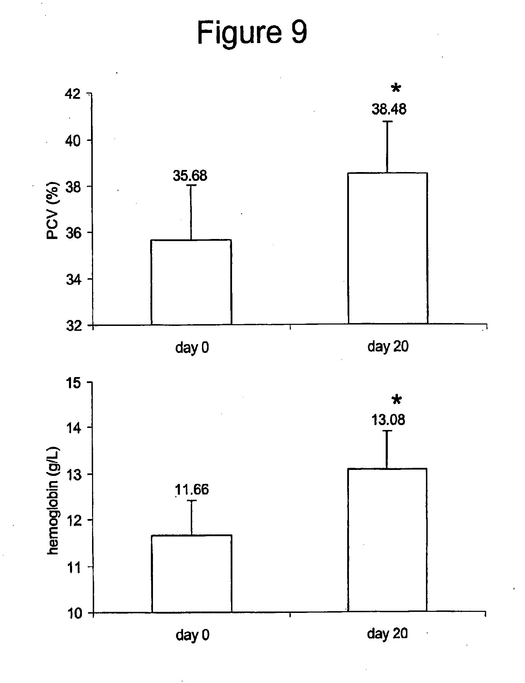

Figure 9 shows hematocrit (PCV) and hemoglobin in dogs with chronic renal failure treated with plasmid-mediated GHRH therapy. The results are presented as means ± SEM. The analysis of hematocrit (PCV) for the GHRH-treated dogs showed a 7.8% increase over the 20 days period, * *P* < 0.06. Hemoglobin for the GHRH-treated dogs increased by 12% in the same period of time, * *P* < 0.05. As in the case of cats, reticulocyte levels increased 140%.

In a pre-clinical study on dog cancer patients, a rapid correction of the anemia was obtained, as early as two weeks after the plasmid injection. At the beginning of the study, the patients were in a catabolic state, with hemoglobin (Hb), hematocrit ("PVC") and red blood cell ("RBC") values significantly lower than normal dogs. After the plasmid injection, the dogs entered a rapid reverse stage, and became biochemically anabolic, mimicking a rapid growth process, as in the study described previously ("growth hormone axis and the immune function") on young rats in the growth phase. Hb, PVC and RBC values increased with 10-25%, values significant statistically, and normalized two weeks after the beginning of the therapy. All values were within the normal limits throughout the experiment (RBC: 5.5-8.5million/ml; hemoglobin: 12-18 g/dl; hematocrit: 37-55%). Although not wanting to be bound by theory, it is likely that stimulation of the GHRH-GH-IGF-I axis in a catabolic state stimulates erytropoiesis through erythropoietin, transferrin, and other pertinent molecules. When the patients are reversed to a normal anabolic state, the natural GH effect is to induce a slight degree of anemia. Nevertheless, in patients with renal failure, the natural course of the disease is towards catabolism. Patients are maintained in balance by these contradictory mechanisms. Thus the Hb, PVC and RBC values are corrected to normal, but never exceed the upper normal limits.

### EXAMPLE 8

### GHRH OR BIOLOGICAL EQUIVALENT TREATMENT EFFECTS ON WASTING AND ABNORMAL PROTEIN METABOLISM ASSOCIATED WITH KIDNEY FAILURE

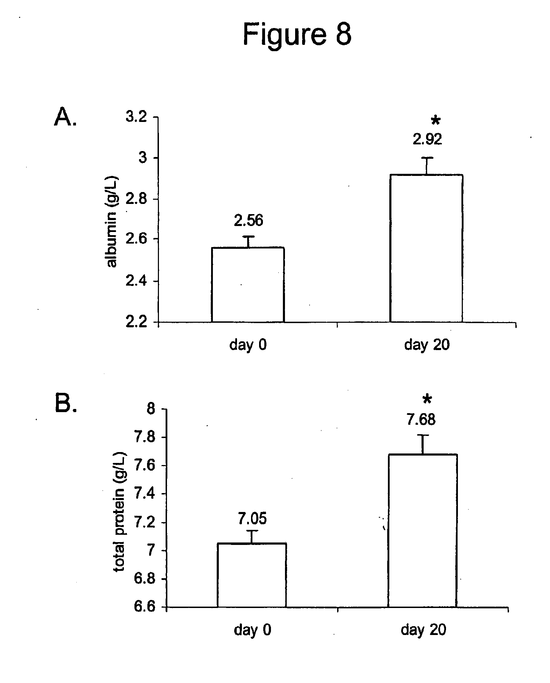

Malnutrition and wasting are important determinants of morbidity and mortality in patients with chronic renal failure on dialysis. Even patients with a relatively modest degree of chronic renal insufficiency are characterized by reduced lean body mass, bone mineral content, and basal energy expenditure (O'Sullivan et al., 2002). Figure 8 shows the albumin and total protein values in cats with chronic renal failure treated with plasmid-mediated GHRH therapy. The results are presented as means ± SEM. The analysis of albumin (normal range for cats 2.4-3.5g/dl) for the GHRH-treated cats showed a 14% increase at 20 days post-treatment, * *P* < 0.00001. In parallel, total protein (normal range for cats 5.3-8.5 g/dl) in GHRH-treated cats increased by 9%, * *P* < 0.00001. Similar increases were seen in the GHRH-treated dogs: a 7.5% increase in albumin (normal range for dogs 2.8-3.9g/dl) and a 5.6% increase in total protein values (normal range for dogs 5.7-7.6/dl) at 20 day after therapy. These changes are correlated with an improved protein metabolism, and a change from a catabolic state to an anabolic state in these patients.

### EXAMPLE 9

### GHRH OR BIOLOGICAL EQUIVALENT TREATMENT EFFECTS ON KIDNEY FUNCTION IN PATIENTS WITH KIDNEY FAILURE

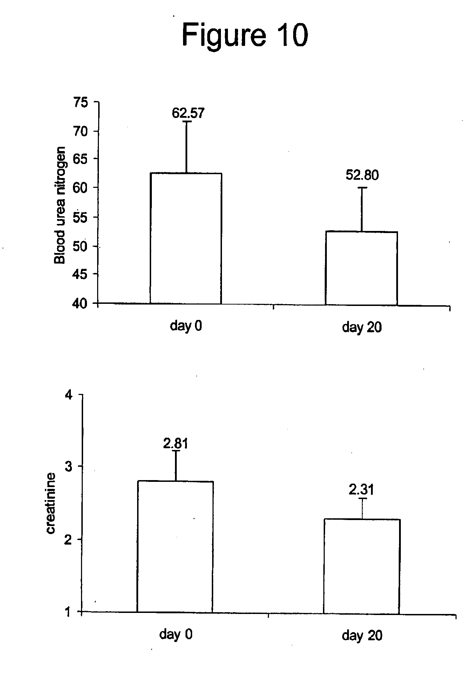

Chronic renal failure ("CRF") has limited therapeutic options for both humans and pets, mostly dialysis and kidney transplant. Any adjuvant therapy that delays tissue destruction in these patients is of crucial interest. Figure 10 shows blood urea nitrogen ("BUN") and creatinine values (indicators of renal function) in dogs with chronic renal failure treated with plasmid-mediated GHRH therapy. The results are presented as means ± SEM. Analysis of blood urea nitrogen for the GHRH-treated dogs showed a 16% reduction in BUN, and in concert a 17.6% decrease in creatinine levels. The rate of decline in glomerular filtration rate (decrease in creatinine clearance, increase in serum creatinine) is highly correlated with the renal survival rate.

### EXAMPLE 10

### GHRH OR BIOLOGICAL EQUIVALENT TREATMENT EFFECTS ON IGF-I AND ITS CONSEQUENCES IN PATIENTS WITH KIDNEY FAILURE

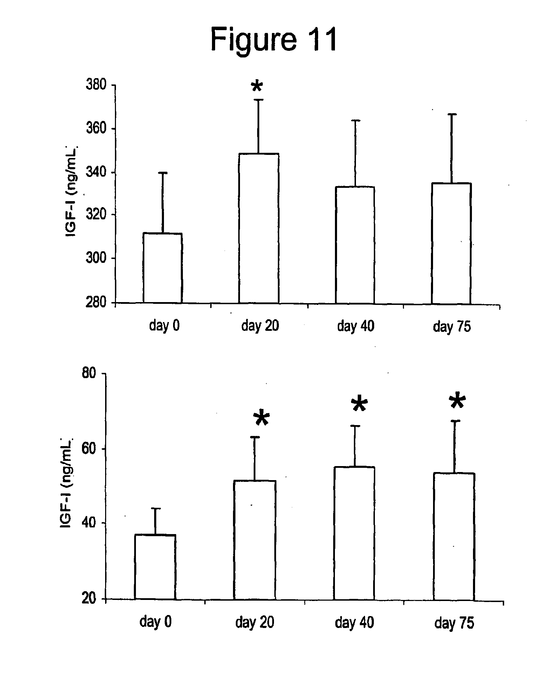

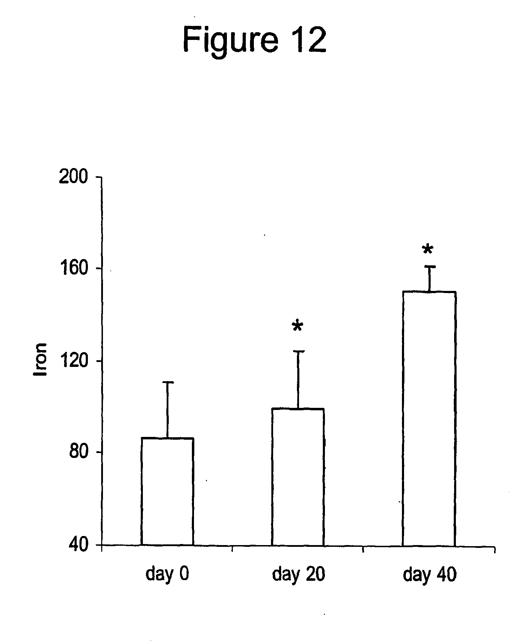

Cats and dogs with chronic renal failure treated with plasmid-GHRH showed an increase in their IGF-I levels, the downstream effector of GHRH, a sign that the therapy was effective in these very ill animals. Cat specific GHRH is indicated in SEQ ID No.: 24, and dog specific GHRH is indicated in SEQ ID No.: 23. Figure 11 shows that 75% of cats and dogs had significantly increased IGF-I levels troughtout the study period, and that the increases were statistically significant (* P < 0.05). Without being bound to the theory, most probably the mechanism of correction of anemia in these animals was not increased erythropoietin (as described in publications using growth hormone or other recombinant proteins), but increased transfferin. This finding is suggested by the increased circulating levels of iron seen in the treated animals (Figure 12). The results are presented as means ± SEM, * P < 0.05.

### EXAMPLE 11

### GHRH OR BIOLOGICAL EQUIVALENT TREATMENT PREVENTS RENAL FAILURE SECONDARY TO LATE STAGE PRIMARY DISEASE

Plasmid GHRH administration is able to prevent kidney dysfunction associated with the advanced stages of tumor necrosis in mice. In the group of treated animals, the necrosis of the tumors may have further affected metabolic functions. Serum values for BUN and creatinine were within normal range for both treated male and female groups, while increased in controls. The mean serum BUN and creatinine levels at the end of the study in GHRH-treated animals were 15.0% (*P* < 0.04) and 20.6% (*P* = 0.025) lower than those of control male mice, respectively. Mean alkaline phosphatase was 51.2% lower in the GHRH-plasmid treated male animals (*P* < 0.0002) relative to control mice. In female mice, there was a 26% decrease in mean serum creatinine levels relative to controls. At necropsy, kidney weight was significantly lower in the male control animals (already in uremia) compared to GHRH plasmid-injected animals. Thus, plasmid GHRH-treated mice with large tumors preserved normal kidney function for longer periods of time.

### EXAMPLE 12

### GHRH OR BIOLOGICAL EQUIVALENT TREATMENT EFFECTS ON QUALITY OF LIFE AND WELFARE OF PATIENTS WITH KIDNEY FAILURE

Health-related quality of life is increasingly recognized as an important outcome in clinical research and patient care. A large number of reports focus on the quality of life in the setting of end-stage renal disease. Significant impairment in health-related quality of life is seen with renal insufficiency, due to anemia, wasting, immune dysfunction, and other complications. Results from these studies indicate that the current emphasis on clinical interventions aimed at preserving renal function are likely to improve the negative impact of kidney disease on health-related quality of life. Owners completed quality of life questionnaires at each visit. The activity levels in treated animals increased in average by 70%, while the appetite increased by 40%.

### EXAMPLE 13

### PHARMACOLOGICAL AND TOXICOLOGICAL EFFECTS OF EXOGENOUS GH ADMINISTRATION IN NORMAL ANIMAL SUBJECTS

Because porcine GH (pGH) is structurally identical to canine GH, pGH was used in different studies on dogs. In one of these studies, pGH was administered for 14 weeks in dogs. Porcine GH caused increased body weight gain in mid- and high-dose groups (2.8 kg and 4.7 kg, respectively), compared to 0.4 kg and 0.8 kg in control and low-dose groups, respectively. In pGH-treated dogs, increased skin thickness seen grossly correlated histologically with increased dermal collagen. There was no gross or histomorphological evidence of edema. There were dose-related increased serum IGF-I levels (approximately 2-10-fold) that correlated with the elevated serum GH levels in pGH-treated dogs. Also, increased serum insulin levels through the mid dose were seen throughout the study. In high-dose dogs, the insulin levels remained elevated over 24 hours after dosage. The serum glucose levels in fasted dogs remained within the control range, and there was no chronic hyperglycemia based on glycosylated hemoglobin levels. Renal glomerular changes, significant polyuria with decreased urine specific gravity, and increased serum insulin levels suggested that the dogs had early insulin-resistant diabetes. There was minimal or no biologically significant effect of pGH on serum T3, T4, and cortisol levels in dogs. Other serum biochemical changes in pGH-treated dogs included decreased urea nitrogen and creatinine, and increased potassium, cholesterol, and triglycerides. Significant increases in serum calcium and phosphorous levels and alkaline phosphatase activity (bone isozyme) correlated with the histological changes in bone. In pGH-treated dogs, there was a dose-related normochromic, normocytic, nonregenerative anemia. The changes described above, except for the anemia, are related to either anabolic or catabolic effects of high doses of GH (Prahalada et al., 1998).

### EXAMPLE 14

### GHRH OR ITS FUNCTIONAL BIOLOGICAL EQUIVALENTS ARE USED AS RECOMBINANT PROTEINS IN THE TREATMENT (OR MANAGEMENT) OF CHRONIC RENAL FAILURE

GHRH or its functional biological equivalents are used as recombinant proteins in the treatment or management of renal failure and impaired growth that is associated with this condition. Several alterations in GH secretion with normal GHRH levels have been reported in patients with chronic renal insufficiency (Diez et al., 1999a). Very often, children with chronic renal failure have alterations in their GH axis, and a correction of this disequilibrium is necessary to achieve normal growth in these patients (Pasqualini et al., 1996). GHRH peptide is used for this purpose. The correction of anemia in CRF patients is followed by better GH response to GHRH stimulation and consequently enhanced growth (Cremagnani et al., 1993a). Also, IGF-I, the downstream effector of GHRH, is shown to maintain glomerular filtration via direct action on the glomerular vasculature and to accelerate tubular regeneration by enhancing DNA synthesis in proximal tubule cells (Hammerman, 1999; Vijayan et al., 1999).

GHRH or its functional biological equivalents can be administered directly to subjects with renal failure in order to treat this condition and its complications. Mice with renal failure are divided into two groups. The treated group receives subcutaneous or intravenous injections of recombinant GHRH peptide. The control group is injected with vehicle alone. Ten days after injection, the treated group will show an increased level of GH, weight and length compared to the control group, and a better renal function. Thus, mice with renal failure treated with GHRH peptide experience improved renal function. However, the necessity for frequent injections of the GHRH or other recombinant proteins is a limiting factor as the patient's (or patient's owner's) compliance is typically low.

Although not wanting to be bound by theory, it is believed that an increase in GHRH will increase GH levels sufficiently to treat kidney failure, treat anemia, reverse wasting, treat immune dysfunction, and extend life expectancy for subjects with renal insufficiency. Hormones (*e.g*. GHRH and GH) often contain a complex feedback-regulated pathway, which are further complicated by chronic conditions such as renal failure, cancer or AIDS. As little as 0.1 mg plasmid delivered under the proper electroporation conditions described herein has an important biological impact that improves kidney function, treat anemia, reversed wasting, and extend life in an ailing feline or canine subject. This plasmid quantity was 100 fold lower than the theoretical one, or the average 1 mg/kg used in rodents.

The treatment of kidney failure and its complications are a consequence of the GHRH molecules present in the subject's circulation, regardless of the means of the delivery. For example, one would obtain the same effect by delivering the appropriate quantities of GHRH or an analog thereof by classical recombinant protein therapy or nucleic acid transfer. However, the method of delivering nucleic acid sequences to the cells of a subject is highly dependent on specific diseases and the encoded gene. Accordingly, successful plasmid-mediated supplementation requires accurate delivery of encoded sequences to the cells of a subject that results in expression of the gene product at levels appropriate to produce a biological effect. The duration of treatment will extend through the course of the disease symptoms, and possibly continuously.

### REFERENCES CITED

### U.S. PATENT DOCUMENTS

U.S. Patent No. 6,274,158 issued on August 14, 2001 with Czeizler et al. listed as inventors.
U.S. Patent No. 6,177,554 issued on January 23, 2001 with Woo, et al. listed as inventors.
U.S. Patent No. 5,994,624 issued on November 30, 1999 with Trolinder , et al. as inventors.
U.S. Patent No. 5,981,274 issued on November 9, 1999 with Tyrrell , et al. as inventors.
U.S. Patent No. 5,955,365 issued on September 21, 1999 with Szoka, et al. listed as inventors.
U.S. Patent No. 5,945,100 issued on August 31, 1999 with Fick as inventor.
U.S. Patent No. 5,935,819 issued on August 10, 1999 with Eichner, et al. listed as inventors.
U.S. Patent No. 5,928,906 issued on July 27, 1999 with Koster, et al. listed as inventors.
U.S. Patent No. 5,925,565 issued on July 20, 1999 with Berlioz, et al. listed as inventors.
U.S. Patent No. 5,847,066 issued on December 8, 1998 with Coy et al. listed as inventors.
U.S. Patent No. 5,846,936 issued on December 8, 1998 with Felix et al. listed as inventors.
U.S. Patent No. 5,846,528 issued on December 8, 1997 with Podsakoff et al. listed as inventors.
U.S. Patent No. 5,792,747 issued on August 11, 1998 with Schally et al. listed as inventors.
U.S. Patent No. 5,789,215 issued on August 4, 1998 with Berns, et al. listed as inventors.
U.S. Patent No. 5,780,448 issued on July 14, 1998 with Davis as inventor.
U.S. Patent No. 5,776,901 issued on July 7, 1998 with Bowers et al. listed as inventors.
U.S. Patent No. 5,736,524 issued on April 7, 1998 with Content et al. listed as inventors.
U.S. Patent No. 5,704,908 issued on January 6, 1998 with Hofmann et al. listed as inventors.
U.S. Patent No. 5,702,932 issued on December 30, 1997 with Hoy et al. listed as inventors.
U.S. Patent No. 5,696,089 issued on December 9, 1997 with Felix et al. listed as inventors.
U.S. Patent No. 5,656,610 issued on August 12, 1997 with Shuler et al. listed as inventors.
U.S. Patent No. 5,610,042 issued on March 11, 1997 with Chang et al. listed as inventors.
U.S. Patent No. 5,591,616 issued on January 7, 1997 with Hiei et al. listed as inventors.
U.S. Patent No. 5,589,466 issued on December 31, 1996 with Felgner et al. listed as inventors.
U.S. Patent No. 5,580,859 issued on December 3, 1996 with Felgner et al. listed as inventors.
U.S. Patent No. 5,563,055 issued on October 8, 1996 with Townsend et al. listed as inventors.
U.S. Patent No. 5,550,318 issued on August 27, 1996 with Adams et al. listed as inventors.
U.S. Patent No. 5,538,880 issued on July 23, 1996 with Lundquist et al. listed as inventors.
U.S. Patent No. 5,538,877 issued on July 23, 1996 with Lundquist et al. listed as inventors.
U.S. Patent No. 5,486,505 issued on January 23, 1996 with Bowers et al. listed as inventors.
U.S. Patent No. 5,464,765 issued on November 7, 1995 with Coffee et al. listed as inventors.
U.S. Patent No. 5,439,440 issued on August 8, 1995 with Hofmann listed as inventor.
U.S. Patent No. 5,384,253 issued on January 24, 1995 with Krzyzek et al. listed as inventors.
U.S. Patent No. 5,374,544 issued on December 20, 1994 with Schwartz et al. listed as inventors.
U.S. Patent No. 5,322,783 issued on June 21, 1994 with Tomes et al. listed as inventors.
U.S. Patent No. 5,302,523 issued on April 12, 1994 with Coffee et al. listed as inventors.
U.S. Patent No. 5,298,422 issued on March 29, 1994 with Schwartz et al. listed as inventors.
U.S. Patent No. 5,292,721 issued on March 8, 1994 with Boyd et al. listed as inventors.
U.S. Patent No. 5,137,872 issued on August 11, 1992 with Seely et al. listed as inventors.
U.S. Patent No. 5,134.120 issued on July 28, 1992 with Boyd et al. listed as inventors.
U.S. Patent No. 5,084,442 issued on January 28, 1992 with Felix et al. listed as inventors.
U.S. Patent No. 5,061,690 issued on October 29, 1991 with Kann et al. listed as inventors.
U.S. Patent No. 5,036,045 issued on July 30, 1991 with Thorner listed as the inventor.
U.S. Patent No. 5,023,322 issued on June 11, 1991 with Kovacs et al. listed as inventors.
U.S. Patent No. 4,956,288 issued on September 11, 1990 with Barsoum listed as inventor.
U.S. Patent No. 4,952,500 issued on August 28,1990 with Finnerty et al. listed as inventors.
U.S. Patent No. 4,839,344 issued on June 13, 1989 with Bowers et al. listed as inventors.
U.S. Patent No. 4,833,166 issued on May 23, 1989 with Grosvenor et al. listed as inventors.
U.S. Patent No. 4,684,611 issued on August 4, 1987 with Schilperoort et al. listed as inventors.
U.S. Patent No. 4,683,202 issued on July 28, 1987 with Mullis listed as inventor.
U.S. Patent No. 4,410,512 issued on October 18, 1983 with Bowers et al. listed as inventors.
U.S. Patent No. RE33,699 issued on September 24, 1991 with Drengler listed as the inventor.
U.S. Patent No. 4,228,158 issued on October 14, 1980 with Momany et al. listed as inventors.
U.S. Patent No. 4,228,156 issued on October 14, 1980 with Momany et al. listed as inventors. '
U.S. Patent No. 4,226,857 issued on October 7, 1980 with Momany et al. listed as inventors.
U.S. Patent No. 4,224,316 issued on September 23, 1980 with Momany et al. listed as inventors.
U.S. Patent No. 4,223,021 issued on September 16, 1980 with Momany et al. listed as inventors.
U.S. Patent No. 4,223,020 issued on September 16, 1980 with Momany et al. listed as inventors.
U.S. Patent No. 4,223,019 issued on September 16, 1980 with Momany et al. listed as inventors.

### Reference List

Aihara, H. and J. Miyazaki. 1998. Gene transfer into muscle by electroporation in vivo. Nat. Biotechnol. 16:867-870.
Aimaretti, G., G. Corneli, P. Razzore, S. Bellone, C. Baffoni, J. Bellone, F. Camanni, and E. Ghigo. 1998. Usefulness of IGF-I assay for the diagnosis of GH deficiency in adults. J. Endocrinol. Invest 21:506-511.
Al Suwaidi, J., D. N. Reddan, K. Williams, K. S. Pieper, R. A. Harrington, R. M. Califf, C. B. Granger, E. M. Ohman, and D. R. Holmes, Jr. 2002. Prognostic implications of abnormalities in renal function in patients with acute coronary syndromes. Circulation 106:974-980.
Almendro, N., T. Bellon, C. Rius, P. Lastres, C. Langa, A. Corbi, and C. Bernabeu. 1996. Cloning of the human platelet endothelial cell adhesion molecule-1 promoter and its tissue-specific expression. Structural and functional characterization. J. Immunol. 157:5411-5421.
Aratani, Y., R. Okazaki, and H. Koyama. 1992. End extension repair of introduced targeting vectors mediated by homologous recombination in mammalian cells. Nucleic Acids Res. 20:4795-4801.
Argente, J., J. Pozo, and J. A. Chowen. 1996. The growth hormone axis: control and effects. Hormone Research 45 Suppl 1:9-11.
Barber, M. D., J. A. Ross, and K. C. Fearon. 1999. Cancer cachexia. Surg. Oncol. 8:133-141.
Bartlett, D. L., S. Charland, and M. H. Torosian. 1994. Growth hormone, insulin, and somatostatin therapy of cancer cachexia. Cancer 73:1499-1504.
Bercu, B. B., R. F. Walker, 1997. Growth Hormone Secretagogues In Children With Altered Growth. Acta Paediatrica 86:102-106.
Bettan, M., F. Emmanuel, R. Darteil, J. M. Caillaud, F. Soubrier, P. Delaere, D. Branelec, A. Mahfoudi, N. Duverger, and D. Scherman. 2000. High-level protein secretion into blood circulation after electric pulse-mediated gene transfer into skeletal muscle. Mol. Ther. 2:204-210.
Blethen, S. L. 1995. Complications of growth hormone therapy in children. Curr. Opin. Pediatr. 7:466-471.
Blethen, S. L. and M. H. MacGillivray. 1997. A risk-benefit assessment of growth hormone use in children. Drug Saf 17:303-316.
Blethen, S. L. and A. C. Rundle. 1996. Slipped capital femoral epiphysis in children treated with growth hormone. A summary of the National Cooperative Growth Study experience. Horm. Res. 46:113-116.
Bohlen, P., F. Esch, P. Brazeau, N. Ling, and R Guillemin. 1983. Isolation and characterization of the porcine hypothalamic growth hormone releasing factor. Biochem. Biophys. Res. Commun. 116:726-734.
Boshart, M., F. Weber, G. Jahn, K. Dorsch-Hasler, B. Fleckenstein, and W. Schaffner. 1985. A very strong enhancer is located upstream of an immediate early gene of human cytomegalovirus. Cell 41:521-530.
Braun, W. E. 2002. Update on kidney transplantation: increasing clinical success, expanding waiting lists. Cleve. Clin. J Med. 69:501-504.
Bureau, M. F., J. Gehl, V. Deleuze, L. M. Mir, and D. Scherman. 2000. Importance of association between permeabilization and electrophoretic forces for intramuscular DNA electrotransfer. Biochim. Biophys. Acta 1474:353-359.
Carbonelli, D. L., E. Corley, M. Seigelchifer, and J. Zorzopulos. 1999. A plasmid vector for isolation of strong promoters in Escherichia coli. FEMS Microbiol. Lett. 177:75-82.
Chandler, S. D., A. Mayeda, J. M. Yeakley, A. R. Krainer, and X. D. Fu. 1997. RNA splicing specificity determined by the coordinated action of RNA recognition motifs in SR proteins. Proc. Natl. Acad. Sci. U. S. A 94:3596-3601.
Chen, C. and H. Okayama. 1987. High-efficiency transformation of mammalian cells by plasmid DNA. Mol. Cell Biol. 7:2745-2752.
Chen, Y., F. C. Fervenza, and R. Rabkin. 2001. Growth factors in the treatment of wasting in kidney failure. J Ren Nutr. 11:62-66.
Chevalier, R. L., S. Goyal, A. Kim, A. Y. Chang, D. Landau, and D. LeRoith. 2000. Renal tubulointerstitial injury from ureteral obstruction in the neonatal rat is attenuated by IGF-1. Kidney Int. 57:882-890.
Christ, E. R, M. H. Cummings, N. B. Westwood, B. M. Sawyer, T. C. Pearson, P. H. Sonksen, and D. L. Russell-Jones. 1997. The importance of growth hormone in the regulation of erythropoiesis, red cell mass, and plasma volume in adults with growth hormone deficiency. J. Clin. Endocrinol. Metab 82:2985-2990.
Claustres, M., P. Chatelain, and C. Sultan. 1987. Insulin-like growth factor I stimulates human erythroid colony formation in vitro. J Clin. Endocrinol. Metab 65:78-82.
Cocea, L. 1997. Duplication of a region in the multiple cloning site of a plasmid vector to enhance cloning-mediated addition of restriction sites to a DNA fragment. Biotechniques 23:814-816.
Corpas, E., S. M. Harman, M. A. Pineyro, R. Roberson, and M. R. Blackman. 1993, Continuous subcutaneous infusions of growth hormone (GH) releasing hormone 1-44 for 14 days increase GH and insulin-like growth factor-I levels in old men. Journal of Clinical Endocrinology & Metabolism 76:134-138.
Correa, P. N., D. Eskinazi, and A. A. Axelrad. 1994. Circulating erythroid progenitors in polycythemia vera are hypersensitive to insulin-like growth factor-1 in vitro: studies in an improved serum-free medium. Blood 83:99-112.
Cremagnani, L., L. Cantalamessa, A. Orsatti, L. Vigna, F. Vallino, and G. Buccianti. 1993a. Recombinant human erythropoietin (rhEPO) treatment potentiates growth hormone (GH) response to growth hormone releasing hormone (GHRH) stimulation in hemodialysis patients [see comments]. Clinical Nephrology 39:282-286.
Cremagnani, L., L. Cantalamessa, A. Orsatti, L. Vigna, F. Vallino, and G. Buccianti. 1993b. Recombinant human erythropoietin (rhEPO) treatment potentiates growth hormone (GH) response to growth hormone releasing hormone (GHRH) stimulation in hemodialysis patients. Clin. Nephrol. 39:282-286.
Crook, E. D., D. O. Washington, and J. M. Flack. 2002. Screening and prevention of chronic kidney disease. J. Natl. Med. Assoc. 94:55S-62S.
Dai, B., H. Wu, E. Holthuizen, and P. Singh. 2001. Identification of a novel cis element required for cell density-dependent down-regulation of insulin-like growth factor-2 P3 promoter activity in Caco2 cells. J. Biol. Chem. 276:6937-6944.
Danko, I. and J. A. Wolff. 1994. Direct gene transfer into muscle. [Review]. Vaccine 12:1499-1502.
Darquet, A. M., B. Cameron, P. Wils, D. Scherman, and J. Crouzet. 1997. A new DNA vehicle for nonviral gene delivery: supercoiled minicircle. Gene Ther. 4:1341-1349.
Darquet, A. M., R. Rangara, P. Kreiss, B. Schwartz, S. Naimi, P. Delaere, J. Crouzet, and D. Scherman. 1999. Minicircle: an improved DNA molecule for in vitro and in vivo gene transfer. Gene Ther. 6:209-218.
Davis, H. L., R. G. Whalen, and B. A. Demeneix. 1993. Direct gene transfer into skeletal muscle in vivo: factors affecting efficiency of transfer and stability of expression. Human Gene Therapy 4:151-159.
Davis, M. P. and D. Dickerson. 2000. Cachexia and anorexia: cancer's covert killer. Support. Care Cancer 8:180-187.
de Boer, H., G. J. Blok, C. Popp-Snijders, L. Stuurman, R. C. Baxter, and d. Van, V. 1996. Monitoring of growth hormone replacement therapy in adults, based on measurement of serum markers. J. Clin. Endocrinol. Metab 81:1371-1377.
Diez, J., P. Iglesias, J. Sastre, J. Mendez, R. Selgas, and A. Gomez-Pan. 1996a. Growth hormone responses to growth hormone-releasing hormone and clonidine before and after erythropoietin therapy in CAPD patients. Nephron 74:548-554.
Diez, J., P. Iglesias, J. Sastre, J. Mendez, R. Selgas, and A. Gomez-Pan. 1996b. Growth hormone responses to growth hormone-releasing hormone and clonidine before and after erythropoietin therapy in CAPD patients. Nephron 74:548-554.
Diez, J. J., P. Iglesias, A. Aguilera, M. A. Bajo, and R. Selgas. 1999a. Effects of cholinergic muscarinic blockade on growth hormone responses to growth hormone-releasing hormone in uraemic patients. Nephrol. Dial. Transplant. 14:1704-1709.
Diez, J. J., P. Iglesias, J. Sastre, A. Aguilera, M. A. Bajo, J. Mendez, P. A. Gomez, and R. Selgas. 1999b. Long-term effects of recombinant human erythropoietin therapy on growth hormone secretion in uremic patients undergoing peritoneal dialysis. Metabolism 48:210-216.
Dolnik, V., M. Novotny, and J. Chmelik. 1993. Electromigration behavior of poly-(L-glutamate) conformers in concentrated polyacrylamide gels. Biopolymers 33:1299-1306.
Dorsch-Hasler, K., G. M. Keil, F. Weber, M. Jasin, W. Schaffner, and U. H. Koszinowski. 1985. A long and complex enhancer activates transcription of the gene coding for the highly abundant immediate early mRNA in murine cytomegalovirus. Proc. Natl. Acad. Sci. U. S. A 82:8325-8329.
Draghia-Akli, R., M. L. Fiorotto, L. A. Hill, P. B. Malone, D. R. Deaver, and R. J. Schwartz. 1999. Myogenic expression of an injectable protease-resistant growth hormone-releasing hormone augments long-term growth in pigs. Nat. Biotechnol. 17:1179-1183.
Draghia-Akli, R., A. S. Khan, K. K. Cummings, D. Parghi, R. H. Carpenter, and P. A. Brown. 2002a. Electrical Enhancement of Formulated Plasmid Delivery in Animals. Technology in Cancer Research & Treatment 1:365-371.
Draghia-Akli, R., X. G. Li, and R. J. Schwartz. 1997. Enhanced growth by ectopic expression of growth hormone releasing hormone using an injectable myogenic vector. Nat. Biotechnol. 15:1285-1289.
Draghia-Akli, R., P. B. Malone, L. A. Hill, K. M. Ellis, R. J. Schwartz, and J. L. Nordstrom. 2002b. Enhanced animal growth via ligand-regulated GHRH myogenic-injectable vectors. FASEB J. 16:426-428.
Dubreuil, P., D. Petitclerc, G. Pelletier, P. Gaudreau, C. Farmer, Mowles, TF, and P. Brazeau. 1990. Effect of dose and frequency of administration of a potent analog of human growth hormone-releasing factor on hormone secretion and growth in pigs. Journal of Animal Science 68:1254-1268.
Duck, S. C., H. P. Schwarz, G. Costin, R. Rapaport, S. Arslanian, A. Hayek, M. Connors, and J. Jaramillo. 1992. Subcutaneous growth hormone-releasing hormone therapy in growth hormone-deficient children: first year of therapy. Journal of Clinical Endocrinology & Metabolism 75:1115-1120.
Elliott, J., J. M. Rawlings, P. J. Markwell, and P. J. Barber. 2000. Survival of cats with naturally occurring chronic renal failure: effect of dietary management. J Small Anim Pract. 41:235-242.
Esch, F. S., P. Bohlen, N. C. Ling, P. E. Brazeau, W. B. Wehrenberg, M. O. Thorner, M. J. Cronin, and R. Guillemin. 1982. Characterization of a 40 residue peptide from a human pancreatic tumor with growth hormone releasing activity. Biochemical & Biophysical Research Communications 109:152-158.
Evans, W. S., M. L. Vance, D. L. Kaiser, R. P. Sellers, J. -L. Borges, T. R. Downs, L. A. Frohman, J. Rivier, W. Vale, and M. O. Thorner. 1985. Effects of intravenous, subcutaneous, and intranasal administration of growth hormone (GH)-releasing hormone-40 on serum GH concentrations in normal men. Journal of Clinical Endocrinology & Metabolism 61:846-850.
Fechheimer, M., J. F. Boylan, S. Parker, J. E. Sisken, G. L. Patel, and S. G. Zimmer. 1987. Transfection of mammalian cells with plasmid DNA by scrape loading and sonication loading. Proc. Natl. Acad. Sci. U. S. A 84:8463-8467.
Fervenza, F. C., M. M. Friedlaender, J. O. Ike, and R. Rabkin. 1998. Insulin-like growth factor-I treatment to enhance renal function in advanced chronic renal failure. Ren Fail. 20:349-356.
Fewell, J. G., F. MacLaughlin, V. Mehta, M. Gondo, F. Nicol, E. Wilson, and L. C. Smith. 2001. Gene therapy for the treatment of hemophilia B using PINC-formulated plasmid delivered to muscle with electroporation. Mol. Ther. 3:574-583.
Franssen, F. M., E. F. Wouters, and A. M. Schols. 2002. The contribution of starvation, deconditioning and ageing to the observed alterations in peripheral skeletal muscle in chronic organ diseases. Clin. Nutr. 21:1-14.
Frohman, L. A., T. R. Downs, E. P. Heimer, and A. M. Felix. 1989. Dipeptidylpeptidase IV and trypsin-like enzymatic degradation of human growth hormone-releasing hormone in plasma. J. Clin. Invest. 83:1533-1540.
Frohman, L. A., J. L. Thominet, C. B. Webb, M. L. Vance, H. Uderman, J. Rivier, W. Vale, and M. O. Thorner. 1984. Metabolic clearance and plasma disappearance rates of human pancreatic tumor growth hormone releasing factor in man. J. Clin. Invest. 73:1304-1311.
Fryer, A. D. and D. B. Jacoby. 1993. Effect of inflammatory cell mediators on M2 muscarinic receptors in the lungs. Life Sci. 52:529-536.
Gehl, J., T. Skovsgaard, and L. M. Mir. 1998. Enhancement of cytotoxicity by electropermeabilization: an improved method for screening drugs. Anticancer Drugs 9:319-325.
Gehl, J., T. H. Sorensen, K. Nielsen, P. Raskmark, S. L. Nielsen, T. Skovsgaard, and L. M. Mir. 1999. In vivo electroporation of skeletal muscle: threshold, efficacy and relation to electric field distribution. Biochim. Biophys. Acta 1428:233-240.
German, M., S. Ashcroft, K. Docherty, H. Edlund, T. Edlund, S. Goodison, H. Imura, G. Kennedy, O. Madsen, D. Melloul, and . 1995. The insulin gene promoter. A simplified nomenclature. Diabetes 44:1002-1004.
Gilbert, R. A., M. J. Jaroszeski, and R. Heller. 1997. Novel electrode designs for electrochemotherapy. Biochim. Biophys. Acta 1334:9-14.
Gopal, T. V. 1985. Gene transfer method for transient gene expression, stable transformation, and cotransformation of suspension cell cultures. Mol. Cell Biol. 5:1188-1190.
Graham, F. L. and A. J. van der Eb. 1973. Transformation of rat cells by DNA of human adenovirus 5. Virology 54:536-539.
Guillemin, R., P. Brazeau, P. Bohlen, F. Esch, N. Ling, and W. B. Wehrenberg. 1982. Growth hormone-releasing factor from a human pancreatic tumor that caused acromegaly. Science 218:585-587.
Hafez, I. M., N. Maurer, and P. R. Cullis. 2001. On the mechanism whereby cationic lipids promote intracellular delivery of polynucleic acids. Gene Ther. 8:1188-1196.
Hamm, A., N. Krott, I. Breibach, R. Blindt, and A. K. Bosserhoff. 2002. Efficient transfection method for primary cells. Tissue Eng 8:235-245.
Hammerman, M. R. 1999. The growth hormone-insulin-like growth factor axis in kidney re-revisited. Nephrol. Dial. Transplant. 14:1853-1860.
Hansen, R. D., C. Raja, and B. J. Allen. 2000. Total body protein in chronic diseases and in aging. Ann. N. Y. Acad. Sci 904:345-52.:345-352.
Harland, R. and H. Weintraub. 1985. Translation of mRNA injected into Xenopus oocytes is specifically inhibited by antisense RNA. J. Cell Biol. 101:1094-1099.
Heller, R., M. J. Jaroszeski, L. F. Glass, J. L. Messina, D. P. Rapaport, R. C. DeConti, N. A. Fenske, R. A. Gilbert, L. M. Mir, and D. S. Reintgen. 1996. Phase I/II trial for the treatment of cutaneous and subcutaneous tumors using electrochemotherapy. Cancer 77:964-971.
Herzog, R. W., J. D. Mount, V. R. Arruda, K. A. High, and C. D. Lothrop, Jr. 2001. Muscle-directed gene transfer and transient immune suppression result in sustained partial correction of canine hemophilia B caused by a null mutation. Mol. Ther. 4:192-200.
Horlick, R. A. and P. A. Benfield. 1989. The upstream muscle-specific enhancer of the rat muscle creatine kinase gene is composed of multiple elements. Mol. Cell Biol. 9:2396-2413.
Hostetter, T. H. and M. Lising. 2002. National Kidney Disease Education Program. J. Natl. Med. Assoc. 94:72S-75S.
Hughes, K. L., M. R. Slater, S. Geller, W. J. Burkholder, and C. Fitzgerald. 2002. Diet and lifestyle variables as risk factors for chronic renal failure in pet cats. Prev. Vet. Med. 55:1-15.
Inouye, C., P. Remondelli, M. Karin, and S. Elledge. 1994. Isolation of a cDNA encoding a metal response element binding protein using a novel expression cloning procedure: the one hybrid system. DNA Cell Biol. 13:731-742.
Inouye, S., A. Nakazawa, and T. Nakazawa. 1985. Determination of the transcription initiation site and identification of the protein product of the regulatory gene xylR for xyl operons on the TOL plasmid. J. Bacteriol. 163:863-869.
Jacob, F., D. J. Polzin, C. A. Osborne, T. A. Allen, C. A. Kirk, J. D. Neaton, C. Lekcharoensuk, and L. L. Swanson. 2002. Clinical evaluation of dietary modification for treatment of spontaneous chronic renal failure in dogs. J Am. Vet. Med. Assoc. 220:1163-1170.
Jardieu, P., R. Clark, D. Mortensen, and K. Dorshkind. 1994. In vivo administration of insulin-like growth factor-I stimulates primary B lymphopoiesis and enhances lymphocyte recovery after bone marrow transplantation. J Immunol. 152:4320-4327.
Jaynes, J. B., J. E. Johnson, J. N. Buskin, C. L. Gartside, and S. D. Hauschka. 1988. The muscle creatine kinase gene is regulated by multiple upstream elements, including a muscle-specific enhancer. Mol. Cell Biol. 8:62-70.
Johnson, N. F., M. D. Hoover, D. G. Thomassen, Y. S. Cheng, A. Dalley, and A. L. Brooks. 1992. In vitro activity of silicon carbide whiskers in comparison to other industrial fibers using four cell culture systems. Am. J. Ind. Med. 21:807-823.
Kawamoto, T., K. Makino, H. Niwa, H. Sugiyama, S. Kimura, M. Amemura, A. Nakata, and T. Kakunaga. 1988. Identification of the human beta-actin enhancer and its binding factor. Mol. Cell Biol. 8:267-272.
Kawamoto, T., K. Makino, S. Orita, A. Nakata, and T. Kakunaga. 1989. DNA bending and binding factors of the human beta-actin promoter. Nucleic Acids Res. 17:523-537.
Khan, A. S., M. L. Fiorotto, L. A. Hill, P. B. Malone, K. K. Cummings, D. Parghi, R. J. Schwartz, R. G. Smith, and R. Draghia-Akli. 2002. Nonhereditary enhancement of progeny growth. Endocrinology 143:3561-3567.
Klamut, H. J., L. O. Bosnoyan-Collins, R. G. Worton, P. N. Ray, and H. L. Davis. 1996. Identification of a transcriptional enhancer within muscle intron 1 of the human dystrophin gene. Hum. Mol. Genet. 5:1599-1606.
Klamut, H. J., S. B. Gangopadhyay, R. G. Worton, and P. N. Ray. 1990. Molecular and functional analysis of the muscle-specific promoter region of the Duchenne muscular dystrophy gene. Mol. Cell Biol. 10:193-205.
Kraus, J., M. Woltje, N. Schonwetter, and V. Hollt. 1998. Alternative promoter usage and tissue specific expression of the mouse somatostatin receptor 2 gene. FEBS Lett. 428:165-170.
Kurtz, A., W. Jelkmann, and C. Bauer. 1982. A new candidate for the regulation of erythropoiesis. Insulin-like growth factor I. FEBS Lett. 149:105-108.
Kurtz, A., R. Matter, K. U. Eckardt, and J. Zapf. 1990. Erythropoiesis, serum erythropoietin, and serum IGF-I in rats during accelerated growth. Acta Endocrinol. (Copenh) 122:323-328.
Lareyre, J. J., T. Z. Thomas, W. L. Zheng, S. Kasper, D. E. Ong, M. C. Orgebin-Crist, and R J. Matusik. 1999. A 5-kilobase pair promoter fragment of the murine epididymal retinoic acid-binding protein gene drives the tissue-specific, cell-specific, and androgen-regulated expression of a foreign gene in the epididymis of transgenic mice. J. Biol. Chem. 274:8282-8290.
Larsen, P. R, J. W. Harney, and D. D. Moore. 1986. Sequences required for cell-type specific thyroid hormone regulation of rat growth hormone promoter activity. J. Biol. Chem. 261:14373-14376.
LeBrun, C. J., L. F. Diehl, K. C. Abbott, P. G. Welch, and C. M. Yuan. 2000. Life expectancy benefits of cancer screening in the end-stage renal disease population. Am. J. Kidney Dis. 35:237-243.
Lee, S. H., W. Wang, S. Yajima, P. A. Jose, and M. M. Mouradian. 1997. Tissue-specific promoter usage in the D1A dopamine receptor gene in brain and kidney. DNA Cell Biol. 16:1267-1275.
Lesbordes, J. C., T. Bordet, G. Haase, L. Castelnau-Ptakhine, S. Rouhani, H. Gilgenkrantz, and A. Kahn. 2002. In vivo electrotransfer of the cardiotrophin-1 gene into skeletal muscle slows down progression of motor neuron degeneration in pmn mice. Hum. Mol. Genet. 11:1615-1625.
Levenson, V. V., E: D. Transue, and I. B. Roninson. 1998. Internal ribosomal entry site-containing retroviral vectors with green fluorescent protein and drug resistance markers. Hum. Gene Ther. 9:1233-1236.
Levin, A. 2001. Identification of patients and risk factors in chronic kidney disease-evaluating risk factors and therapeutic strategies. Nephrol. Dial. Transplant. 16 Suppl 7:57-60.:57-60.
Li, C., S. Ke, Q. P. Wu, W. Tansey, N. Hunter, L. M. Buchmiller, L. Milas, C. Charnsangavej, and S. Wallace. 2000. Tumor irradiation enhances the tumor-specific distribution of poly(L-glutamic acid)-conjugated paclitaxel and its antitumor efficacy. Clin. Cancer Res. 6:2829-2834.
Li, X., E. M. Eastman, R. J. Schwartz, and R Draghia-Akh. 1999. Synthetic muscle promoters: activities exceeding naturally occurring regulatory sequences. Nat. Biotechnol. 17:241-245.
Lin, H., K. E. Yutzey, and S. F. Konieczny. 1991. Muscle-specific expression of the troponin I gene requires interactions between helix-loop-helix muscle regulatory factors and ubiquitous transcription factors. Mol. Cell Biol. 11:267-280.
Liu, Y., H. Li, K. Tanaka, N. Tsumaki, and Y. Yamada. 2000. Identification of an enhancer sequence within the first intron required for cartilage-specific transcription of the alpha2(XI) collagen gene. J. Biol. Chem. 275:12712-12718.
Lucas, M. L., L. Heller, D. Coppola, and R. Heller. 2002. IL-12 plasmid delivery by in vivo electroporation for the successful treatment of established subcutaneous B16.F10 melanoma. Mol. Ther. 5:668-675.
Lucas, M. L., M. J. Jaroszeski, R. Gilbert, and R. Heller. 2001. In vivo electroporation using an exponentially enhanced pulse: a new waveform. DNA Cell Biol. 20:183-188.
Macejak, D. G. and P. Sarnow. 1991. Internal initiation of translation mediated by the 5' leader of a cellular mRNA. Nature 353:90-94.
Madry, H., R. Reszka, J. Bohlender, and J. Wagner. 2001. Efficacy of cationic liposome-mediated gene transfer to mesangial cells in vitro and in vivo. J. Mol. Med. 79:184-189.
Makis, A. C., N. Chaliasos, E. C. Hatzimichael, and K. L. Bourantas. 2001. Recombinant human erythropoietin therapy in a transfusion-dependent beta-thalassemia major patient. Ann. Hematol. 80:492-495.
Mathews, K. A., D. L. Holmberg, and C. W. Miller. 2000. Kidney transplantation in dogs with naturally occurring end-stage renal disease. J Am. Anim Hosp. Assoc. 36:294-301.
Matsubara, H., Y. Gunji, T. Maeda, K. Tasaki, Y. Koide, T. Asano, T. Ochiai, S. Sakiyama, and M. Tagawa. 2001. Electroporation-mediated transfer of cytokine genes into human esophageal tumors produces anti-tumor effects in mice. Anticancer Res. 21:2501-2503.
Matsuo, A., I. Tooyama, S. Isobe, Y. Oomura, I. Akiguchi, K. Hanai, J. Kimura, and H. Kimura. 1994. Immunohistochemical localization in the rat brain of an epitope corresponding to the fibroblast growth factor receptor-1. Neuroscience 60:49-66.
McNally, M. A., J. S. Lebkowski, T. B. Okarma, and L. B. Lerch. 1988. Optimizing electroporation parameters for a variety of human hematopoietic cell lines. Biotechniques 6:882-886.
Miklavcic, D., K. Beravs, D. Semrov, M. Cemazar, F. Demsar, and G. Sersa. 1998. The importance of electric field distribution for effective in vivo electroporation of tissues. Biophys. J 74:2152-2158.
Miller, S. B. and R. Rabkin. 1997. The use of growth factors to increase glomerular filtration rate in chronic renal failure patients. Curr. Opin. Nephrol. Hypertens. 6:401-404.
Mirza, A. M., S. Ezzat, and A. A. Axelrad. 1997. Insulin-like growth factor binding protein-1 is elevated in patients with polycythemia vera and stimulates erythroid burst formation in vitro. Blood 89:1862-1869.
Mumper, R. J., J. Wang, S. L. Klakamp, H. Nitta, K. Anwer, F. Tagliaferri, and A. P. Rolland. 1998. Protective interactive noncondensing (PINC) polymers for enhanced plasmid distribution and expression in rat skeletal muscle. J. Control Release 52:191-203.
Muramatsu, T., S. Arakawa, K. Fukazawa, Y. Fujiwara, T. Yoshida, R. Sasaki, S. Masuda, and H. M. Park. 2001. In vivo gene electroporation in skeletal muscle with special reference to the duration of gene expression. Int. J Mol. Med. 7:37-42.
Murray, R. D. and S. M. Shalet. 2000. Growth hormone: current and future therapeutic applications. Expert. Opin. Pharmacother. 1:975-990.
Nabel, E. G., G. Plautz, F. M. Boyce, J. C. Stanley, and G. J. Nabel. 1989. Recombinant gene expression in vivo within endothelial cells of the arterial wall. Science 244:1342-1344.
Nairn, R. S., G. M. Adair, T. Porter, S. L. Pennington, D. G. Smith, J. H. Wilson, and M. M. Seidman. 1993. Targeting vector configuration and method of gene transfer influence targeted correction of the APRT gene in Chinese hamster ovary cells. Somat. Cell Mol. Genet. 19:363-375.
Nampoory, M. R., K. V. Johny, J. N. Costandi, R. K. Gupta, M. P. Nair, M. Samhan, I. A. al Muzairai, and M. al Mousawi. 2002. Inferior long-term outcome of renal transplantation in patients with diabetes mellitus. Med. Princ. Pract. 11:29-34.
Narum, D. L., S. Kumar, W. O. Rogers, S. R. Fuhrmann, H. Liang, M. Oakley, A. Taye, B. K. Sim, and S. L. Hoffman. 2001. Codon optimization of gene fragments encoding Plasmodium falciparum merzoite proteins enhances DNA vaccine protein expression and immunogenicity in mice. Infect. Immun. 69:7250-7253.
Nelson, K. A. 2001. Modern management of the cancer anorexia-cachexia syndrome. Curr. Pain Headache Rep. 5:250-256.
Neumann, E., M. Schaefer-Ridder, Y. Wang, and P. H. Hofschneider. 1982. Gene transfer into mouse lyoma cells by electroporation in high electric fields. EMBO J. 1:841-845.
Nomoto, S., Y. Tatematsu, T. Takahashi, and H. Osada. 1999. Cloning and characterization of the alternative promoter regions of the human LIMK2 gene responsible for alternative transcripts with tissue-specific expression. Gene 236:259-271.
O'Sullivan, A. J., J. A. Lawson, M. Chan, and J. J. Kelly. 2002. Body composition and energy metabolism in chronic renal insufficiency. Am. J Kidney Dis. 39:369-375.
Ohlsson, H., S. Thor, and T. Edlund. 1991. Novel insulin promoter- and enhancer-binding proteins that discriminate between pancreatic alpha- and beta-cells. Mol. Endocrinol. 5:897-904.
Omirulleh, S., M. Abraham, M. Golovkin, I. Stefanov, M. K. Karabaev, L. Mustardy, S. Morocz, and D. Dudits. 1993. Activity of a chimeric promoter with the doubled CaMV 35S enhancer element in protoplast-derived cells and transgenic plants in maize. Plant Mol. Biol. 21:415-428.
Otani, Y., Y. Tabata, and Y. Ikada. 1996. Rapidly curable biological glue composed of gelatin and poly(L-glutamic acid). Biomaterials 17:1387-1391.
Otani, Y., Y. Tabata, and Y. Ikada. 1998. Hemostatic capability of rapidly curable glues from gelatin, poly(L-glutamic acid), and carbodiimide. Biomaterials 19:2091-2098.
Papassotiriou, I., E. Voskaridou, A. Stamoulakatou, and D. Loukopoulos. 2000. Increased erythropoietin level induced by hydroxyurea treatment of sickle cell patients. Hematol. J. 1:295-300.
Pasqualini, T., J. Ferraris, P. Fainstein-Day, A. A. Eymann, C. S. Moyano, S. Ruiz, J. Ramirez, and R. Gutman. 1996. Growth acceleration in children with chronic renal failure treated with growth-hormone-releasing hormone (GHRH). Medicina (B Aires) 56:241-246.
Payen, E., M. Bettan, P. Rouyer-Fessard, Y. Beuzard, and D. Scherman. 2001. Improvement of mouse beta-thalassemia by electrotransfer of erythropoietin cDNA. Exp. Hematol. 29:295-300.
Pech, M., C. D. Rao, K. C. Robbins, and S. A. Aaronson. 1989. Functional identification of regulatory elements within the promoter region of platelet-derived growth factor 2. Mol. Cell Biol. 9:396-405.
Pelletier, J. and N. Sonenberg. 1988. Internal initiation of translation of eukaryotic mRNA directed by a sequence derived from poliovirus RNA. Nature 334:320-325.
Pinkert, C. A., D. M. Ornitz, R. L. Brinster, and R. D. Palmiter. 1987. An albumin enhancer located 10 kb upstream functions along with its promoter to direct efficient, liver-specific expression in transgenic mice. Genes Dev. 1:268-276.
Polzin, D. J., C. A. Osborne, S. Ross, and F. Jacob. 2000. Dietary management of feline chronic renal failure: where are we now? In what direction are we headed? J Feline. Med. Surg. 2:75-82.
Potrykus, I., J. Paszkowski, M. W. Saul, J. Petruska, and R. D. Shillito. 1985. Molecular and general genetics of a hybrid foreign gene introduced into tobacco by direct gene transfer. Mol. Gen. Genet. 199:169-177.
Potter, H., L. Weir, and P. Leder. 1984. Enhancer-dependent expression of human kappa immunoglobulin genes introduced into mouse pre-B lymphocytes by electroporation. Proc. Natl. Acad. Sci. U. S. A 81:7161-7165.
Prahalada, S., L. G. Stabinski, H. Y. Chen, R. E. Morrissey, G. De Burlet, D. Holder, D. H. Patrick, C. P. Peter, and M. J. van Zwieten. 1998. Pharmacological and toxicological effects of chronic porcine growth hormone administration in dogs [see comments]. Toxicol. Pathol. 26:185-200.
Prentice, H., R. A. Kloner, T. Prigozy, T. Christensen, L. Newman, Y. Li, and L. Kedes. 1994. Tissue restricted gene expression assayed by direct DNA injection into cardiac and skeletal muscle. Journal of Molecular & Cellular Cardiology 26:1393-1401.
Raghavachari, N. and W. E. Fahl. 2002. Targeted gene delivery to skin cells in vivo: a comparative study of liposomes and polymers as delivery vehicles. J. Pharm. Sci. 91:615-622.
Rao, V. K. 2002. Kidney transplantation in older patients: benefits and risks. Drugs Aging 19:79-84.
Rippe, R. A., D. A. Brenner, and H. L. Leffert. 1990. DNA-mediated gene transfer into adult rat hepatocytes in primary culture. Mol. Cell Biol. 10:689-695.
Robins, K., S. McCabe, T. Scheiner, J. Strasser, R. Clark, and P. Jardieu. 1994. Immunological effects of insulin-like growth factor-I--enhancement of immunoglobulin synthesis. Clin. Exp. Immunol. 95:337-342.
Sakhuja, V., V. Jha, S. Varma, K. Joshi, K. L. Gupta, K. Sud, and H. S. Kohli. 2000. Renal involvement in multiple myeloma: a 10-year study. Ren Fail. 22:465-477.
Sambrook, J., E. F. Fritsch, and T. Maniatis. 1989. Molecular Cloning - A Laboratory Manual. Cold Spring Harbor Laboratory Press, Cold Spring Harbor, NY.
Satozawa, N., K. Takezawa, T. Miwa, S. Takahashi, M. Hayakawa, and H. Ooka. 2000. Differences in the effects of 20 K- and 22 K-hGH on water retention in rats [In Process Citation]. Growth Horm. IGF. Res. 10:187-192.
Skroch, P., C. Buchman, and M. Karin. 1993. Regulation of human and yeast metallothionein gene transcription by heavy metal ions. Prog. Clin. Biol. Res. 380:113-28.:113-128.
Smith, L. C. and J. L. Nordstrom. 2000. Advances in plasmid gene delivery and expression in skeletal muscle. Curr. Opin. Mol. Ther. 2:150-154.
Sohmiya, M. and Y. Kato. 2000. Effect of long-term treatment with recombinant human growth hormone on erythropoietin secretion in an anemic patient with panhypopituitarism. J Endocrinol. Invest 23:31-36.
Somiari, S., J. Glasspool-Malone, J. J. Drabick, R. A. Gilbert, R. Heller, M. J. Jaroszeski, and R. W. Malone. 2000. Theory and in vivo application of electroporative gene delivery. Mol. Ther. 2:178-187.
Song, S., J. Embury, P. J. Laipis, K. I. Berns, J. M. Crawford, and T. R. Flotte. 2001. Stable therapeutic serum levels of human alpha-1 antitrypsin (AAT) after portal vein injection of recombinant adeno-associated virus (rAAV) vectors. Gene Ther. 8:1299-1306.
Soubrier, F., B. Cameron, B. Manse, S. Somarriba, C. Dubertret, G. Jaslin, G. Jung, C. L. Caer, D. Dang, J. M. Mouvault, D. Scherman, J. F. Mayaux, and J. Crouzet. 1999. pCOR: a new design of plasmid vectors for nonviral gene therapy. Gene Ther. 6:1482-1488.
Sowade, B., O. Sowade, J. Mocks, W. Franke, and H. Wamke. 1998. The safety of treatment with recombinant human erythropoietin in clinical use: a review of controlled studies. Int. J. Mol. Med. 1:303-314.
Sun, W., L. Wang, Z. Zhang, M. Chen, and X. Wang. 2003. Intramuscular transfer of naked calcitonin gene-related peptide gene prevents autoimmune diabetes induced by multiple low-dose streptozotocin in C57BL mice. Eur. J Immunol. 33:233-242.
Tanaka, T., N. Ichimaru, S. Takahara, K. Yazawa, M. Hatori, K. Suzuki, Y. Isaka, T. Moriyama, E. Imai, H. Azuma, T. Nakamura, A. Okuyama, and H. Yamanaka. 2002. In vivo gene transfer of hepatocyte growth factor to skeletal muscle prevents changes in rat kidneys after 5/6 nephrectomy. Am. J. Transplant. 2:828-836.
Terada, Y., H. Tanaka, T. Okado, S. Inoshita, M. Kuwahara, T. Akiba, S. Sasaki, and F. Marumo. 2001. Efficient and ligand-dependent regulated erythropoietin production by naked dna injection and in vivo electroporation. Am. J Kidney Dis. 38:550-S53.
Thorner, M. O., L. A. Frohman, D. A. Leong, J. Thominet, T. Downs, P. Hellmann, J. Chitwood, J. M. Vaughan, and W. Vale. 1984. Extrahypothalamic growth-hormone-releasing factor (GRF) secretion is a rare cause of acromegaly: plasma GRF levels in 177 acromegalic patients. Journal of Clinical Endocrinology & Metabolism 59:846-849.
Thorner, M. O., M. L. Hartman, M. L. Vance, S. S. Pezzoli, and E. J. Ampleford. 1995. Neuroendocrine regulation of growth hormone secretion. [Review]. Neuroscience & Biobehavioral Reviews 19:465-468.
Thorner, M. O., M. L. Vance, W. S. Evans, A. D. Rogol, J. Rivier, W. Vale, Blizzard, and RM. 1986. Clinical Studies With GHRH In Man. Hormone Research 24:91-98.
Toneguzzo, F., A. Keating, S. Glynn, and K. McDonald. 1988. Electric field-mediated gene transfer: characterization of DNA transfer and patterns of integration in lymphoid cells. Nucleic Acids Res. 16:5515-5532.
Tripathy, S. K., E. C. Svensson, H. B. Black, E. Goldwasser, M. Margalith, Hobart, PM, and J. M. Leiden. 1996. Long-term expression of erythropoietin in the systemic circulation of mice after intramuscular injection of a plasmid DNA vector. Proc. Natl. Acad. Sci. USA 93:10876-10880.
Tronche, F., A. Rollier, I. Bach, M. C. Weiss, and M. Yaniv. 1989. The rat albumin promoter: cooperation with upstream elements is required when binding of APF/HNF1 to the proximal element is partially impaired by mutation or bacterial methylation. Mol. Cell Biol. 9:4759-4766.
Tronche, F., A. Rollier, P. Herbomel, I. Bach, S. Cereghini, M. Weiss, and M. Yaniv. 1990. Anatomy of the rat albumin promoter. Mol. Biol. Med. 7:173-185.
Trudel, M. and F. Costantini. 1987. A 3' enhancer contributes to the stage-specific expression of the human beta-globin gene. Genes Dev. 1:954-961.
Tsumaki, N., T. Kimura, K. Tanaka, J. H. Kimura, T. Ochi, and Y. Yamada. 1998. Modular arrangement of cartilage- and neural tissue-specific cis-elements in the mouse alpha2(XI) collagen promoter. J. Biol. Chem. 273:22861-22864..
Tsunekawa, B., M. Wada, M. Ikeda, H. Uchida, N. Naito, and M. Honjo. 1999. The 20-kilodalton (kDa) human growth hormone (hGH) differs from the 22-kDa hGH in the effect on the human prolactin receptor. Endocrinology 140:3909-3918.
Tsurumi, Y., S. Takeshita, D. Chen, M. Kearney, S. T. Rossow, J. Passeri, J. R. Horowitz, J. F. Symes, and J. M. Isner. 1996. Direct intramuscular gene transfer of naked DNA encoding vascular endothelial growth factor augments collateral development and tissue perfusion. Circulation 94:3281-3290.
Tur-Kaspa, R., L. Teicher, B. J. Levine, A. I. Skoultchi, and D. A. Shafritz. 1986. Use of electroporation to introduce biologically active foreign genes into primary rat hepatocytes. Mol. Cell Biol. 6:716-718.
Urena, P., A. Bonnardeaux, K. U. Eckardt, A. Kurtz, and T. B. Drueke. 1992. Insulin-like growth factor I: a modulator of erythropoiesis in uraemic patients? Nephrol. Dial. Transplant. 7:40-44.
Valerio, G., S. Di Maio, M. Salerno, A. Argenziano, R Badolato, and A. Tenore. 1997. Assessment of red blood cell indices in growth-honnone-treated children. Horm. Res. 47:62-66.
Vance, M. L. 1990. Growth-hormone-releasing hormone. [Review] [52 refs]. Clinical Chemistry 36:415-420.
Vance, M. L., D. L. Kaiser, W. S. Evans, R. Furlanetto, W. Vale, J. Rivier, and M. O. Thorner. 1985. Pulsatile growth hormone secretion in normal man during a continuous 24-hour infusion of human growth hormone releasing factor (1-40). Evidence for intermittent somatostatin secretion. J. Clin. Invest. 75:1584-1590.
Vansteenkiste, J., R. Pirker, B. Massuti, F. Barata, A. Font, M. Fiegl, S. Siena, J. Gateley, D. Tomita, A. B. Colowick, and J. Musil. 2002. Double-blind, placebo-controlled, randomized phase III trial of darbepoetin alfa in lung cancer patients receiving chemotherapy. J. Natl. Cancer Inst. 94:1211-1220.
Verhelst, J., R. Abs, M. Vandeweghe, J. Mockel, J. J. Legros, G. Copinschi, C. Mahler, B. Velkeniers, L. Vanhaelst, A. Van Aelst, D. De Rijdt, A. Stevenaert, and A. Beckers. 1997. Two years of replacement therapy in adults with growth hormone deficiency. Clin. Endocrinol. (Oxf) 47:485-494.
Vijayan, A., S. C. Franklin, T. Behrend, M. R. Hammerman, and S. B. Miller. 1999. Insulin-like growth factor I improves renal function in patients with end-stage chronic renal failure. Am. J Physiol 276:R929-R934.
Vilquin, J. T., P. F. Kennel, M. Paturneau-Jouas, P. Chapdelaine, N. Boissel, P. Delaere, J. P. Tremblay, D. Scherman, M. Y. Fiszman, and K. Schwartz. 2001. Electrotransfer of naked DNA in the skeletal muscles of animal models of muscular dystrophies. Gene Ther. 8:1097-1107.
Vittone, J., M. R. Blackman, J. Busby-Whitehead, C. Tsiao, K. J. Stewart, J. Tobin, T. Stevens, M. F. Bellantoni, M. A. Rogers, G. Baumann, J. Roth, S. M. Harman, and R. G. S. Spencer. 1997. Effects of single nightly injections of growth hormone-releasing hormone (GHRH 1-29) in healthy elderly men. Metabolism: Clinical and Experimental 46:89-96.
Wada, M., H. Uchida, M. Ikeda, B. Tsunekawa, N. Naito, S. Banba, E. Tanaka, Y. Hashimoto, and M. Honj o. 1998. The 20-kilodalton (kDa) human growth hormone (hGH) differs from the 22-kDa hGH in the complex formation with cell surface hGH receptor and hGH-binding protein circulating in human plasma. Mol. Endocrinol. 12:146-156.
Weinroth, S. E., D. M. Parenti, and G. L. Simon. 1995. Wasting syndrome in AIDS: pathophysiologic mechanisms and therapeutic approaches. Infect. Agents Dis. 4:76-94.
Welle, S. 1998. Growth hormone and insulin-like growth factor-I as anabolic agents. Curr. Opin. Clin. Nutr. Metab Care 1:257-262.
Wells, K. E., J. Maule, R. Kingston, K. Foster, J. McMahon, E. Damien, A. Poole, and D. J. Wells. 1997. Immune responses, not promoter inactivation, are responsible for decreased long-term expression following plasmid gene transfer into skeletal muscle. FEBS Lett. 407:164-168.
Wiethoff, C. M., J. G. Smith, G. S. Koe, and C. R. Middaugh. 2001. The potential role of proteoglycans in cationic lipid-mediated gene delivery. Studies of the interaction of cationic lipid-DNA complexes with model glycosaminoglycans. J. Biol. Chem. 276:32806-32813.
Wilson, J. M., L. K. Birinyi, R. N. Salomon, P. Libby, A. D. Callow, and R. C. Mulligan. 1989. Implantation of vascular grafts lined with genetically modified endothelial cells. Science 244:1344-1346.
Wolff, J. A., R. W. Malone, P. Williams, W. Chong, G. Acsadi, A. Jani, Felgner, and PL. 1990. Direct gene transfer into mouse muscle in vivo. Science 247:1465-1468.
Wu, G. Y. and C. H. Wu. 1988a. Evidence for targeted gene delivery to Hep G2 hepatoma cells in vitro. Biochemistry 27:887-892.
Wu, G. Y. and C. H. Wu. 1988b. Receptor-mediated gene delivery and expression in vivo. J. Biol. Chem. 263:14621-14624.
Wu, H. K., J. A. Squire, Q. Song, and R. Weksberg. 1997. Promoter-dependent tissue-specific expressive nature of imprinting gene, insulin-like growth factor II, in human tissues. Biochem. Biophys. Res. Commun. 233:221-226.
Wuhl, E. and F. Schaefer. 2002. Effects of growth hormone in patients with chronic renal failure: experience in children and adults. Horm. Res. 58 Suppl 3:35-8.:35-38.
Xie, T. D. and T. Y. Tsong. 1993. Study of mechanisms of electric field-induced DNA transfection. V. Effects of DNA topology on surface binding, cell uptake, expression, and integration into host chromosomes of DNA in the mammalian cell. Biophys. J. 65:1684-1689.
Yasui, A., K. Oda, H. Usunomiya, K. Kakudo, T. Suzuki, T. Yoshida, H. M. Park, K. Fukazawa, and T. Muramatsu. 2001. Elevated gastrin secretion by in vivo gene electroporation in skeletal muscle. Int. J Mol. Med. 8:489-494.
Yin, D. and J. G. Tang. 2001. Gene therapy for streptozotocin-induced diabetic mice by electroporational transfer of naked human insulin precursor DNA into skeletal muscle in vivo. FEBS Lett. 495:16-20.
Yorifuji, T. and H. Mikawa. 1990. Co-transfer of restriction endonucleases and plasmid DNA into mammalian cells by electroporation: effects on stable transformation. Mutat. Res. 243:121-126.
Yutzey, K. E. and S. F. Konieczny. 1992. Different E-box regulatory sequences are functionally distinct when placed within the context of the troponin I enhancer. Nucleic Acids Res. 20:5105-5113.
Zhao-Emonet, J. C., O. Boyer, J. L. Cohen, and D. Klatzmann. 1998. Deletional and mutational analyses of the human CD4 gene promoter: characterization of a minimal tissue-specific promoter. Biochim. Biophys. Acta 1442:109-119.
Ziegler, T. R., J. M. Lazarus, L. S. Young, R. Hakim, and D. W. Wilmore. 1991. Effects of recombinant human growth hormone in adults receiving maintenance hemodialysis. J Am. Soc. Nephrol. 2:1130-1135.

## Claims

1. A composition comprising an isolated nucleic acid expression construct that encodes a growth-hormone-releasing-hormone ("GHRH"), for use in extending life expectancy of a chronically ill subject with chronic renal failure.

2. The composition of claim 1, further comprising a transfection-facilitating polypeptide or a charged polypeptide.

3. The composition of claim 2, wherein the transfection-facilitating polypeptide or charged polypeptide comprises poly-L-glutamate.

4. The composition of claim 1, wherein the nucleic acid expression construct is formulated in a 0.01% poly-L-glutamate solution.

5. The composition of claim 1, wherein the isolated nucleic acid expression construct is at least 90% identical to a sequence selected from a group consisting of SeqID No.: 11, SeqID No.:12, SeqID No.: 13, SeqID No.: 14, SeqID No.: 17, SeqID No.: 18, SeqID No.: 19, SeqID No.: 20, and SeqID No.: 21.

6. The composition of claim 1, wherein the expressed amount of the encoded GHRH facilitates growth hormone ("GH") secretion in the subject.

7. The composition of claim 1, wherein the expressed amount of the encoded GHRH facilitates insulin-like growth factor I ("IGF-I") secretion in the subject.

8. The composition of claim 1, wherein the encoded GHRH comprises an amino acid sequence at least 90% identical to (SeqID No.: 6):
-X₁-X₂-DAIFTNSYRKVL-X₃-QLSARKILQDI-X₄-X₅-RQQGE-X₆-NQE-X₇-GA-OH
wherein the formula has the following characteristics :
X₁ is a D- or L-isomer of the amino acid tyrosine ("Y"), or histidine ("H");
X₂ is a D- or L-isomer of the amino acid alanine ("A"), valine ("V"), or isoleucine ("I"),
X₃ is a D- or L-isomer of the amino acid alanine ("A") or glycine ("G");
X₄ is a D- or L-isomer of the amino acid methionine ("M") or leucine ("L");
X₅ is a D- or L-isomer of the amino acid serine ("S") or asparagine ("N");
X₆ is a D- or L-isomer of the amino acid arginine ("R") or serine ("S");
X₇ is a D- or L-isomer of the amino acid glutamine ("Q") or arginine ("R"); or a combination thereof.

9. The use of an isolated nucleic acid expression construct that encodes a growth-hormone-releasing-hormone ("GHRH") for the manufacture of a medicament for extending life expectancy of a chronically ill subject with chronic renal failure.

10. The use of claim 9, wherein said treatment comprises electroporating a tissue of the subject as a method for delivering the isolated nucleic acid expression construct into the cells of the subject.

11. The use of claim 10, wherein electroporating the tissue is a method comprising:
a. penetrating a tissue in the subject with a plurality of needle electrodes, wherein the plurality of needle electrodes are arranged in a spaced relationship and the tissue of the subject comprises muscle cells;
b. introducing the isolated nucleic acid expression construct into the tissue between the plurality of needle electrodes in an amount in a range of about 0.1-5 mg; and
c. applying at least one electrical pulse to the plurality of needle electrodes, wherein the electrical pulse allows the isolated nucleic acid expression construct to traverse a muscle cell membrane.

12. The use of claim 11, wherein the electrical pulse to the plurality of needle electrodes comprises an Amp-intensity electric field having a range of about 0.4-1 Amp, with a range of about 3-5 pulses, at about 52 milliseconds/pulse, and about a 1 second interval between electrical pulses.

13. The use of claim 11, further comprising inserting the needles into a muscle tissue through an area of intact skin, wherein the needles are about 21 gauge and about 1 inch (about 2.54 cm) in length.

14. The use of claim 9, wherein the isolated nucleic acid expression construct further comprises a transfection-facilitating polypeptide or a charged polypeptide.

15. The use of claim 14, wherein the transfection-facilitating polypeptide or charged polypeptide comprises poly-L-glutamate.

16. The use of claim 9, wherein the nucleic acid expression construct is formulated in a 0.01% poly-L-glutamate solution.

17. The use of claim 9, wherein the isolated nucleic acid expression construct that is at least 90% identical to a sequence selected from a group consisting of : SeqID No.: 11, SeqID No.: 12, SeqID No.: 13, SeqID No.: 14, SeqID No.: 17, SeqID No.: 18, SeqID No.: 19, SeqID No.: 20, and SeqID No.: 21.

18. The use of claim 9, wherein the expressed amount of the encoded GHRH facilitates growth hormone ("GH") secretion in the subject.

19. The use of claim 9, wherein the expressed amount of the encoded GHRH facilitates insulin-like growth factor I ("IGF-I") secretion in the subject.

20. The use of claim 9, wherein the encoded GHRH comprises an amino acid sequence at least 90% identical to (SEQID No.: 6):
-X₁-X₂-DAIFTNSYRKVL-X₃-QLSARKLLQDI-X₄-X₅-RQQGE-X₆-NQE-X₇-GA-OH
wherein the formula has the following characteristics :
X₁ is a D- or L-isomer of the amino acid tyrosine ("Y"), or histidine ("H");
X₂ is a D- or L-isomer of the amino acid alanine ("A"), valine ("V"), or isoleucine ("I");
X₃ is a D- or L-isomer of the amino acid alanine ("A") or glycine ("G");
X₄ is a D- or L-isomer of the amino acid methionine ("M") or leucine ("L");
X₅ is a D- or L-isomer of the amino acid serine ("S") or asparagine ("N");
X₆ is a D- or L-isomer of the amino acid arginine ("R") or serine ("S");
X₇ is a D- or L-isomer of the amino acid glutamine ("Q") or arginine ("R"); or a combination thereof

21. A recombinant growth-hormone-releasing-hormone ("GHRH") for use in extending life expectancy of a chronically ill subject with chronic renal failure.

22. A recombinant growth-hormone-releasing-hormone ("GHRH") as claimed in claim 21, wherein the recombinant GHRH comprises an amino acid sequence at least 90% identical to:
-X₁-X₂-DAIFTNSYRKVL-X₃-QLSARKLLQDI-X₄-X₅-RQQGE-X₆-NQE-X₇-GA-OH
wherein the formula has the following characteristics :
X₁ is a D- or L-isomer of the amino acid tyrosine ("Y"), or histidine ("H");
X₂ is a D- or L-isomer of the amino acid alanine ("A"), valine ("V"), or isoleucine ("I");
X₃ is a D- or L-isomer of the amino acid alanine ("A") or glycine ("G");
X₄ is a D- or L-isomer of the amino acid methionine ("M") or leucine ("L");
X₅ is a D- or L-isomer of the amino acid serine ("S") or asparagine ("N");
X₆ is a D- or L-isomer of the amino acid arginine ("R") or serine ("S");
X₇ is a D- or L-isomer of the amino acid glutamine ("Q") or arginine ("R"); or a combination thereof.

23. A recombinant growth-hormone-releasing-hormone ("GHRH") as claimed in claim 21, wherein the recombinant GHRH facilitates growth hormone ("GH") secretion in the subject.

24. A recombinant growth-hormone-releasing-hormone ("GHRH") as claimed in claim 21, .wherein the recombinant GHRH facilitates insulin-like growth factor I ("IGF-I") secretion in the subject.

25. The use of claim 9, wherein a symptom of chronic renal failure comprises serum creatinine values being elevated above a baseline value.

26. A recombinant growth-hormone-releasing-hormone ("GHRH") as claimed in claim 21, wherein a symptom of chronic renal failure comprises serum creatinine values being elevated above a baseline value.

## Patentansprüche

1. Zusammensetzung, umfassend ein isoliertes Nukleinsäure-Expressionskonstrukt, das ein Somatotropin-Releasing-Hormon (*growth-hormone-releasing-hormone; "*GHRH") kodiert, zur Verwendung für das Verlängern der Lebenserwartung eines chronisch kranken Subjekts mit chronischer Niereninsuffizienz.

2. Zusammensetzung nach Anspruch 1, weiter umfassend ein die Transfektion förderndes Polypeptid oder ein geladenes Polypeptid.

3. Zusammensetzung nach Anspruch 2, wobei das die Transfektion fördernde Polypeptid oder geladene Polypeptid Poly-L-glmutamat umfaßt.

4. Zusammensetzung nach Anspruch 1 , wobei das Nukleinsäure-Expressionskonstrukt in einer 0,01 % Poly-L-glutamat-Lösung formuliert ist.

5. Zusammensetzung nach Anspruch 1, wobei das isolierte Nukleinsäure-Expressionskonstrukt mindestens 90% identisch zu einer Sequenz ist, ausgewählt aus der Gruppe, bestehend aus: SeqID Nr.:1 1, SeqID Nr.: 12, SeqID Nr.: 13, SeqID Nr.: 14, SeqID Nr.: 17, SeqID Nr. 18, SeqID Nr.: 19, SeqID Nr.: 20 und SeqID Nr.: 21.

6. Zusammensetzung nach Anspruch 1, wobei die exprimierte Menge des kodierten GHRH die Sekretion von Somatotropin (*growth hormone;* "GH") in dem Subjekt fördert.

7. Zusammensetzung nach Anspruch 1, wobei die exprimierte Menge des kodierten GHRH die Sekretion von Insulin-ähnlichem Wachstumsfaktor I *(insulin-like growth factor I;* "IGF-1") in dem Subjekt fördert.

8. Zusammensetzung nach Anspruch 1, wobei das kodierte GHRH eine Aminosäuresequenz umfaßt, die mindestens 90% identisch zu (SeqID Nr.: 6) ist:
-X₁-X₂-DAIFTNSYRKVL-X₃-QLSARKLLQDI-X₄-X₅-RQQGE-X₆-NQE-X₇-GA-OH
wobei die Formel die folgenden Eigenschaften aufweist:
X₁ ist ein D- oder L-Isomer der Aminosäure Tyrosin ("Y") oder Histidin ("H"),
X₂ ist ein D- oder L-Isomer der Aminosäure Alanin ("A"), Valin ("V") oder Isoleucin ("I"),
X₃ ist ein D- oder L-Isomer der Aminosäure Alanin ("A") oder Glycin ("G"),
X₄ ist ein D- oder L-Isomer der Aminosäure Methionin ("M") oder Leucin ("L"),
X₅ ist ein D- oder L-Isomer der Aminosäure Serin ("S") oder Asparagin ("N"),
X₆ ist ein D- oder L-Isomer der Aminosäure Arginin ("R") oder Serin ("S"),
X₇ ist ein D- oder L-Isomer der Aminosäure Glutamin ("Q") oder Arginin ("R"), oder eine Kombination davon.

9. Verwendung eines isolierten Nukleinsäure-Expressionskonstrukts, das ein Somatotropin-Releasing-Hormon (*growth-hormone-releasing-hormone;* "GHRH") kodiert, für die Herstellung eines Medikaments zur Verlängerung der Lebenserwartung eines chronisch kranken Subjekts mit chronischer Niereninsuffizienz.

10. Verwendung nach Anspruch 9, wobei die Behandlung das Elektroporieren eines Gewebes des Subjekts als ein Verfahren zur Verabreichung des isolierten Nukleinsäure-Expressionskonstrukts in die Zellen des Subjekts umfaßt.

11. Verwendung nach Anspruch 10, wobei das Elektroporieren des Gewebes ein Verfahren ist, umfassend:
a. Durchstechen eines Gewebes in dem Subjekt mit einer Vielzahl von Nadelelektroden, wobei die Vielzahl von Nadelelektroden in Abständen angeordnet sind und das Gewebe des Subjekts Muskelzellen umfaßt,
b. Einbringen des isolierten Nukleinssure-Expressionskonstrukts in das Gewebe zwischen der Vielzahl von Nadelelektroden in einer Menge im Bereich von etwa 0,1 bis 5 mg, und
c. Anwenden mindestens eines elektrischen Pulses auf die Vielzahl von Nadelelektroden, wobei der elektrische Puls dem isolierten Nukleinsäure-Expressionskonsfirukt ermöglicht, eine Muskelzellmembran zu durchqueren.

12. Verwendung nach Anspruch 11, wobei der elektrische Puls an die Vielzahl von Nadelelektroden ein elektrisches Amp-Intensitätsfeld, das einen Bereich von etwa 0,4 bis 1 Amp aufweist, umfaßt, mit einem Bereich von etwa 3 bis 5 Pulsen bei etwa 52 Millisekunden pro Puls und etwa einem Ein-Sekunden-Intervall zwischen elektrischen Pulsen.

13. Verwendung nach Anspruch 11, weiter umfassend das Einführen der Nadeln in ein Muskelgewebe durch einen Bereich intakter Haut, wobei die Nadeln etwa Nadeln mit einem Gauge-Wert von 21 und etwa 1 Inch (etwa 2,54 cm) lang sind.

14. Verwendung nach Anspruch 9, wobei das isolierte Nukleinsäure-Expressionskonstrukt weiter ein die Transfektion förderndes Polypeptid oder ein geladenes Polypeptid umfaßt.

15. Verwendung nach Anspruch 14, wobei das die Transfektion fördernde Polypeptid oder geladene Polypeptid Poly-L-glutamat umfaßt.

16. Verwendung nach Anspruch 9, wobei das Nukleinsäure-Expressionskonstrukt in einer 0,01% Poly-L-glutamat-Lösung formuliert ist.

17. Verwendung nach Anspruch 9, wobei das isolierte Nukleinsäure-Expressionskonstrukt mindestens 90% identisch zu einer Sequenz ist, ausgewählt aus einer Gruppe, bestehend aus: SeqID Nr.: 11, SeqID Nr.: 12, SeqID Nr.: 13, SeqID Nr.: 14, SeqID Nr.: 17, SeqID Nr.: 18, SeqID Nr.: 19, SeqID Nr.: 20 und SeqlD Nr.: 21.

18. Verwendung nach Anspruch 9, wobei die exprimierte Menge des kodierten GHRH die Sekretion von Somatotropin *(growth hormone;* "GH") in dem Subjekt fördert.

19. Verwendung nach Anspruch 9, wobei die exprimierte Menge des kodierten GHRH die Sekretion von Insulin-ähnlichem Wachstumsfaktor I (*insulin-like growth factor I;* "IGF-I") in dem Subjekt fördert.

20. Verwendung nach Anspruch 9, wobei das kodierte GHRH eine Aminosäuresequenz umfaßt, die mindestens 90% identisch zu (SEQID Nr.: 6) ist:
-X₁-X₂-DAIFTNSYRKVL-X₃-QLSARKLLQDI-X₄-X₅-RQQGE-X₆-NQE-X₇-GA-OH
wobei die Formel die folgenden Eigenschaften aufweist:
X₁ ist ein D- oder L-Isomer der Aminosäure Tyrosin ("Y") oder Histidin ("H"),
X₂ ist ein D- oder L-Isomer der Aminosäure Alanin ("A"), Valin ("V") oder Isoleucin ("I"),
X₃ ist ein D- oder L-Isomer der Aminosäure Alanin ("A") oder Glycin ("G"),
X₄ ist ein D- oder L-Isomer der Aminosäure Methionin ("M") oder Leucin ("L"),
X₅ ist ein D- oder L-Isomer der Aminosäure Serin ("S") oder Asparagin ("N"),
X₆ ist ein D- oder L-Isomer der Aminosäure Arginin ("R") oder Serin ("S"),
X₇ ist ein D- oder L-Isomer der Aminosäure Glutamin ("Q") oder Arginin ("R"), oder eine Kombination davon.

21. Rekombinantes Somatotropin-Releasing-Hormon (*growth-hormone-releasing-hormone;* "GHRH") zur Verwendung für das Verlängern der Lebenserwartung eines chronisch kranken Subjekts mit chronischer Niereninsuffizienz.

22. Rekombinantes Somatotropin-Releasing-Hormon (*growth-hormone-releasing-hormone;* "GHRH") wie in Anspruch 21 beansprucht, wobei das rekombinante GHRH eine Aminosäuresequenz umfaßt, die mindestens 90% identisch ist zu:
-X₁-X₂-DAIFTNSYRKVL-X₃-QLSARKLLQDI-X₄-X₅-RQQGE-X₆-NQE-X₇-GA-OH
wobei die Formel die folgenden Eigenschaften aufweist:
X₁ ist ein D- oder L-Isomer der Aminosäure Tyrosin ("Y") oder Histidin ("H"),
X₂ ist ein D- oder L-Isomer der Aminosäure Alanin ("A"), Valin ("V") oder Isoleucin ("I"),
X₃ ist ein D- oder L-Isomer der Aminosäure Alanin ("A") oder Glycin ("G"),
X₄ ist ein D- oder L-isomer der Aminosäure Methionin ("M") oder Leucin ("L"),
X₅ ist ein D- oder L-Isomer der Aminosäure Serin ("S") oder Asparagin ("N"),
X₆ ist ein D- oder L-Isomer der Aminosäure Arginin ("R") oder Serin ("S"),
X₇ ist ein D- oder L-Isomer der Aminosäure Glutamin ("Q") oder Arginin ("R"), oder eine Kombination davon.

23. Rekombinantes Somatotropin-Releasing-Hormon (*growth-hormone-releasing-hormone;* "GHRH") wie in Anspruch 21 beansprucht, wobei das rekombinante GHRH die Sekretion von Somatotropin (*growth hormone;* "GH") in dem Subjekt fördert.

24. Rekombinantes Somatotropin-Releasing-Hormon (*growth-hormone-releasing-hormone;* "GHRH") wie in Anspruch 21 beansprucht, wobei das rekombinante GHRH die Sekretion von Insulin-ähnlichem Wachstumsfaktor I (*insulin-like growth factor I;* "IGF-I") in dem Subjekt fördert.

25. Verwendung nach Anspruch 9, wobei ein Symptom von chronischer Niereninsuffizienz über einen Basiswert erhöhte Serum-Kreatininwerte umfaßt.

26. Rekombinantes Somatotropin-Releasing-Hormon (*growth-hormone-releasing-hormone;* "GHRH") wie in Anspruch 21 beansprucht, wobei ein Symptom von chronischer Niereninsuffizienz über einen Basiswert erhöhte Serum-Kreatininwerte umfaßt.

## Revendications

1. Composition comprenant une construction d'expression d'un acide nucléique isolé qui code pour une hormone de libération de l'hormone de croissance ("GHRH"), destinée à prolonger l'espérance de vie d'un sujet souffrant de manière chronique d'une insuffisance rénale chronique.

2. Composition selon la revendication 1, comprenant en outre un polypeptide facilitant la transfection ou un polypeptide chargé.

3. Composition selon la revendication 2, dans laquelle le polypeptide facilitant la transfection ou polypeptide chargé comprend du poly-L-glutamate.

4. Composition selon la revendication 1, dans laquelle la construction d'expression de l'acide nucléique est formulée dans une solution de poly-L-glutamate à 0,01 %.

5. Composition selon la revendication 1, dans laquelle la construction d'expression de l'acide nucléique isolé est au moins à 90 % identique à une séquence choisie dans un groupe constitué par : SEQ ID n° 11, SEQ ID n° 12, SEQ ID n° 13, SEQ ID n° 14, SEQ ID n° 17, SEQ ID n° 18, SEQ ID n° 19, SEQ ID n° 20, et SEQ ID n° 21.

6. Composition selon la revendication 1, dans laquelle la quantité exprimée de la GHRH codée facilite la sécrétion d'hormone de croissance ("GH") chez le sujet.

7. Composition selon la revendication 1, dans laquelle la quantité exprimée de la GHRH codée facilite la sécrétion du facteur de croissance I analogue à l'insuline ("IGF-I") chez le sujet.

8. Composition selon la revendication 1, dans laquelle la GHRH codée comprend une séquence d'acides aminés au moins à 90 % identique à (SEQ ID n° 6) :
-X₁-X₂-DAIFTNSYRKVL-X₃-QLSARKLLQDI-X₄-X₅-RQQGE-X₆-NQE-X₇-GA-OH
dans laquelle la formule a les caractéristiques suivantes :
X₁ est un isomère D ou L de l'acide aminé tyrosine ("Y") ou histidine ("H") ;
X₂ est un isomère D ou L de l'acide aminé alanine ("A"), valine ("V") ou isoleucine ("I") ;
X₃ est un isomère D ou L de l'acide aminé alanine ("A") ou glycine ("G");
X₄ est un isomère D ou L de l'acide aminé méthionine ("M") ou leucine ("L") ;
X₅ est un isomère D ou L de l'acide aminé sérine ("S") ou asparagine ("N") ;
X₆ est un isomère D ou L de l'acide aminé arginine ("R") ou sérine ("S");
X₇ est un isomère D ou L de l'acide aminé glutamine ("Q") ou arginine ("R") ; ou une combinaison de celles-ci.

9. Utilisation d'une construction d'expression d'un acide nucléique isolé qui code pour une hormone de libération de l'hormone de croissance ("GHRH") pour la fabrication d'un médicament destiné à prolonger l'espérance de vie d'un sujet souffrant de manière chronique d'une insuffisance rénale chronique.

10. Utilisation selon la revendication 9, dans laquelle ledit traitement comprend l'électroporation d'un tissu du sujet comme procédé d'administration de la construction d'expression de l'acide nucléique isolé dans les cellules du sujet.

11. Utilisation selon la revendication 10, dans laquelle l'électroporation du tissu est un procédé comprenant :
a. la pénétration d'un tissu du sujet avec une pluralité d'électrodes-aiguilles, la pluralité d'électrodes-aiguilles étant disposée dans une relation espacée et le tissu du sujet comprenant des cellules musculaires ;
b. l'introduction de la construction d'expression de l'acide nucléique isolé dans le tissu entre la pluralité d'électrodes-aiguilles en une quantité dans la plage d'environ 0,1 à 5 mg ; et
c. l'application d'au moins une impulsion électrique à la pluralité d'électrodes-aiguilles, l'impulsion électrique permettant à la construction d'expression de l'acide nucléique isolé de traverser une membrane de cellule musculaire.

12. Utilisation selon la revendication 11, dans laquelle l'impulsion électrique envoyée à la pluralité d'électrodes-aiguilles comprend un champ électrique ayant une intensité dans la plage d'environ 0,4 à 1 A, avec une plage d'environ 3 à 5 impulsions, à environ 52 millisecondes/impulsion, et un intervalle d'environ 1 seconde entre les impulsions électriques.

13. Utilisation selon la revendication 11, comprenant en outre l'insertion des aiguilles dans un tissu musculaire à travers une zone de peau intacte, dans laquelle les aiguilles font environ 21 gauge de diamètre et environ 1 pouce (environ 2,54 cm) de long.

14. Utilisation selon la revendication 9, dans laquelle la construction d'expression de l'acide nucléique isolé comprend en outre un polypeptide facilitant la transfection ou un polypeptide chargé.

15. Utilisation selon la revendication 14, dans laquelle le polypeptide facilitant la transfection ou polypeptide chargé comprend du poly-L-glutamate.

16. Utilisation selon la revendication 9, dans laquelle la construction d'expression de l'acide nucléique est formulée dans une solution de poly-L-glutamate à 0,01 %.

17. Utilisation selon la revendication 9, dans laquelle la construction d'expression de l'acide nucléique isolé est au moins à 90 % identique à une séquence choisie dans un groupe constitué par : SEQ ID n° 11, SEQ ID n° 12, SEQ ID n° 13, SEQ ID n° 14, SEQ ID n° 17, SEQ ID n° 18, SEQ ID n° 19, SEQ ID n° 20, et SEQ ID n° 21.

18. Utilisation selon la revendication 9, dans laquelle la quantité exprimée de la GHRH codée facilite la sécrétion d'hormone de croissance ("GH") chez le sujet.

19. Utilisation selon la revendication 9, dans laquelle la quantité exprimée de la GHRH codée facilite la sécrétion du facteur de croissance I analogue à l'insuline ("IGF-I") chez le sujet.

20. Utilisation selon la revendication 9, dans laquelle la GHRH codée comprend une séquence d'acides aminés au moins à 90 % identique à (SEQ ID n° 6) :
-X₁-X₂-DAIFTNSYRKVL-X₃-QLSARKLLQDI-X₄-X₅-RQQGE-X₆-NQE-X₇-GA-OH
dans laquelle la formule a les caractéristiques suivantes :
X₁ est un isomère D ou L de l'acide aminé tyrosine ("Y") ou histidine ("H") ;
X₂ est un isomère D ou L de l'acide aminé alanine ("A"), valine ("V") ou isoleucine ("I") ;
X₃ est un isomère D ou L de l'acide aminé alanine ("A") ou glycine ("G");
X₄ est un isomère D ou L de l'acide aminé méthionine ("M") ou leucine ("L") ;
X₅ est un isomère D ou L de l'acide aminé sérine ("S") ou asparagine ("N") ;
X₆ est un isomère D ou L de l'acide aminé arginine ("R") ou sérine ("S") ;
X₇ est un isomère D ou L de l'acide aminé glutamine ("Q") ou arginine ("R") ; ou une combinaison de celles-ci.

21. Hormone de libération de l'hormone de croissance ("GHRH") recombinante destinée à prolonger l'espérance de vie d'un sujet souffrant de manière chronique d'une insuffisance rénale chronique.

22. Hormone de libération de l'hormone de croissance ("GHRH") recombinante selon la revendication 21, dans laquelle la GHRH recombinante comprend une séquence d'acides aminés au moins à 90 % identique à :
-X₁-X₂-DAIFTNSYRKVL-X₃-QLSARKLLQDI-X₄-X₅-RQQGE-X₆-NQE-X₇-GA-OH
dans laquelle la formule a les caractéristiques suivantes :
X₁ est un isomère D ou L de l'acide aminé tyrosine ("Y") ou histidine ("H") ;
X₂ est un isomère D ou L de l'acide aminé alanine ("A"), valine ("V") ou isoleucine ("I") ;
X₃ est un isomère D ou L de l'acide aminé alanine ("A") ou glycine ("G") ;
X₄ est un isomère D ou L de l'acide aminé méthionine ("M") ou leucine ("L") ;
X₅ est un isomère D ou L de l'acide aminé sérine ("S") ou asparagine ("N") ;
X₆ est un isomère D ou L de l'acide aminé arginine ("R") ou sérine ("S");
X₇ est un isomère D ou L de l'acide aminé glutamine ("Q") ou arginine ("R") ; ou une combinaison de celles-ci.

23. Hormone de libération de l'hormone de croissance ("GHRH") recombinante selon la revendication 21, dans laquelle la GHRH recombinante facilite la sécrétion d'hormone de croissance ("GH") chez le sujet.

24. Hormone de libération de l'hormone de croissance ("GHRH") recombinante selon la revendication 21, dans laquelle la GHRH recombinante facilite la sécrétion du facteur de croissance 1 analogue à l'insuline ("IGF-I") chez le sujet.

25. Utilisation selon la revendication 9, dans laquelle un symptôme d'insuffisance rénale chronique comprend des valeurs de créatinine sérique élevées par rapport à une valeur de base.

26. Hormone de libération de l'hormone de croissance ("GHRH") recombinante selon la revendication 21, dans laquelle un symptôme d'insuffisance rénale chronique comprend des valeurs de créatinine sérique élevées par rapport à une valeur de base.
